(19) Europäisches Patentamt
European Patent Office
Office européen des brevets

(11) EP 2 351 769 A2

(12) # EUROPEAN PATENT APPLICATION

(43) Date of publication:
**03.08.2011 Bulletin 2011/31**

(21) Application number: **10178276.1**

(22) Date of filing: **17.12.2002**

(51) Int Cl.:
***C07K 14/065*** *(2006.01)* ***C12N 15/863*** *(2006.01)*
***A61K 39/275*** *(2006.01)* ***A61P 31/20*** *(2006.01)*
***A61P 37/00*** *(2006.01)*

(84) Designated Contracting States:
**CH DE ES FR IT LI**

(62) Document number(s) of the earlier application(s) in accordance with Art. 76 EPC:
**02808241.0 / 1 575 614**

(71) Applicant: **AiCuris GmbH & Co. KG**
**42117 Wuppertal (DE)**

(72) Inventors:
- **Weber, Olaf**
  **42489, Wülfrath (DE)**
- **Friederichs, Sonja-Maria**
  **53721, Siegburg (DE)**
- **Siegling, Angela**
  **1230, Wien (AT)**
- **Schlapp, Tobias**
  **47574, Goch (DE)**
- **Mercer, Andrew Allan**
  **9016, Dunedin (NZ)**
- **Fleming, Stephen Bruce**
  **Fairfield, Dunedin (NZ)**
- **Volk, Hans-Dieter**
  **10117, Berlin (DE)**

(74) Representative: **Witte, Weller & Partner**
**Postfach 10 54 62**
**70047 Stuttgart (DE)**

Remarks:
This application was filed on 22-09-2010 as a divisional application to the application mentioned under INID code 62.

(54) **Recombinant proteins of Parapoxvirus ovis and pharmaceutical compositions therefrom**

(57) The invention relates to polynucleotides coding for the PPVO viral genome, to fragments of the polynucleotides coding for the PPVO genome and to polynucleotides coding for individual open reading frames (ORFs) of the PPVO viral genome. The invention also relates to recombinant proteins expressed from the above mentioned polynucleotides and to fragments of said recombinant proteins, and to the use of said recombinant proteins or fragments for the preparation of pharmaceutical compositions.

EP 2 351 769 A2

## Description

Field of the invention

The present invention relates to polynucleotides and recombinant proteins of *Parapoxvirus ovis* (PPVO) and their use, alone or in combination with other substances, for the manufacture of pharmaceutical compositions.

Background of the invention

It is known that latent and chronically persistent viral infections can be activated or reactivated by immunosuppression, or conversely that the immune system suppresses acute diseases which may be caused by a latent virus (for example a latent herpes virus infection recurs as a result of immunosuppression in the form of lip vesicles in cases of stress or the administration of cortisone). It is also known that chronically persistent latent viral infections can only be treated with difficulty or not at all using conventional low-molecular-weight antiviral substances.

It was demonstrated that class I restricted cytotoxic T cells were capable of inhibiting hepatocellular HBV gene expression in HBV-transgenic mice, and that this process was caused by TNF-$\alpha$ and IFN-$\gamma$.

It is also known that in the case of chronically persistent viral infections a superinfection with another virus can produce antiviral effects against the chronically persistent virus. The dependence of this effect on interferons such as IFN-$\gamma$, as well as other cytokines and chemokines, such as TNF-$\alpha$, which are secreted by T cells, natural killer cells and macrophages, has been demonstrated.

BAYPAMUN®, a pharmaceutical product for inducing "paraspecific immunity", i.e., a pharmaceutical product for inducing the unspecific immune system, is used therapeutically, metaphylactically and prophylactically for the treatment of animals in need. BAYPAMUN® is manufactured from chemically inactivated PPVO strain D1701 (see German Patent DE3504940). The inactivated PPVO induces in animals non-specific protection against infections with the most diverse types of pathogens. It is assumed that this protection is mediated via various mechanisms in the body's own defense system. These mechanisms include the induction of interferons, the activation of natural killer cells, the induction of "colony-stimulating activity" (CSA) and the stimulation of lymphocyte proliferation. Earlier investigations of the mechanism of action demonstrated the stimulation of interleukin-2 and interferon-$\alpha$.

The processes for the production of the above-mentioned pharmaceutical compositions are based on the replication of the virus in cultures of suitable host cells.

One aspect of the invention relates to the use of particle-like structures comprising recombinant proteins of the invention. These particle-like structures can be, e.g., fusion proteins, protein-coated particles or virus-like particles.

Methods to produce fusion proteins, protein-coated particles or virus-like particles comprising recombinant proteins of the invention are well known to persons skilled in the art: Casal (Biotechnol. Genet. Eng. Rev. 2001, 18: 73-87) describes the use of baculovirus expression systems for the generation of virus-like particles. Ellis (Curr. Opin. Biotechnol. 1996, 7(6): 646-52) presents methods to produce virus-like particles and the application of suitable adjuvants. Roy (Intervirol-ogy 1996, 39(1-2): 62-71) presents genetically engineered particulate virus-like structures and their use as vaccine delivery systems. Methods to produce fusion proteins are also well known to the person skilled in the art (Gaudin et al., Gen. Virol. 1995, 76: 1541-56; Hughson, Curr. Biol. 1995, 5(3): 365-74; Uhlen et al., Curr. Opin. Biotechnol. 1992, 3(4): 363-369). Known to the person skilled in the art is also the preparation of protein-coated micro- and nanospheres (Arshady, Biomaterials 1993, 14(1): 5-15). Proteins can be attached to biodegradable microspheres (Cleland, Pharm Biotechnol. 1997, 10: 1-43) or attached to other polymer microsheres (Hanes et al., Pharm. Biotechnol. 1995, 6: 389-412) such as, e.g., polysaccharides (Janes et al., Adv. Drug Deliv. Rev. 2001, 47(1): 83-97).

PPVO NZ2 is another Parapoxvirus strain that exhibits immunostimulatory effects when administered in inactivated form to mammals.

The closest prior art describes the construction of an expression library representing about 95 % of the PPVO NZ2 genome using the *Vaccina lister* virus to create recombinant viruses comprising the complete *Vaccina lister* genome and various fragments of the PPVO genome (Mercer et al. 1997, Virology, 229: 193-200). For the construction of the library, 16 PPVO DNA fragments with an average size of 11,4 kb were inserted into the *Vaccinia lister* genome. Each fragment was mapped relative to the PPVO restriction endonuclease maps but was otherwise uncharacterized (Fig. 1). It was found that a major portion of the PPVO genes were expressed in cells infected by the recombinant virus. The authors also showed that the entirety of all PPVO proteins expressed by some of the recombinant viruses of the expression library was able to provide protection against challenge with virulent PPVO. Expression of PPVO genes of the individual recombinant viruses has been demonstrated by immunofluorescence and immune precipitation (Mercer et al. 1997, Virology, 229: 193-200).

To identify components of PPVO responsible for the vaccinating activity of PPVO, the *Vaccinia lister*/ PPVO NZ2 expression library was applied.

Based on the above background it was desirable to develop PPVO based pharmaceutical compositions with antiviral and anti-tumor efficacy as well as with efficacy in paraimmunization and other desirable therapeutic effects. It was also desirable to obtain a pharmaceutical composition that exerts its full therapeutic effect while showing fewer side effects. It was furthermore desirable to find methods to produce PPVO based pharmaceutical compositions in large quantities and in economically advantageous manners.

These desirable effects have been achieved by the systematic use of selected recombinant proteins of PPVO alone or in combination with other recombinant proteins from PPVO for the preparation of pharmaceutical compositions for the treatment of objects in need.

## Summary of the invention

The invention relates to polynucleotides coding for the PPVO viral genome, to fragments of the polynucleotides coding for the PPVO genome and to polynucleotides coding for individual open reading frames (ORFs) of the PPVO viral genome. The invention also relates to fragments of said polynucleotides of at least 15 or 30 or 100 base pairs in length. The invention also relates to recombinant proteins expressed from the above mentioned polynucleotides and to fragments of said recombinant proteins of at least 5 or 10 or 30 amino acids, and to the use of recombinant proteins or fragments for the preparation of pharmaceutical compositions.

"Fragments" of a polynucleotide, within the meaning of the invention, shall be understood as polynucleotides that have the same nucleotide sequence as contiguous parts of the full length (the original) polynucleotide.

"Active fragments", within the meaning of the invention, shall be those fragments of the PPVO genome the expression products of which have demonstrated to be pharmacologically active according to the invention, when inserted into the *Vaccina lister* genome and expressed in a suitable host.

Whereas the use of the complete PPVO virus for the manufacture of vaccines against PPVO challenge has been described, the present invention relates to the use of polynucleotides coding for the PPVO viral genome and selected fragments of the PPVO viral genome and of selected PPVO expression products, alone or in combination with others, for the preparation of improved pharmaceutical compositions for the treatment of various diseases.

The systematic use of selected genomic fragments of PPVO and their recombinant expression products makes it possible to produce pharmaceutical compositions which contain fewer (and may not contain any) inactive components (i.e., polynucleotides and proteins of PPVO) in addition to the active components.

These pharmaceutical compositions which contain less, or do not contain any additional inactive components are generally preferred by doctors and patients compared to the less well defined biological preparations of inactivated virus material. Furthermore, the possibility of producing the recombinant product in fermentation processes allows an economically advantageous mode of production. It is well known to persons skilled in the art that an economically advantageous mode of production can be achieved, e.g., by using rapidly growing production organisms (host organisms) which might also place low demands on the culture medium employed. Microorganisms which can advantageously be used as hosts for the production of recombinant proteins include, e.g., but are not limited to, *Escherichia coli, Bacillus spec., Corynebacterium spec., Streptomyces spec.,* as well as yeasts, e.g., *Saccharomyces cerevisiae, Candida spec., Pichia spec., Hanselula spec.,* and filamentous fungi, e.g., *Aspergillus spec., Penicillium spec.* and other suitable microorganisms.

Recombinant proteins of the invention can also be produced from cell lines expressing the proteins of interest. These cell lines can be recombinant mammalian cell lines, recombinant insect cell lines (e.g., using the baculovirus transfection system) or other suitable expression systems. Transfection can be achieved by various techniques known to the skilled person, one of which is the use or recombinant viruses such as the *Vaccinia virus*/PPVO recombinants (VVOVs) described in the examples.

## Description of the invention

The invention relates to fragments of the PPVO genome of at least 15 or 30 or 100 base pairs in length, and recombinant proteins expressed therefrom and to the use of said fragments and recombinant proteins for the preparation of pharmaceutical compositions. The invention also relates to individual genes (ORFs) of PPVO and their expression products, and their use, alone or in combination with others, for the preparation of pharmaceutical compositions.

A protein, within the meaning of the invention, is any polypeptide of at least five amino acids. A recombinant protein, within the meaning of the invention, is any protein that is expressed in a cell, to which the coding polynucleotide was introduced using recombinant DNA technology.

A polynucleotide, within the meaning of the invention, is meant to comprise, polyribonucleotides and/or polydesoxyribonucleotides.

Pharmaceutical compositions of the invention can be used as immunotherapeutic or immunoprophylactic agents for the treatment of infectious and non-infectious immunodeficiencies. They can also be used for the treatment of tumor diseases, cancer, viral infections and diseases associated therewith, such as, e.g., hepatitis, papillomatosis, herpes virus infections, liver fibrosis, for the prevention or prophylaxis of infectious diseases after stress (e.g. operations), for the prevention and prophylaxis of infectious diseases by administration prior to operations or procedures (e.g. preceding implantations of artificial limbs or dental procedures), for the prophylactic and metaphylactic treatment of non-viral infections, for the healing of wounds, and in particular for accelerating wound-healing processes and for promoting the healing of poorly healing or non-healing wounds (e.g. Ulcus cruris), for diseases such as multiple sclerosis, warts and other skin neoplasms, for allergic diseases, for preventing the onset of systemic allergies and for topical allergies and for improving well-being, e.g. in old age, for autoimmune diseases, chronic inflammatory diseases, such as, e.g., Crohn's disease, COPD and asthma. It is an object of the invention to use of polynucleotides and recombinant proteins of PPVO for the production of pharmaceutical compositions for the treatment of the above mentioned conditions and diseases in humans and animals.

The viral strains of the invention are PPVO NZ2 and homologues, such as D1701, NZ7, NZ10 and orf-11 strains. It is also possible to use polynucleotides and recombinant proteins of the progeny of these strains obtained by passaging and/or adaptation using specific cells, such as e.g. WI-38, MRC-5 or Vero cells.

We have found that the identified recombinant proteins are effective for the treatment of viral diseases, cancer and other diseases or conditions in which a Th1 type immune response is of benefit. The results obtained also imply that PPVO gene products or parts thereof protect hepatitis virus-expressing hepatocytes (e.g. hepatitis B virus, HBV, or hepatitis C virus, HCV) from immune attack through HBV or HCV specific cytotoxic CD8+ T cells circulating in the blood because T cells will not leave the blood stream if their specific antigen is not presented by liver sinus endothelial cells (LSEC, that anatomically separate hepatocytes from T cells passing the liver with the blood). Therefore, we expect to have a recombinant protein that is derived from the ORFs 120-R3 (base pairs 122616 - 136025 Bp, recombinant virus VVOV82) that is able to down-modulate or prevent side effects such as necroinflammatory liver disease when immunostimulants, e.g. cytokines or any others including the proteins described above administered to e.g. hepatitis patients.

Considering the knowledge about the influence of a Th1 type immune induction in conditions and diseases such as latent and or chronic viral infections, proliferative diseases such as cancer and the capability of recombinant proteins that contain gene products of PPVO or parts thereof to induce a Th1 immune response or a local immune response selectively, we claim the use of polynucleotides and recombinant polypeptides of PPVO and recombinant proteins that contain gene products of PPVO or parts thereof for the manufacture of pharmaceutical compositions for use in humans and animals. The recombinant proteins are made from products or parts thereof of the following open reading frames (ORFs) of PPVO NZ2: 64r-96r (recombinants VVOV 285 and VVOV 330 as well as VVOV 243 and VVOV 283), 18r-57 (recombinants VVOV 97, VVOV 96 and VVOV 245), 4r-14r (recombinant VVOV 215). The recombinant protein may also be made from gene products or parts thereof of ORFs 120-R3 (recombinant VVOV 82). The proteins may be prepared and used in any combination.

Recombinant proteins of PPVO within the meaning of the invention shall be understood as proteins that derive from PPVO and are expressed in homologous or heterologous systems other than the systems in which PPVO is naturally produced. Examples for recombinant proteins of PPVO are proteins of PPVO which are expressed using *Vaccinia* virus vectors and fibroblasts as host cells or baculovirus vectors and insect cells as host cells. Recombinant proteins, within the meaning of the invention, could also be produced in bacterial cells (e.g., *Escherichia coli, Bacillus spec., Streptomyces spec.*) or in yeast (e.g. *Saccharomyces cerevisiae, Candida spec., Pichia pastoris, Hansenula spec.*) systems. In these cases, polynucleotides of the PPVO genome would typically be brought into the respective host genome so that PPVO genes are expressed by the host. Recombinant proteins of PPVO could also be expressed by the object in need in the sense of a gene therapy.

Recombinant proteins, within the meaning of the invention, could also be recombinant virus particles that contain PPVO derived proteins. Recombinant proteins, within the meaning of the invention, could also be in form of viral-like particles that are formed or assembled from PPVO derived proteins. Recombinant proteins, within the meaning of the invention, could also be chimeric proteins that contain PPVO gene products.

In a preferred embodiment of the invention the recombinant proteins are attached to particle-like structures or be part of particle-like structures.

In another preferred embodiment of the invention the recombinant proteins are attached to, or part of, fusion proteins.

In another preferred embodiment of the invention the recombinant proteins are attached to, or part of, protein-coated particles.

In another preferred embodiment of the invention the recombinant proteins are attached to, or part of, virus-like particles.

Particle-like structures, such as particle-like fusion proteins, protein-coated particles or virus-like particles can be phagocytosed and processed by monocytes or macrophages. The process of phagocytosis enhances the efficacy of recombinant proteins of the invention in uses within the meaning of the invention.

A particle-like structure, within the meaning of the invention, is particulate matter in particle-like form of which the average particle size and other characteristics are suitable for medical application. Preferred particle-like structures are, e.g., fusion proteins, protein-coated particles, or virus-like particles.

Immunomodulating activity is defined as local or systemic suppression and/or stimulation and/or induction of any Th-1 or Th-2 type cytokine response or of any effector function of these cytokines, (e.g. cytolytic or antiviral activity or humoral response) or the modulation of MHC cross-presentation. Immunomodulating activity could also be the induction of apoptosis in antigen presenting cells or recruiting of antigen presenting cells.

Nucleotides and recombinant proteins of the invention can be administered at the same time or sequentially, administered with other agents and drugs, e.g. with drugs that treat the disease or are supportive, e.g. in the case of cancer therapy with antineoplastic or other anti-cancer agents or/and anti-coagulants or vitamins, pain relief and others.

The nucleotides and recombinant proteins can be administered systemically (e.g., intravenously, subcutaneously, intramuscularly, intracutaneously, intraperitoneally), locally (e.g., into a tumor) or orally (per os). The recombinant proteins or products thereof should be formulated appropriately, e.g. in a non-pyrogenic solution or suspension for i.v. use or in capsules for implantation or in capsules for per os use. Pharmaceutical compositions of the invention can be administered, e.g., oral, nasal, anal, vaginal etc., as well as parenteral administration. Pharmaceutical compositions of the invention can be in the form of suspensions, solutions, syrups, elixirs or appropriate formulations in polymers as well as liposomes.

Recombinant proteins of the invention can also be prepared with suitable recombinant cell lines and other cell lines. Alternatively, non-recombinant cell lines, such as WI-38, MRC-5, Vero cells could be infected with recombinant viruses that carry the recombinant genes using viral vectors such as, but not limited to, the *Vaccina* virus (e.g., *Vaccina lister*). In addition, other suitable viruses can be used in combination with other suitable cells (e.g., using *Vaccinia* virus vectors and fibroblasts as host cells or *baculovirus* vectors and insect cells as host cells). It is advantageous to cultivate the recombinant cell cultures in high-cell-density fermentations to achieve favorable productivity and a good overall process performance.

The invention relates to purified and isolated polynucleotides with the sequence of SEQ ID 01. The invention also relates to purified and isolated polynucleotides of at least 15 or 30 or 100 nucleotides which bind under stringent conditions to the polynucleotide of SEQ ID 01 or its complementary sequences.

Stringent conditions, within the meaning of the invention are 65°C in a buffer containing 1 mM EDTA, 0.5 M $NaHPO_4$ (pH 7.2), 7 % (w/v) SDS.

The invention also relates to purified and isolated polynucleotides which comprise the polynucleotide sequence of SEQ ID 01 or polynucleotide sequences encoding the same amino acid sequence and fragments of at least 15 or 30 or 100 nucleotides thereof. The invention also relates to recombinant proteins of five and more amino acids encoded by these polynucleotides.

The invention also relates to purified and isolated polynucleotides which show at least 99 %, 95 % or 90 % or 80% sequence homology to the polynucleotides of the previous paragraph.

*Homology* of biological sequences, within the meaning of the invention, shall be understood as the homology between two biological sequences as calculated by the algorithm of Needleman and Wunsch. (1970. J. Mol. Biol. 48: 443-453) using the BLOSUM62 substitution matrix (Henikoff and Henikoff 1992. Proc. Natl. Acad. Sci. USA 89: 10915-10919) for proteins and penalties of +4 and -3 for identical and non-identical bases, respectively, when comparing polynucleotide sequences. For comparison of protein sequences the *gap creation penalty* and the *gap extension penalty* are 8 and 2, respectively. For comparison of polynucleotide sequences the *gap creation penalty* and the *gap extension penalty* are 20 and 3, respectively.

The invention also relates to purified and isolated polynucleotides which are *active fragments* of the PPVO genome, with a sequence selected from a group of sequences consisting of nucleotides 122616-136025 of SEQ ID 01 (PPVO insert of VVOV 82), 31003-46845 of SEQ ID 01 (PPVO insert of VVOV 96), 24056-33789 of SEQ ID 01 (PPVO insert of VVOV 97), 10264-20003 of SEQ ID 01 (PPVO insert of VVOV 215), 82324-92502 of SEQ ID 01 (PPVO insert of VVOV 243), 47952-66263 of SEQ ID 01 (PPVO insert of VVOV 245), 89400-103483 of SEQ ID 01 (PPVO insert of VVOV 283), 74804-88576 of SEQ ID 01 (PPVO insert of VVOV 285), and 102490-108393 of SEQ ID 01 (PPVO insert of VVOV 330).

The invention also relates to purified and isolated polynucleotide which encode for the same amino acid sequence as the active fragments of the PPVO genome of the previous paragraph and to polynucleotides of at least 15 or 30 or 100 nucleotides binding under stringent conditions to the above mentioned active fragments of the PPVO genome or its complementary sequence.

The invention also relates to polynucleotides with 99 %, 95 %, or 90 %, or 80 % sequence homology to sequences consisting of nucleotides 122616-136025 of SEQ ID 01 (PPVO insert of VVOV 82), 31003-46845 of SEQ ID 01 (PPVO insert of VVOV 96), 24056-33789 of SEQ ID 01 (PPVO insert of VVOV 97), 10264-20003 of SEQ ID 01 (PPVO insert of VVOV 215), 82324-92502 of SEQ ID 01 (PPVO insert of VVOV 243), 47952-66263 of SEQ ID 01 (PPVO insert of VVOV 245), 89400-103483 of SEQ ID 01 (PPVO insert of VVOV 283), 74804-88576 of SEQ ID 01 (PPVO insert of VVOV 285), and 102490-108393 of SEQ ID 01 (PPVO insert of VVOV 330) or the respective complementary sequences.

The invention also relates to purified and isolated polynucleotide, with a sequence of nucleotides 3 to 539 (ORF L1), 781 to 449 (ORF L2r), 1933 to 1664 (ORF L3r), 3269 to 2790 (ORF L4r), 2799 to 3851 (ORF L5), 2962 to 3753 (ORF L6), 3784 to 3122 (ORF L7r), 4341 to 4129 (ORF L8r), 4904 to 4428 (ORF 1ar), 6517 to 4970 (ORF 1r), 8042 to 6684 (ORF 2r), 9989 to 8070 (ORF 3r), 11195 to 10062 ORF 4r), 11493 to 11227 (ORF 5r), 11802 to 12038 (ORF 6), 12358 to 12080 (ORF 7r), 13980 to 12364 (ORF 8r), 14826 to 14053 (ORF 9ar), 15080 to 15394 (ORF 10), 16838 to 15423 (ORF 11r), 19021 to 16847 (ORF 12r), 19704 to 19156 (ORF 13r), 20314 to 19736 (ORF 14r), 20401 to 22101 (ORF 15), 22125 to 22940 (ORF 6), 23003 to 23866 (ORF 17), 26908 to 23873 (ORF 18r), 26926 to 27213 (ORF 19), 27626 to 27216 (ORF 20r), 29754 to 27616 (ORF 21r), 32217 to 29800 (ORF 22r), 33380 to 32418 (ORF 23r), 33602 to 33393 (ORF 24r), 34466 to 33612 (ORF 25r), 34735 to 34502 (ORF 26r), 35905 to 34739 (ORF 27r), 37194 to 35905 (ORF 28r), 37200 to 39248 (ORF 29), 41037 to 39229 (ORF 30r), 41374 to 42066 (ORF 31), 42336 to 41731 (ORF 32r), 42407 to 41997 (ORF 33r), 42410 to 43765 (ORF 34), 43770 to 43958 (ORF 35), 43980 to 44534 (ORF 36), 45727 to 44537 (ORF 37r), 45760 to 46557 (ORF 38), 46567 to 47568 (ORF 39), 47572 to 48303 (ORF 40), 48352 to 48621 (ORF 41), 49887 to 48634 (ORF 42r), 49917 to 50693 (ORF 43), 50719 to 51102 (ORF 44), 51059 to 51511 (ORF 44a), 51584 to 52591 (ORF 45), 52509 to 53066 (ORF 46), 53523 to 53023 (ORF 47r), 53607 to 57473 (ORF 48), 58070 to 57528 (ORF 49r), 57700 to 58662 (ORF 50), 59674 to 58673 (ORF 51r), 62089 to 59678 (ORF 52r), 62198 to 62881 (ORF 53), 62909 to 63862 (ORF 55), 63858 to 64271 (ORF 56), 64309 to 66831 (ORF 57), 67266 to 66799 (ORF 58r), 67803 to 67273 (ORF 58ar), 67915 to 68607 (ORF 59), 68624 to 70984 (ORF 60), 70994 to 72898 (ORF 61), 72938 to 73507 (ORF 62), 73540 to 74211 (ORF 63), 76120 to 74207 (ORF 64r), 76749 to 76186 (ORF 65r), 77698 to 76799 (ORF 66r), 79343 to 77709 (ORF 67r), 79816 to 79367 (ORF 68r), 80529 to 79858 (ORF 69r), 80774 to 80529 (ORF 70r), 82815 to 80788 (ORF 71r), 83835 to 82834 (ORF 72r), 83874 to 85583 (ORF 73), 85535 to 84402 (ORF 74r), 88096 to 85574 (ORF 75r), 87759 to 88667 (ORF 76), 88920 to 88642 (ORF 77r), 91652 to 88938 (ORF 78r), 91667 to 92674 (ORF 79), 93466 to 92681 (ORF 80r), 93761 to 93486 (ORF 81r), 94060 to 93788 (ORF 82r), 94238 to 94080 (ORF 83r), 94508 to 94242 (ORF 84r), 95571 to 94498 (ORF 85r), 96187 to 95600 (ORF 86r), 96202 to 97665 (ORF 87), 97915 to 97643 (ORF 88r), 98251 to 99537 (ORF 89), 99537 to 99974 (ORF 90), 100001 to 101140 (ORF 91), 101168 to 104650 (ORF 92), 106354 to 104795 (ORF 93r), 107947 to 106400 (ORF 94r), 108256 to 107990 ORF 95r), 108719 to 108300 (ORF 96r), 109679 to 108738 (ORF 97r), 109861 to 109682 (ORF 98r), 110830 to 10033 (ORF 99r), 110208 to 110417 (ORF 100), 110469 to 110651 (ORF 100a), 110915 to 111397 (ORF 101), 111419 to 111913 (ORF 102), 111949 to 112485 (ORF 103), 112593 to 113450 (ORF 104), 113323 to 112967 ORF 105r), 113526 to 114152 (ORF 106), 114199 to 115236 (ORF 107), 115353 to 115787 (ORF 108), 115859 o 116551 (ORF 109), 116729 to 117523 (ORF 110), 117572 to 117114 (ORF 111r), 117423 to 118085 (ORF 12), 118968 to 118375 (ORF 114r), 118508 to 119119 (ORF 115), 119588 to 120202 (ORF 116), 120314 to 21231 (ORF 117), 121380 to 123920 (ORF 118), 121288 to 122256 (ORF 119), 122350 to 123924 (ORF 120), 123962 to 125566 (ORF 121), 125193 to 124591 (ORF 122r), 125689 to 123935 (ORF 123r), 123839 to 123297 ORF 123ar), 125652 to 126170 (ORF 124), 126121 to 125699 (ORF 125r), 126279 to 127769 (ORF 126), 127851 to 128408 (ORF 127), 128520 to 130076 (ORF 128), 130105 to 131700 (ORF 129), 131790 to 133283 (ORF 130), 133246 to 133920 (ORF 131), 133972 to 134370 (ORF 132), 134418 to 134693 (ORF 133a), 134402 to 134992 (ORF R1), 134853 to 134419 (ORF R2r), 135628 to 135897 (ORF R3), 136780 to 137112 ORF R4), and 137558 to 137022 (ORF R5r) of SEQ ID 01, which encode for the identified open reading frames (ORFs) listed in Table 7. ORFs of this paragraph of which the start position is a larger number than the stop position are coded by the complementary sequence of SEQ ID 01. The names of these ORFs end with the letter "r". The invention also relates to the complementary sequences of the sequences of this paragraph.

The invention also relates to polynucleotides which encode for the same amino acid sequence as encoded by the identified ORFs of the previous paragraph. The invention also relates to polynucleotides of at least 15, 30 or 100 nucleotides binding under stringent conditions to the identified ORFs. The invention also relates to polynucleotides which show at least 99 %, 95 % or 90 % or 80 % sequence homology to the sequences of the previous paragraph or which are functional variants a sequence of the previous paragraph.

*A functional variant* of a gene, within the meaning of the invention, shall be defined as a gene which is at least 99 %, or 95 %, or 90 %, or 80 % homologous to the first gene and which has a similar biological function as the first gene. A functional variant of a gene can also be a second gene encoding the same amino acid sequence as does the first gene (or as does a functional variant thereof), employing the degeneration of the genetic code. A functional variant of a gene can also be a polynucleotide comprising the same sequence as has said gene, however said polynucleotide being shorter (i.e., by means of deletions of one or several nucleotides at one or both ends of the polynucleotide) or said polynucleotide having additional nucleotides at one or both ends of the identical part of the polynucleotide.

*A functional variant* of a protein, within the meaning of the invention, shall be defined as another protein which is at least 99 %, or 95 %, or 90 %, or 80 % homologous to the first protein and which has a similar biological function as has the original protein.

The invention also relates to recombinant proteins encoded by nucleotides of the invention and parts and fragments of said proteins which are at least 5 or 7 or 10 or 30 amino acids long.

The invention also relates to recombinant proteins encoded by nucleotides of the invention and parts and fragments of said proteins which are at least 5 or 7 or 10 or 30 amino acids long, said recombinant proteins being attached to a carrier protein or to another carrier. Attaching a protein to a carrier protein can improve or strengthen the immune response to said protein, thereby enhancing the therapeutic or prophylactic effect of administering said protein to a subject.

The invention also relates to vectors containing polynucleotides of the invention and cells containing these vectors or polynucleotides of the invention.

The invention also relates to the use of recombinant proteins and polynucleotides of the invention, alone or in combination with at least one other recombinant protein or polynucleotide of the invention for the manufacture of pharmaceutical compositions.

Combinations of recombinant proteins (or polynucleotides) according to the invention, comprise

- combinations of at least two recombinant proteins encoded by SEQ ID 01 (or combinations of at least two fragments of a polynucleotide of SEQ ID 01),
- combinations of at least two recombinant proteins encoded by the same *active fragment* of the PPVO genome, i.e., two or more recombinant proteins encoded by the same VVOV of Table 3, Table 4, Table 5, and Table 6 (or combinations of at least two fragments of the same *active fragment* (VVOV) of the PPVO genome),
- combinations of at least two recombinant proteins, encoded by at least two distinct *active fragments* of the PPVO genome, i.e., from distinct VVOVs of Table 3, Table 4, Table 5, and Table 6 (or combinations of at least two fragments of at least two *distinct* active fragments (VVOVs) of the PPVO genome), or
- combinations of at least two distinct recombinant proteins encoded by ORFs of Table 7 (or combinations of at least two polynucleotides with the sequence of any of the ORFs listed in Table 7).

The invention also relates to the use of recombinant viruses comprising the *Vaccina lister* genome and selected fragments of the PPVO genome for the manufacture of pharmaceutical compositions.

The invention also relates to the use of recombinant proteins and polynucleotides of the invention for the manufacture of pharmaceutical compositions for the treatment of virus related diseases, viral infections, non-viral infections, proliferative diseases, inflammatory diseases, allergic diseases, and autoimmune diseases.

Viral infections, within the meaning of the invention, shell be understood as diseases associated with viral infections of the human or animal body, such as hepatitis, papillomatosis, herpes virus infections, liver fibrosis, HIV infections, AIDS, and influenza.

Non-viral infections, within the meaning of the invention, shell be understood as diseases associated with non-viral infections of the human or animal body, such as infections with mycobacteria, mycoplasma, amoebia, and plasmodia.

Proliferative diseases, within the meaning of the invention, shell be understood as diseases associated with proliferative disorders, such as cancer, leukemia, warts, tumor diseases, and other skin neoplasms.

Inflammatory diseases, within the meaning of the invention, shell be understood as diseases associated with acute or chronic inflammatory conditions, such as inflammation of the skin or organs, Crohn's disease, COPD, Asthma, but also conditions related to the healing of wounds, e.g. Ulcus cruris., and others.

Allergic diseases, within the meaning of the invention, shell be understood as comprising both systemic and topical allergies.

Autoimmune diseases within the meaning of the invention, shell be understood as comprising systemic lupus erythematosus, Sjogren's syndrome, Hashimoto's thyroiditis, rheumatoid arthritis, and juvenile diabetes mellitus, and other autoimmune diseases.

The invention also relates to the use of recombinant viruses comprising a *Vaccinia lister* genome and fragments of a PPVO genome for the manufacture of pharmaceutial compositions.

The invention also relates to the use of recombinant viruses comprising a *Vaccinia lister* genome and at least one heterologous gene to express at least one heterologous gene in a subject, e.g., for prophylactic and/or therapeutic purposes.

The invention also relates to the use of a recombinant viruses comprising a *Vaccinia lister* genome and at least one heterologous gene for gene therapy.

"Gene therapy", within the meaning of the invention, shall be understood as the act of administering to a subject polynucleotides (and, if necessary, suitable adjuvants or suitable carriers) for the purpose of obtaining a prophylactic or therapeutic effect in said subject. Typically, the polynucleotides administered are expressed in the subject and the expressed gene products exert a prophylactic or therapeutic effect.

The invention also relates to

(a) a particle-like structure comprising a recombinant polypeptide encoded by an open reading frame (ORF) of the polynucleotide of SEQ ID NO: 1 or functional variants of said polypeptides,

(b) the use of a particle-like structure of (a) for the preparation of a medicament,

(c) the use of a particle-like structure of (a) for the preparation of a medicament for the treatment of virus related diseases, viral infections, non-viral infections, proliferative diseases, inflammatory diseases, allergic diseases, and/or autoimmune diseases,

(d) pharmaceutical compositions comprising a particle-like structure of (a), and to

(e) pharmaceutical compositions comprising a particle-like structure of (a) for the treatment of virus related diseases, viral infections, non-viral infections, proliferative diseases, inflammatory diseases, allergic diseases, and/or autoimmune diseases.

The present invention comprises the aspects defined in the following sentences, which form a part of the present description, however are no claims, in accordance with the decision J15/88 of the Legal Board of Appeal of the European Patent Office.

1. Purified and isolated polynucleotide with the sequence of SEQ ID 01 or its complementary sequence and fragments and functional variants thereof.

2. Purified and isolated polynucleotide with a sequence selected from a group of sequences consisting of nucleotides number 122616 to 136025 of SEQ ID 01 (PPVO insert of VVOV 82), 31003 to 46845 of SEQ ID 01 (PPVO insert of VVOV 96), 24056 to 33789 of SEQ ID 01 (PPVO insert of VVOV 97), 10264 to 20003 of SEQ ID 01 (PPVO insert of VVOV 215), 82324 to 92502 of SEQ ID 01 (PPVO insert of VVOV 243), 47952 to 66263 of SEQ ID 01 (PPVO insert of VVOV 245), 89400 to 103483 of SEQ ID 01 (PPVO insert of VVOV 283), 74804 to 88576 of SEQ ID 01 (PPVO insert of VVOV 285), and 102490 to 108393 of SEQ ID 01 (PPVO insert of VVOV 330) or their complementary sequences and functional variants thereof.

3. Purified and isolated polynucleotide with a sequence selected from a group of sequences consisting of nucleotides 3 to 539 (ORF L1), 781 to 449 (ORF L2r), 3269 to 2790 (ORF L4r), 2799 to 3851 (ORF L5), 2962 to 3753 (ORF L6), 3784 to 3122 (ORF L7r), 4341 to 4129 (ORF L8r), 8042 to 6684 (ORF 2r), 9989 to 8070 (ORF 3r), 11493 to 11227 (ORF 5r), 11802 to 12038 (ORF 6), 12358 to 12080 (ORF 7r), 13980 to 12364 (ORF 8r), 14826 to 14053 (ORF 9ar), 15080 to 15394 (ORF 10), 16838 to 15423 (ORF 11r), 19021 to 16847 (ORF 12r), 20314 to 19736 (ORF 14r), 20401 to 22101 (ORF 15), 22125 to 2940 (ORF 16), 23003 to 23866 (ORF 17), 26908 to 23873 (ORF 18r), 26926 to 27213 (ORF 19), 27626 to 27216 (ORF 20r), 29754 to 27616 (ORF 21r), 32217 to 29800 (ORF 22r), 33380 to 32418 (ORF 23r), 33602 to 33393 (ORF 24r), 34466 to 33612 (ORF 25r), 34735 to 34502 (ORF 26r), 35905 to 34739 (ORF 27r), 37194 to 35905 (ORF 28r), 37200 to 39248 (ORF 29), 41037 to 39229 (ORF 30r), 41374 to 42066 (ORF 31), 42336 to 41731 (ORF 32r), 42407 to 41997 (ORF 33r), 42410 to 43765 (ORF 34), 43770 to 43958 (ORF 35), 43980 to 44534 (ORF 36), 45727 to 44537 (ORF 37r), 45760 to 46557 (ORF 38), 46567 to 47568 (ORF 39), 47572 to 48303 (ORF 40), 48352 to 48621 (ORF 41), 49887 to 48634 (ORF 42r), 49917 to 50693 (ORF 43), 50719 to 51102 (ORF 44), 51059 to 51511 (ORF 44a), 51584 to 52591 (ORF 5), 52509 to 53066 (ORF 46), 53523 to 53023 (ORF 47r), 53607 to 57473 (ORF 48), 58070 to 57528 (ORF 49r), 57700 to 58662 (ORF 50), 59674 to 58673 (ORF 51r), 63858 to 64271 (ORF 56), 64309 to 66831 (ORF 57), 67266 to 66799 (ORF 58r), 67803 to 67273 (ORF 58ar), 67915 to 68607 (ORF 59), 68624 to 70984 (ORF 60), 70994 to 72898 (ORF 61), 72938 to 73507 (ORF 62), 73540 to 74211 (ORF 63), 76120 to 74207 (ORF 64r), 76749 to 76186 (ORF 65r), 77698 to 76799 (ORF 66r), 79343 to 77709 (ORF 67r), 79816 to 79367 (ORF 68r), 80529 to 79858 (ORF 69r), 80774 to 80529 (ORF 70r), 82815 to 80788 (ORF 71r), 83835 to 82834 (ORF 72r), 83874 to 85583 (ORF 73), 85535 to 84402 (ORF 74r), 88096 to 85574 (ORF 75r), 87759 to 88667 (ORF 76), 91652 to 88938 (ORF 78r), 91667 to 92674 (ORF 79), 93466 to 92681 ORF 80r), 93761 to 93486 (ORF 81r), 94060 to 93788 (ORF 82r), 94238 to 94080 (ORF 83r), 94508 to 94242 (ORF 84r), 95571 to 94498 (ORF 85r), 96187 to 95600 (ORF 86r), 96202 to 97665 (ORF 87), 97915 to 97643 (ORF 88r), 98251 to 99537 (ORF 89), 99537 to 99974 (ORF 90),100001 to 101140 (ORF 91), 101168 to 104650 (ORF 92), 109679 to 108738 (ORF 97r), 109861 to 109682 (ORF 98r), 110830 to 110033 (ORF 99r), 110208 to 110417 (ORF 100), 110469 to 110651 (ORF 100a), 110915 to 111397 (ORF 101), 111419 to 111913 (ORF 102), 111949 to 112485 (ORF 103), 112593 to 113450 (ORF 104), 113323 to 112967 (ORF 105r), 113526 to 114152 (ORF 106), 114199 to 115236 (ORF 107), 115353 to 115787 (ORF 108), 115859 to 116551 (ORF 109), 117572 to 117114 (ORF 111r), 117423 to 118085 (ORF 112), 118968 to 118375 (ORF 114r), 118508 to 119119 (ORF 115), 119588 to 120202 (ORF 116), 120314 to 121231 (ORF 117), 121380 to 123920 (ORF 118), 121288 to 122256 (ORF 119), 122350 to 123924 (ORF 120), 123962 to 125566 (ORF 121), 125193 to 124591 (ORF 122r), 125689 to 123935 (ORF 123r), 123839 to 123297 (ORF 123ar), 125652 to 126170 (ORF 124), 126121 to 125699 (ORF 125r), 126279 to 127769 (ORF 126), 127851 to 128408 (ORF 127), 128520 to 130076 (ORF 128), 136780 to 137112 (ORF R4), and137558 to 137022 (ORF R5r) of SEQ ID 01 or their complementary sequence and functional variants thereof.

4. Recombinant proteins of encoded by a polynucleotide of any of sentence 1, 2 or 3, or functional variants or fragments of more than five amino acids thereof.

5. Vector comprising a polynucleotide of sentence 1, 2 or 3.

6. Cells containing vectors of claim 5 or a polynucleotide of sentence 1, 2 or 3.

7. Use of a recombinant protein encoded by a polynucleotide selected from a group of polynucleotides consisting of

(a) a polynucleotide of sentence 1,

(b) a polynucleotide of sentence 2, and

(c) a polynucleotide with a sequence selected from a group of sequences consisting of nucleotides 3 to 539 (ORF L1), 781 to 449 (ORF L2r), 1933 to 1664 (ORF L3r), 3269 to 2790 (ORF L4r), 2799 to 3851 (ORF L5), 2962 to 3753 (ORF L6), 3784 to 3122 (ORF L7r), 4341 to 4129 (ORF L8r), 4904 to 4428 (ORF 1ar), 6517 to 4970 (ORF 1r), 8042 to 6684 (ORF 2r), 9989 to 8070 (ORF 3r), 11195 to 10062 ORF 4r), 11493 to 11227 (ORF 5r), 11802 to 12038 (ORF 6), 12358 to 12080 (ORF 7r), 13980 to 12364 (ORF 8r), 14826 to 14053 (ORF 9ar), 15080 to 15394 (ORF 10), 16838 to 15423 (ORF 11r), 19021 to 16847 (ORF 12r), 19704 to 19156 (ORF 13r), 20314 to 19736 (ORF 14r), 20401 to 22101 (ORF 15), 22125 to 22940 (ORF 6), 23003 to 23866 (ORF 17), 26908 to 23873 (ORF 18r), 26926 to 27213 (ORF 19), 27626 to 27216 (ORF 20r), 29754 to 27616 (ORF 21r), 32217 to 29800 (ORF 22r), 33380 to 32418 (ORF 23r), 33602 to 33393 (ORF 24r), 34466 to 33612 (ORF 25r), 34735 to 34502 (ORF 26r), 35905 to 34739 (ORF 27r), 37194 to 35905 (ORF 28r), 37200 to 39248 (ORF 29), 41037 to 39229 (ORF 30r), 41374 to 42066 (ORF 31), 42336 to 41731 (ORF 32r), 42407 to 41997 (ORF 33r), 42410 to 43765 (ORF 34), 43770 to 43958 (ORF 35), 43980 to 44534 (ORF 36), 45727 to 44537 (ORF 37r), 45760 to 46557 (ORF 38), 46567 to 47568 (ORF 39), 47572 to 48303 (ORF 40), 48352 to 48621 (ORF 41), 49887 to 48634 (ORF 42r), 49917 to 50693 (ORF 43), 50719 to 51102 (ORF 44), 51059 to 51511 (ORF 44a), 51584 to 52591 (ORF 45), 52509 to 53066 (ORF 46), 53523 to 53023 (ORF 47r), 53607 to 57473 (ORF 48), 58070 to 57528 (ORF 49r), 57700 to 58662 (ORF 50), 59674 to 58673 (ORF 51r), 62089 to 59678 (ORF 52r), 62198 to 62881 (ORF 53), 62909 to 63862 (ORF 55), 63858 to 64271 (ORF 56), 64309 to 66831 (ORF 57), 67266 to 66799 (ORF 58r), 67803 to 67273 (ORF 58ar), 67915 to 68607 (ORF 59), 68624 to 70984 (ORF 60), 70994 to 72898 (ORF 61), 72938 to 73507 (ORF 62), 73540 to 74211 (ORF 63), 76120 to 74207 (ORF 64r), 76749 to 76186 (ORF 65r), 77698 to 76799 (ORF 66r), 79343 to 77709 (ORF 67r), 79816 to 79367 (ORF 68r), 80529 to 79858 (ORF 69r), 80774 to 80529 (ORF 70r), 82815 to 80788 (ORF 71r), 83835 to 82834 (ORF 72r), 83874 to 85583 (ORF 73), 85535 to 84402 (ORF 74r), 88096 to 85574 (ORF 75r), 87759 to 88667 (ORF 76), 88920 to 88642 (ORF 77r), 91652 to 88938 (ORF 78r), 91667 to 92674 (ORF 79), 93466 to 92681 (ORF 80r), 93761 to 93486 (ORF 81r), 94060 to 93788 (ORF 82r), 94238 to 94080 (ORF 83r), 94508 to 94242 (ORF 84r), 95571 to 94498 (ORF 85r), 96187 to 95600 (ORF 86r), 96202 to 97665 (ORF 87), 97915 to 97643 (ORF 88r), 98251 to 99537 (ORF 89), 99537 to 99974 (ORF 90), 100001 to 101140 (ORF 91), 101168 to 104650 (ORF 92), 106354 to 104795 (ORF 93r), 107947 to 106400 (ORF 94r), 108256 to 107990 ORF 95r), 108719 to 108300 (ORF 96r), 109679 to 108738 (ORF 97r), 109861 to 109682 (ORF 98r), 110830 to 10033 (ORF 99r), 110208 to 110417 (ORF 100), 110469 to 110651 (ORF 100a), 110915 to 111397 (ORF 101), 111419 to 111913 (ORF 102), 111949 to 112485 (ORF 103), 112593 to 113450 (ORF 104), 113323 to 112967 ORF 105r), 113526 to 114152 (ORF 106), 114199 to 115236 (ORF 107), 115353 to 115787 (ORF 108), 115859 o 116551 (ORF 109), 116729 to 117523 (ORF 110), 117572 to 117114 (ORF 111r), 117423 to 118085 (ORF 12), 118968 to 118375 (ORF 114r), 118508 to 119119 (ORF 115), 119588 to 120202 (ORF 116), 120314 to 21231 (ORF 117), 121380 to 123920 (ORF 118), 121288 to 122256 (ORF 119), 122350 to 123924 (ORF 120), 123962 to 125566 (ORF 121), 125193 to 124591 (ORF 122r), 125689 to 123935 (ORF 123r), 123839 to 123297 ORF 123ar), 125652 to 126170 (ORF 124), 126121 to 125699 (ORF 125r), 126279 to 127769 (ORF 126), 127851 to 128408 (ORF 127), 128520 to 130076 (ORF 128), 130105 to 131700 (ORF 129), 131790 to 133283 (ORF 130), 133246 to 133920 (ORF 131), 133972 to 134370 (ORF 132), 134418 to 134693 (ORF 133a), 134402 to 134992 (ORF R1), 134853 to 134419 (ORF R2r), 135628 to 135897 (ORF R3), 136780 to 137112 ORF R4), and 137558 to 137022 (ORF R5r) of SEQ ID 01,

alone or in combination with at least one other recombinant protein encoded by a polynucleotide selected from said group of polynucleotides, for the manufacture of a pharmaceutical composition.

8. Pharmaceutical composition comprising one or more recombinant proteins which can be expressed from polynucleotides selected from a group of polynucleotides consisting of

(a) a polynucleotide of sentence 1,

(b) a polynucleotide of sentence 2, and

(c) a polynucleotide with a sequence selected from a group of sequences consisting of nucleotides 3 to 539 (ORF L1), 781 to 449 (ORF L2r), 1933 to 1664 (ORF L3r), 3269 to 2790 (ORF L4r), 2799 to 3851 (ORF L5), 2962 to 3753 (ORF L6), 3784 to 3122 (ORF L7r), 4341 to 4129 (ORF L8r), 4904 to 4428 (ORF 1ar), 6517 to 4970 (ORF 1r), 8042 to 6684 (ORF 2r), 9989 to 8070 (ORF 3r), 11195 to 10062 ORF 4r), 11493 to 11227 (ORF 5r), 11802 to 12038 (ORF 6), 12358 to 12080 (ORF 7r), 13980 to 12364 (ORF 8r), 14826 to 14053 (ORF 9ar), 15080 to 15394 (ORF 10), 16838 to 15423 (ORF 11r), 19021 to 16847 (ORF 12r), 19704 to 19156 (ORF 13r), 20314 to 19736 (ORF 14r), 20401 to 22101 (ORF 15), 22125 to 22940 (ORF 6), 23003 to 23866 (ORF 17), 26908 to 23873 (ORF 18r), 26926 to 27213 (ORF 19), 27626 to 27216 (ORF 20r), 29754 to 27616 (ORF 21r), 32217 to 29800 (ORF 22r), 33380 to 32418 (ORF 23r), 33602 to 33393 (ORF 24r), 34466 to 33612 (ORF 25r), 34735 to 34502 (ORF 26r), 35905 to 34739 (ORF 27r), 37194 to 35905 (ORF 28r), 37200 to 39248 (ORF 29), 41037 to 39229 (ORF 30r), 41374 to 42066 (ORF 31), 42336 to 41731 (ORF 32r), 42407 to 41997 (ORF 33r), 42410 to 43765 (ORF 34), 43770 to 43958 (ORF 35), 43980 to 44534 (ORF 36), 45727 to 44537 (ORF 37r), 45760 to 46557 (ORF 38), 46567 to 47568 (ORF 39), 47572 to 48303 (ORF 40), 48352 to 48621 (ORF 41), 49887 to 48634 (ORF 42r), 49917 to 50693 (ORF 43), 50719 to 51102 (ORF 44), 51059 to 51511 (ORF 44a), 51584 to 52591 (ORF 45), 52509 to 53066 (ORF 46), 53523 to 53023 (ORF 47r), 53607 to 57473 (ORF 48), 58070 to 57528 (ORF 49r), 57700 to 58662 (ORF 50), 59674 to 58673 (ORF 51r), 62089 to 59678 (ORF 52r), 62198 to 62881 (ORF 53), 62909 to 63862 (ORF 55), 63858 to 64271 (ORF 56), 64309 to 66831 (ORF 57), 67266 to 66799 (ORF 58r), 67803 to 67273 (ORF 58ar), 67915 to 68607 (ORF 59), 68624 to 70984 (ORF 60), 70994 to 72898 (ORF 61), 72938 to 73507 (ORF 62), 73540 to 74211 (ORF 63), 76120 to 74207 (ORF 64r), 76749 to 76186 (ORF 65r), 77698 to 76799 (ORF 66r), 79343 to 77709 (ORF 67r), 79816 to 79367 (ORF 68r), 80529 to 79858 (ORF 69r), 80774 to 80529 (ORF 70r), 82815 to 80788 (ORF 71r), 83835 to 82834 (ORF 72r), 83874 to 85583 (ORF 73), 85535 to 84402 (ORF 74r), 88096 to 85574 (ORF 75r), 87759 to 88667 (ORF 76), 88920 to 88642 (ORF 77r), 91652 to 88938 (ORF 78r), 91667 to 92674 (ORF 79), 93466 to 92681 (ORF 80r), 93761 to 93486 (ORF 81r), 94060 to 93788 (ORF 82r), 94238 to 94080 (ORF 83r), 94508 to 94242 (ORF 84r), 95571 to 94498 (ORF 85r), 96187 to 95600 (ORF 86r), 96202 to 97665 (ORF 87), 97915 to 97643 (ORF 88r), 98251 to 99537 (ORF 89), 99537 to 99974 (ORF 90), 100001 to 101140 (ORF 91), 101168 to 104650 (ORF 92), 106354 to 104795 (ORF 93r), 107947 to 106400 (ORF 94r), 108256 to 107990 ORF 95r), 108719 to 108300 (ORF 96r), 109679 to 108738 (ORF 97r), 109861 to 109682 (ORF 98r), 110830 to 10033 (ORF 99r), 110208 to 110417 (ORF 100), 110469 to 110651 (ORF 100a), 110915 to 111397 (ORF 101), 111419 to 111913 (ORF 102), 111949 to 112485 (ORF 103), 112593 to 113450 (ORF 104), 113323 to 112967 ORF 105r), 113526 to 114152 (ORF 106), 114199 to 115236 (ORF 107), 115353 to 115787 (ORF 108), 115859 o 116551 (ORF 109), 116729 to 117523 (ORF 110), 117572 to 117114 (ORF 111r), 117423 to 118085 (ORF 12), 118968 to 118375 (ORF 114r), 118508 to 119119 (ORF 115), 119588 to 120202 (ORF 116), 120314 to 21231 (ORF 117), 121380 to 123920 (ORF 118), 121288 to 122256 (ORF 119), 122350 to 123924 (ORF 120), 123962 to 125566 (ORF 121), 125193 to 124591 (ORF 122r), 125689 to 123935 (ORF 123r), 123839 to 123297 ORF 123ar), 125652 to 126170 (ORF 124), 126121 to 125699 (ORF 125r), 126279 to 127769 (ORF 126), 127851 to 128408 (ORF 127), 128520 to 130076 (ORF 128), 130105 to 131700 (ORF 129), 131790 to 133283 (ORF 130), 133246 to 133920 (ORF 131), 133972 to 134370 (ORF 132), 134418 to 134693 (ORF 133a), 134402 to 134992 (ORF R1), 134853 to 134419 (ORF R2r), 135628 to 135897 (ORF R3), 136780 to 137112 ORF R4), and 137558 to 137022 (ORF R5r) of SEQ ID 01.

9. Use of recombinant proteins encoded by polynucleotides selected from a group of polynucleotides consisting of

(a) a polynucleotide of sentence 1,

(b) a polynucleotide of sentence 2, and

(c) a polynucleotide with a sequence selected from a group of sequences consisting of nucleotides 3 to 539 (ORF L1), 781 to 449 (ORF L2r), 1933 to 1664 (ORF L3r), 3269 to 2790 (ORF L4r), 2799 to 3851 (ORF L5), 2962 to 3753 (ORF L6), 3784 to 3122 (ORF L7r), 4341 to 4129 (ORF L8r), 4904 to 4428 (ORF 1ar), 6517 to 4970 (ORF 1r), 8042 to 6684 (ORF 2r), 9989 to 8070 (ORF 3r), 11195 to 10062 ORF 4r), 11493 to 11227 (ORF 5r), 11802 to 12038 (ORF 6), 12358 to 12080 (ORF 7r), 13980 to 12364 (ORF 8r), 14826 to 14053 (ORF 9ar), 15080 to 15394 (ORF 10), 16838 to 15423 (ORF 11r), 19021 to 16847 (ORF 12r), 19704 to 19156 (ORF 13r), 20314 to 19736 (ORF 14r), 20401 to 22101 (ORF 15), 22125 to 22940 (ORF 6), 23003 to 23866 (ORF 17), 26908 to 23873 (ORF 18r), 26926 to 27213 (ORF 19), 27626 to 27216 (ORF 20r), 29754 to 27616 (ORF 21r), 32217 to 29800 (ORF 22r), 33380 to 32418 (ORF 23r), 33602 to 33393 (ORF 24r), 34466 to 33612 (ORF 25r), 34735 to 34502 (ORF 26r), 35905 to 34739 (ORF 27r), 37194 to 35905 (ORF 28r), 37200 to 39248 (ORF 29), 41037 to 39229 (ORF 30r), 41374 to 42066 (ORF 31), 42336 to 41731 (ORF 32r), 42407 to 41997 (ORF 33r), 42410 to 43765 (ORF 34), 43770 to 43958 (ORF 35), 43980 to 44534 (ORF 36), 45727 to 44537 (ORF 37r), 45760 to 46557 (ORF 38), 46567 to 47568 (ORF 39), 47572 to 48303 (ORF 40), 48352 to 48621 (ORF 41), 49887 to 48634 (ORF 42r), 49917 to 50693 (ORF 43), 50719 to 51102 (ORF 44), 51059 to 51511 (ORF 44a), 51584 to 52591 (ORF 45), 52509 to 53066 (ORF 46), 53523 to 53023 (ORF 47r), 53607 to 57473 (ORF 48), 58070 to 57528 (ORF 49r), 57700 to 58662 (ORF 50), 59674 to 58673 (ORF 51r), 62089 to 59678 (ORF 52r), 62198 to 62881 (ORF 53), 62909 to 63862 (ORF 55), 63858 to 64271 (ORF 56), 64309 to 66831 (ORF 57), 67266 to 66799 (ORF 58r), 67803 to 67273 (ORF 58ar), 67915 to 68607 (ORF 59), 68624 to 70984 (ORF 60), 70994 to 72898 (ORF 61), 72938 to 73507 (ORF 62), 73540 to 74211 (ORF 63), 76120 to 74207 (ORF 64r), 76749 to 76186 (ORF 65r), 77698 to 76799 (ORF 66r), 79343 to 77709 (ORF 67r), 79816 to 79367 (ORF 68r), 80529 to 79858 (ORF 69r), 80774 to 80529 (ORF 70r), 82815 to 80788 (ORF 71r), 83835 to 82834 (ORF 72r), 83874 to 85583 (ORF 73), 85535 to 84402 (ORF 74r), 88096 to 85574 (ORF 75r), 87759 to 88667 (ORF 76), 88920 to 88642 (ORF 77r), 91652 to 88938 (ORF 78r), 91667 to 92674 (ORF 79), 93466 to 92681 (ORF 80r), 93761 to 93486 (ORF 81r), 94060 to 93788 (ORF 82r), 94238 to 94080 (ORF 83r), 94508 to 94242 (ORF 84r), 95571 to 94498 (ORF 85r), 96187 to 95600 (ORF 86r), 96202 to 97665 (ORF 87), 97915 to 97643 (ORF 88r), 98251 to 99537 (ORF 89), 99537 to 99974 (ORF 90), 100001 to 101140 (ORF 91), 101168 to 104650 (ORF 92), 106354 to 104795 (ORF 93r), 107947 to 106400 (ORF 94r), 108256 to 107990 ORF 95r), 108719 to 108300 (ORF 96r), 109679 to 108738 (ORF 97r), 109861 to 109682 (ORF 98r), 110830 to 10033 (ORF 99r), 110208 to 110417 (ORF 100), 110469 to 110651 (ORF 100a), 110915 to 111397 (ORF 101), 111419 to 111913 (ORF 102), 111949 to 112485 (ORF 103), 112593 to 113450 (ORF 104), 113323 to 112967 ORF 105r), 113526 to 114152 (ORF 106), 114199 to 115236 (ORF 107), 115353 to 115787 (ORF 108), 115859 o 116551 (ORF 109), 116729 to 117523 (ORF 110), 117572 to 117114 (ORF 111r), 117423 to 118085 (ORF 12), 118968 to 118375 (ORF 114r), 118508 to 119119 (ORF 115), 119588 to 120202 (ORF 116), 120314 to 21231 (ORF 117), 121380 to 123920 (ORF 118), 121288 to 122256 (ORF 119), 122350 to 123924 (ORF 120), 123962 to 125566 (ORF 121), 125193 to 124591 (ORF 122r), 125689 to 123935 (ORF 123r), 123839 to 123297 ORF 123ar), 125652 to 126170 (ORF 124), 126121 to 125699 (ORF 125r), 126279 to 127769 (ORF 126), 127851 to 128408 (ORF 127), 128520 to 130076 (ORF 128), 130105 to 131700 (ORF 129), 131790 to 133283 (ORF 130), 133246 to 133920 (ORF 131), 133972 to 134370 (ORF 132), 134418 to 134693 (ORF 133a), 134402 to 134992 (ORF R1), 134853 to 134419 (ORF R2r), 135628 to 135897 (ORF R3), 136780 to 137112 ORF R4), and 137558 to 137022 (ORF R5r) of SEQ ID 01,

and/or polynucleotides selected from said group of polynucleotides for the manufacture of a pharmaceutical composition for the treatment of virus related diseases, viral infections, non-viral infections, proliferative diseases, inflammatory diseases, allergic diseases, and/or autoimmune diseases.

10. Use of recombinant viruses comprising a *Vaccina lister* genome and fragments of a PPVO genome for the manufacture of pharmaceutial compositions.

11. Use of recombinant viruses comprising a *Vaccinia lister* genome and at least one heterologous gene to express at least one heterologous gene in a subject.

12. Use of a recombinant viruses comprising a *Vaccinia lister* genome and at least one heterologous gene for gene therapy.

13. A particle-like structure comprising a recombinant polypeptide encoded by an open reading frame (ORF) of the polynucleotide of SEQ ID NO: 1 or functional variants of said recombinant polypeptide.

14. Use of a particle-like structure of sentence 13 for the preparation of a medicament.

15. Use of a particle-like structure of sentence 13 for the preparation of a medicament for the treatment of virus related diseases, viral infections, non-viral infections, proliferative diseases, inflammatory diseases, allergic diseases, and/or autoimmune diseases.

16. A pharmaceutical composition comprising a particle-like structure of sentence 13.

17. A pharmaceutical composition comprising a particle-like structure of sentence 13 for the treatment of virus related diseases, viral infections, non-viral infections, proliferative diseases, inflammatory diseases, allergic diseases, and/or autoimmune diseases.

## Brief description of the figures

Figure 1 shows the genomic locations of the DNA fragments constituting the insertion library. The position of each DNA fragment is shown against the KpnI map of PPVO NZ2 (Mercer et al. 1997, Virology, 229: 193-200).

## Examples

Example 1: Determination of the integrated PPVO Fragments in the active VVOVs.

DNA preparation from *Vaccinia lister*/PPVO recombinants was performed as follows:

BK-KL 3A cells were grown to confluency in 175 $cm^2$ flasks (Becton Dickson Labware, Heidelberg, Germany). Cells were infected with a recombinant *Vaccina lister*/PPVO virus (VVOV) of Mercer et al. (1997, Virology, 229: 193-200) at a MOI (multiplicity of infection) of 0.01-0.32 and incubated at 37°C until 100 % CPE (cytopathic effect) had been reached. The infected cells were frozen at -80°C, thawed and processed as follows, with modification to the RNA extraction method of Vilcek et al. (1994, J. Clin. Microbiol. 32: 2225-2231). Using 2 ml PLG Heavy Eppendorf tubes (Eppendorf, Hamburg, Germany) 0.5 ml aliquots of cellular suspension were incubated with 100 μg Proteinase K (Roche Molecular Biochemicals, Mannheim, Germany) and 50 μl SDS (Sigma-Aldrich, Chemie GmbH, Taufkirchen, Germany) at 56°C for 25 min. 0.5 ml Roti®-Phenol/Chloroform (Carl Roth GmbH, Karlsruhe, Germany) was added and the tubes were inverted for several times. After centrifugation at 12000 x g for 10 min, the upper phase was transferred into a fresh tube and two volumes of ethanol (Merck Eurolab GmbH, Darmstadt, Germany) and 1/10 volume of sodium acetate (Sigma-Aldrich, Chemie GmbH, Taufkirchen, Germany) was added. The reagents were mixed several times and stored at -80°C for 3 h. The tubes were centrifuged at 14000 x g for 30 min, the supernatant was decanted and the pellet was air-dried for 5-10 min. Finally the DNA pellet was resuspended in 30 μl nuclease free water and stored at -20°C until used.

DNA concentration was measured spectrophotometrically on a Bio-Photometer 6131 (Eppendorf, Hamburg, Germany) at 260/280 nm nm. The DNA yield of different sample preparations spanned from 100 ng/ml up to 1 μg/ml.

Polymerase chain reaction (PCR) of terminal flanking regions of the integrated fragments in the *Vaccinia lister*/PPVO recombinants was performed as follows:

Three different PCR amplification systems were used for amplifying the terminal flanking regions. Each reaction mixture of 50 μl contained 100 ng - 1 μg resuspended DNA and primers (Table 1)) were added in a final concentration of 300 nM. Amplifications were carried out on a Mastercycler® gradient (Eppendorf, Hamburg, Germany).

The 3-prime flanking region of recombinant VVOV 285 had been analyzed using 2 x Ready-Mix™ PCR Master Mix (1.5 mM $MgCl_2$) (AB Gene, Hamburg, Germany). 1 μl BSA (MBI Fermentas GmbH, St. Leon-Rot, Germany) was added to each reaction. Denaturation was performed at 94°C for 3 min, followed by 30 cycles (94°C for 30 s, 58.7°C - 65.3°C for 30 s, 72°C for 1 min) and 72°C for 5 min.

The 5-prime flanking region of the PPVO insert of recombinant VVOV 285, the 3-prime flanking region of VVOV 97, and both terminal flanking regions of VVOV 215, VVOV 243, VVOV 245 were amplified using PfuTurbo® DNA Polymerase (Stratagene, Amsterdam, Netherlands). The reactions were setup with 2.5 U of enzyme, 1.5 mM $MgCl_2$ and 200 μM of each dNTP. Denaturation was performed at 94°C for 3 min, followed by 30 cycles (94°C for 30 s, 58.7°C - 65.3°C for 30 s, 72°C for 1 min) and 72°C for 5 min.

The amplification of the 5-prime flanking region of VVOV 97 and VVOV 82, the 3-prime flanking region of VVOV 96 and VVOV 283 were performed with Platinium® Pfx DNA Polymerase (Life Technologies GmbH, Karlsruhe, Germany). A reaction of 50 μl contained 1.25 U polymerase, 1-1.5 mM $MgCl_2$ and 300 μM of each dNTP. Additional use of PCRx Enhancer Solution was necessary for amplification of the 5-prime flanking regions of VVOV 96 (1x concentrated) and the 3-prime flanking regions of VVOV 82 (2x concentrated). Denaturation was performed at 94°C for 2 min, followed by 30 cycles (94°C for 15 s, 54.6°C - 60.7°C for 30 s, 68°C for 1-2 min) and 68°C for 5-7 min.

18 μl of each amplification product was analyzed by agarose gel electrophoresis on 1.5-2 % SeaKem LE agarose (Biozym, Hessisch Oldendorf, Germany). After staining in a ethidium bromide solution for 20 min the DNA fragments were visualized on an UV transilluminator UVT-20 M/W (Herolab, Wiesloch, Germany).

The sequence of the amplified DNA-fragments were determined by standard sequencing procedures and compared to the published *Vaccinia lister* thymidine kinase-sequence and the genome sequence of PPVO NZ2 to determine exactly the integrated PPVO NZ2 sequences.

PCR-primers, amplification and sequencing of the terminal flanking regions of the integrated fragments in the *Vaccinia lister*/PPVO NZ2 recombinants

**Table 1**

| VVOV | Amplified terminal region of NZ2 insert | Primers used for amplification | | Length of amplification product [bp] |
|---|---|---|---|---|
| | | Primer name | Sequence 5' → 3' | |
| VVOV 215 | 5' | VAC-P11-1 | ATTACAGTGATGCCTACATGCCG | 264 |
| | | PPVO 14r-1 | GCTGTAGTCGTGGTCCGGC | |
| | 3' | PPVO 4r-2 | CTTCCTAGGCTTCTACCGCACG | 402 |
| | | VAC-TK-1 | CGGTTTACGTTGAAATGTCCCAT | |
| VVOV 245 | 5' | VAC-P11-1 | ATTACAGTGATGCCTACATGCCG | 553 |
| | | PPVO 57-1 | CTGGCCAACGACGCCTTC | |
| | 3' | PPVO 40-1 | TCTGGTACCCCTTGCCGG | 321 |
| | | VAC-TK-1 | CGGTTTACGTTGAAATGTCCCAT | |
| VVOV 285 | 5' | VAC-P11-1 | ATTACAGTGATGCCTACATGCCG | 241 |
| | | PPVO 78r-5 | GAACCCGCTCTCGCTCGA | |
| | 3' | PPVO 64r-1 | GCCGGGCAAGTGTCTGGTC | 320 |
| | | VAC-TK-1 | CGGTTTACGTTGAAATGTCCCAT | |
| VVOV 330 | 5' | VAC-P11-1 | ATTACAGTGATGCCTACATGCCG | 392 |
| | | PPVO 92-1 | CTCGAAGTAGCTGATGTCGCG | |
| | 3' | PPVO 96r-1 | AGAGCTTTACGTAGACTCTCCAAGTGTC | 462 |
| | | VAC-TK-1 | CGGTTTACGTTGAAATGTCCCAT | |
| VVOV 96 | 5' | VAC-TK-fwd | ATACGGAACGGGACTATGGACG | 239 |
| | | PPVO 22r-3 | GCGGTGGCCATGTACGTG | |
| | 3' | PPVO 22r-4 | GGTTGTGGCGATGGTCGG | 1055 |
| | | VAC-TK-1 | CGGTTTACGTTGAAATGTCCCAT | |
| VVOV 97 | 5' | VAC-TK-fwd | ATACGGAACGGGACTATGGACG | 309 |
| | | PPVO 18r-1 | CTTGATGAGCCGGACGCA | |
| | 3' | PPVO 25r-1 | CCGAGTTGGAGAGGAAGGAGC | 318 |
| | | VAC-TK-1 | CGGTTTACGTTGAAATGTCCCAT | |
| VVOV 243 | 5' | VAC-P11-1 | ATTACAGTGATGCCTACATGCCG | 478 |
| | | PPVO 79-1 | CTGTTGGAGGATGAGGTCAAGGA | |
| | 3' | PPVO 71r-1 | CGTGCTCATGCCTGTGGAC | 269 |
| | | VAC-TK-1 | CGGTTTACGTTGAAATGTCCCAT | |

(continued)

| VVOV | Amplified terminal region of NZ2 insert | Primers used for amplification | | Length of amplification product [bp] |
|---|---|---|---|---|
| | | Primer name | Sequence 5' → 3' | |
| VVOV 283 | 5' | | | |
| | | | | |
| | 3' | PPVO 92-4 | CGACATCCTCACCTGCAAGAAG | 234 |
| | | VAC-TK-1 | CGGTTTACGTTGAAATGTCCCAT | |
| VVOV 82 | 5' | VAC-TK-fwd | ATACGGAACGGGACTATGGACG | 275 |
| | | PPVO 120-1 | TACAGGCAGCCCGTGACC | |
| | 3' | PPVO R3R4-3 | GCCGTGTGTCACGTTGATGC | 1960 |
| | | VAC-TK-1 | CGGTTTACGTTGAAATGTCCCAT | |

Example 2: Induction of interferon gamma and tumor necrosis factor alpha by PPVO gene products

The 16 recombinants were tested of their ability to induce tumor necrosis factor alpha (TNF-α) and interferon gamma (IFN-γ) in whole blood cultures.

Whole blood cultures containing blood and RPMI medium (Life Technologies GmbH, Karlsruhe, Germany) in the ratio of 1:5 were stimulated with the recombinant viruses. A pure *Vaccinia lister* and a whole PPVO preparation served as controls. All preparations were used at a final dilution of 1:10. The stimulation for the IFN-γ determination was done together with ConcanavalinA (SIGMA, St. Louis, MO), because the virus alone does not induce IFN-γ. Then the cells were incubated for 24 h (TNF-α) and or 72 h (IFN-γ). The cytokine concentration was then determined in the cell culture supernatants by TNF-α or IFN-γ specific ELISA. These time points were found to be optimal when the experimental conditions were determined using whole PPVO as a control.

It was possible to identify 5 active recombinant viruses (VVOV 96, VVOV 97, VVOV 243, VVOV 285, and VVOV 330) that induced both TNF-α and IFN-γ secretion and, thus, could mimic the effect of the whole PPVO. The results are depicted in Table 2.

TNF-α was determined after 24 h stimulation of blood cells with the recombinant virus or the controls, respectively. IFN-γ was determined after 72 h stimulation of blood cells with the recombinant virus or the controls. Stimulation was performed in the presence of the mitogen ConA. The relative induction in percent of the *Vaccinia* virus control is shown. Therefore, values greater than 100 % are due to the activity of the PPVO fragments. Active PPVO fragments are in bold. The data represent mean values of three different blood donors.

**Table 2**

| Recombinant Virus Clone or control | Interferon Induction (%) | TNF Induction (%) |
|---|---|---|
| Vaccinia virus control | 100 | 100 |
| NZ-2 control | 2224 | 264 |
| VVOV 80 | 200 | 66 |
| VVOV 82 | 173 | 65 |
| VVOV 85 | 209 | 94 |
| VVOV 86 | 138 | 73 |
| **VVOV 96** | **1638** | **1016** |
| **VVOV 97** | **1713** | **1285** |
| VVOV 212 | 94 | 62 |
| VVOV 213 | 192 | 38 |

(continued)

| Recombinant Virus Clone or control | Interferon Induction (%) | TNF Induction (%) |
|---|---|---|
| VVOV 215 | 97 | 82 |
| VVOV 216 | 197 | 71 |
| **VVOV 243** | **1446** | **933** |
| VVOV 245 | 98 | 45 |
| VVOV 247 | 85 | 74 |
| VVOV 283 | 115 | 78 |
| **VVOV 285** | **1128** | **1127** |
| **VVOV 330** | **1762** | **2135** |

The recombinant *Vaccinia lister*/ PPVO viruses that induce both interferon gamma and TNF-α expression are listed in column 1, the corresponding PPVO sequence in column 2 and all open reading frames (ORFs) that are completely or partially contained in the recombinant are depicted in column 3.

**Table 3**

| Active recombinant PPVO *Vaccinia* virus | PPVO NZ2 Sequence [Bp] that is contained in the recombinant | PPVO NZ2 ORFs that are contained in the recombinant |
|---|---|---|
| VVOV 97 | 24056 - 33789 | 18r - 25r |
| VVOV 96 | 31003 - 46845 | 22r - 39 |
| VVOV 285 | 74804 - 88576 | 64r - 76 |
| VVOV 243 | 82324 - 92502 | 71r - 79 |
| VVOV 330 | 102490 - 108393 | 92 - 96r |

**Example 3:** Local immunomodulation by PPVO gene products in liver sinus endothelial cells (LSEC)

We have established a new cell-based assay system that allows testing of hepatoprotective properties of recombinant PPVO proteins expressed in different systems (e.g., *Vaccinia* virus). This assay system uses primary murine liver cells, which play the central role in deciding whether immunity or tolerance is induced in the liver, the LSEC. The unique ability of LSEC to present exogenous antigens to CD8+ T cells on MHC class I molecules allows immune surveillance of hepatocytes as viral antigens released by infected hepatocytes are likely to be taken by LSEC and presented to cells of the immune system. The new assay allows to measure the ability of LSEC to interact antigen-specifically with CD8+ T cells, that are responsible for tissue destruction in necroinflammatory hepatitis.

Pure populations of LSEC are isolated from murine liver by a stepwise procedure of portal-vein perfusion with collagenase A (0.05 %), mechanical dispersion and further enzymatic digestion in a rotatory waterbath for 40 min at 37°C (245 rpm), gradient centrifugation (metrizamide 1.089g/$cm^3$) and centrifugal elutriation using a Beckman Avanti J25I centrifuge equipped with a JE-6B rotor and a standard elutriation chamber. LSEC cell populations isolated by this method are typically around 95-99 % pure as measured by uptake of endothelial cell specific substrate (acetylated low density lipoprotein). LSEC were seeded onto collagen type I coated 24 well tissue culture plates at a density of 100.000 cells per well and were further cultured in Dulbecco's modified Eagle Medium supplemented with 5 % fetal calf serum (specially tested not to interfere with the assay system) and 2 % glutamine. Three days after isolation, when LSEC gained a postmitotic and quiescient state, we tested for the ability of LSEC to present soluble ovalbumin to (ovalbumin-specific) CD8+ T cells. LSEC were incubated with 1 μM of ovalbumin for three hours (antigen dose and time were previously shown to be optimal for testing of substances suspected to influence antigen-presentation), washed and incubated with a CD8+ T cell hybridoma (200.000 cells/well) that recognizes the peptide SIINFEKL. SIINFEKL is recognized in a H2b context and directly binds on the MHC-I molecules. Therefore, it has not to be processed by the cell. This allows to differentiate between accessory functions of LSEC (such as MHC-I expression) and antigen-processing function.

The extent of CD8+ T cell activation was measured by determining the extent of IL-2 release from T cells by specific sandwich ELISA.

Using *Vaccinia* virus expressed recombinant proteins derived from PPVO we have been able to attribute hepatoprotective activity to individual clones. To be able to compare different clones directly with respect to their ability to influence cross-presentation by LSEC, we used equal amounts of "infectious units".

We found that LSEC cross-present exogenous ovalbumin very efficiently on MHC class I molecules ($k^b$) to CD8+ T cells. To our surprise we found if LSEC were incubated with several recombinant PPVO proteins we observed subsequently a potent downregulation of cross-presentation by more than 60 % compared to the mock-treated control that includes all but the active ingredient. Several regions within the genome of PPVO have immunregulatory properties. Especially the region termed 82 (43 % reduction) which is located at the 3' end of the genome appears to be responsible for the overall effect of PPVO on cross-presentation by LSEC. Further regions (VVOV 215, VVOV 212, VVOV 247 and VVOV 86) bear further immunregulatory potential, although to a lesser degree (around 30 % reduction in cross-presentation). It further appears that genes coding for proteins that downregulate cross-presentation are arranged in clusters. It is of interest to note that we identified two gene clusters coding for proteins that improved cross-presentation (VVOV 330, VVOV 283, VVOV 285, VVOV 97, and VVOV 96). However, for unknown reasons the downregulatory effect of the proteins mentioned above is dominant in the activity of PPVO on cross-presentation.

Our results strongly suggest that PPVO contains a mixture of different proteins that in a complementary way work to eliminate hepatocytes from hepatitis B virus while conserving hepatic integrity and avoiding long lasting damage secondary to hepatic fibrosis. As PPVO contains a gene with high homology to the anti-inflammatory agent IL-10 (located in the 5-prime region of the genome) we wondered whether the potent downregulatory effect of the clone 82 was due to expression of ovine IL-10. This assumes that there is cross-reactivity between murine and ovine IL-10 at the level of receptor recognition. We have been unable to demonstrate involvement of ovine IL-10 on the immunoregulatory potential of PPVO. Recombinant murine IL-10 did not show any influence on cross-presentation through LSEC and several monoclonal antibodies to murine and human IL-10 did not influence PPVO mediated downregulation of cross-presentation. We conclude that the immunoregulatory component of PPVO is probably not IL-10 but a new, so far not identified mediator. The data for the MHC-I cross-presentation - down-modulating recombinant virus are depicted in Table 4, those for the MHC-I cross-presentation - stimulating recombinant viruses in Table 5.

The recombinant *Vaccinia lister*/ PPVO virus that down-modulates the MHC-I cross presentation is designated in column 1, the corresponding PPVO sequence in column 2 and all open reading frames (ORFs) that are completely or partially contained in the recombinant are depicted in column 3.

**Table 4**

| Active recombinant PPVO Vaccinia virus | PPVO NZ2 Sequence [Bp] that is contained in the recombinant | PPVO NZ2 ORFs that are contained in the recombinant |
|---|---|---|
| VVOV 82 | 122616 - 136025 | 120-R3 |

The recombinant *Vaccinia lister*/ PPVO viruses that stimulate the MHC-I cross presentation are designated in column 1, the corresponding PPVO sequence in column 2 and all open reading frames (ORFs) that are completely or partially contained in the recombinant are depicted in column 3.

**Table 5**

| Active recombinant PPVO Vaccinia virus | PPVO NZ2-Sequence [Bp] that is contained in the recombinant | PPVO NZ2-ORFs that are contained in the recombinant |
|---|---|---|
| VVOV 97 | 24056 - 33789 | 18r - 25r |
| VVOV 96 | 31003 - 46845 | 22r - 39 |
| VVOV 285 | 74804 - 88576 | 64r - 76 |
| VVOV 283 | 89,4 - 103483 | 78r - 92 |
| VVOV 330 | 102490 - 108393 | 92 - 96r |

**Example 4:** Determination of the immunostimulatory activity of the Vaccinia lister / PPVO recombinants in the Aujeszky mouse model

We also tested the activity of recombinant *Vaccinia lister*/PPVO NZ2-viruses in the Aujeszky mouse model, a lethal challenge model of acute Suid Herpesvirus 1 disease for determining the activity of various immunostimulators (e.g. Baypamun®, CpG oligonucleotides).

a) Conditions employed for the mice

The NMRI mice (outbreed strain HdsWin:NMRI; female; weight: 18-20 g; obtained via Harlan/Winkelmann, Borchen, Germany) were kept in autoclavable polycarbonate crates lined with sawdust in an S2 isolation stall at 20-22°C (atmospheric humidity: 50-60 %) and subjected to an artificial day/night rhythm (illumination from 6:30 h to 18:30 h and darkness from 18:30 h to 6:30 h). They had free access to feed and water.

b) Challenge model

Groups of mice consisting of 10 mice per group were used for the tests. All of the animals in one group were given the same test substance.

After the mice were supplied they were kept in the animal stall for 2-3 days. Then the *Vaccinia lister*/PPVO NZ2 recombinants were diluted with PBS (Life Technologies GmbH, Karlsruhe, Germany) to a titer equivalent of approx. $10^8$ $TCID_{50}$/ml and thermally inactivated (twice for one hour at 58°C). Of these solutions 0.2 ml was administered per mouse intraperitoneally.

24 hours after the treatment the mice were infected with the pseudorabies virus of the Hannover H2 strain by intraperitoneal administration. For this purpose the virus was diluted in PBS to a test titer of approx. $10^4$ $TCID_{50}$/ml and 0.2 ml of this suspension was administered.

As a negative control one group of mice was treated with PBS and then infected. The mice in this group died 3-8 days after infection. A large proportion of the mice treated the *Vaccinia lister*/PPVO NZ2 recombinants VVOV 215, VVOV 245, VVOV 285 or VVOV 330 survived infection with the pseudorabies virus. 10 days after the infection with the virus the test was ended.

The level of induced immunostimulation was determined by comparing the number of dead mice in the PBS control group with the number of dead mice in the test groups and was quantified by the efficacy index (EI). This index indicates the percentage proportion of mice protected against the lethal effects of the Aujeszky virus infection through immune stimulation by the substance to be tested. It is calculated by means of the following formula:

$$EI = (b-a)/b \times 100,$$

where *b* is the percentage proportion of the dead mice in the control group and *a* the percentage proportion of the dead mice in the test group.

A chi-square test was used for the statistical evaluation. This test reveals the minimum activity indices indicating a significant difference between the mortality rate of those mice treated with the test substance and those treated with PBS. Activity indices of ≥ 60 % are significant where at least 5 of the mice used in tests with n=6 mice per group in the PBS control group and at least 7 of the mice used in tests with n=10 in the PBS control group do not survive the infection with the Aujeszky virus.

Altogether 3 separate tests were carried out in each case. The testing of *Vaccinia lister*/PPVO NZ2 recombinants in the Aujeszky mouse model shows the following:

Surprisingly, after the treatment of the mice with the *Vaccinia lister*/PPVO NZ2 recombinants VVOV 215, VVOV 245, VVOV 285 or VVOV 330 the average activity indices of higher than 60 % demonstrated immunostimulation. By contrast all of the other *Vaccinia lister*/PPVO NZ2 recombinants were ineffective. The data is summarized in Table 6.

The recombinant *Vaccinia lister*/ PPVO viruses that protected mice from herpesvirus induced death are designated in column 1, the corresponding PPVO sequence in column 2 and all open reading frames (ORFs) that are completely or partially contained in the recombinant are depicted in column 3.

**Table 6**

| **Active recombinant PPVO *Vaccinia* virus** | **PPVO NZ2-Sequence [Bp] that is contained in the recombinant** | **PPVO NZ2 ORFs that are contained in the recombinant** |
|---|---|---|
| VVOV 215 | 10264 - 20003 | 4r - 14r |
| VVOV 245 | 47952 - 66263 | 40r - 57 |
| VVOV 285 | 74804 - 88576 | 64r - 76 |
| VVOV 330 | 102490 - 108393 | 92 - 96r |

Sequences of the Parapox ovis open reading frames. ORFs the names of which end with "r" are encoded on the complementary DNA strand. Base pair positions in the "from" and "to" column are relative to SEQ ID 01.

**Table 7**

| ORF | from | to | N-term | C-term | Comment |
|---|---|---|---|---|---|
| L1 | 3 | 539 | IRGFAG | PQKVFRL | long termal repeat (LTR)-protein, retroviral pseudoprotease |
| L2r | 781 | 449 | MSEGGRL | LLGLLFP | LTR-protein, retroviral pseudopro-tease |
| L3r | 1933 | 1664 | MTVHPPK | VLPPNSL | LTR-protein, retroviral pseudopro-tease |
| L4r | 3269 | 2790 | MHPSPRR | PVSHPFL | LTR-protein, retroviral pseudopro-tease |
| L5 | 2799 | 3851 | MGDREGE | FEDGVKC | LTR-protein, retroviral pseudopro-tease |
| L6 | 2962 | 3753 | MCTVATF | GAPRAGW | LTR-protein, similar to 134r, retroviral pseudoprotease |
| L7r | 3784 | 3122 | MTPTSRE | ARTAPPR | LTR-protein, retroviral pseudopro-tease |
| L8r | 4341 | 4129 | MPGEGQY | NGGLGKI | LTR-protein, retroviral pseudopro-tease |
| 1ar | 4904 | 4428 | MEFCHTE | DTAWYIS | dUTPase |
| 1r | 6517 | 4970 | MLSRESV | RAMLTRP | homolog of G1L in NZ2, Ankyrin-repeats |
| 2r | 8042 | 6684 | MFFWFWC | SGEGVPV | |
| 3r | 9989 | 8070 | MLGFWGK | VLPSVSR | involved in maturation of EEV (Extracellular Enveloped Virions) |
| 4r | 11195 | 10062 | MWPFSSI | EFCKPIN | Phospholipase D-type enzyme |
| 5r | 11493 | 11227 | MLIYGPR | RLLKDFP | homolog of B3L in NZ2 |
| 6 | 11802 | 12038 | MGVVMCG | APAGVTE | |
| 7r | 12358 | 12080 | MPVKVKQ | ASREFIV | ubiquitination protein with RING-finger-motiv (related to yeast proteins APC11 and HRT1) |
| 8r | 13980 | 12364 | MEEELTR | SPMVVFN | no Vaccinia virus homolog |
| 9ar | 14826 | 14053 | MIRIGGG | DNMRVDD | |
| 10 | 15080 | 15394 | MDGGVHK | EQMCRRQ | virion core DNA-binding phos-phoprotein |
| 11r | 16838 | 15423 | MAPPVIE | AKNVITH | polyA polymerase |
| 12r | 19021 | 16847 | MLQLLKR | NNRGFRK | |
| 13r | 19704 | 19156 | MACECAS | NNCGISF | interferon resistance protein, homology to mammalian PACT (protein activator of the inter-feron-induced protein kinase) also called PRKRA (dsRNA dependent activator of Interferon-induced protein kinase), 13r-protein con-tains a dsRBD motiv (double-stranded RNA binding domain) and a 'DRADA'-domain that is typical for RNA-editing enzymes) |
| 14r | 20314 | 19736 | MDEDRLR | KKGKPKS | RNA polymerase |
| 15 | 20401 | 22101 | MDFVRRK | VVLQGRA | |
| 16 | 22125 | 22940 | MVDSGTH | PENVVLL | |
| 17 | 23003 | 23866 | MASYISG | RTHTVYV | |
| 18r | 26908 | 23873 | MLFEMEL | SKPVFTG | DNA polymerase |

(continued)

| ORF | from | to | N-term | C-term | Comment |
|---|---|---|---|---|---|
| 19 | 26926 | 27213 | MEPRFWG | AKVRPLV | distant homolog of the ERV1/ALR-protein-family (ERV1: yeast pro-tein, Essential for Respiration and Vegatative growth, ALR: mammal-ian protein, Augmenter of Liver Regeneration) |
| 20r | 27626 | 27216 | MEAINVF | RAYEGML | |
| 21r | 29754 | 27616 | MLLYPKK | LLGDGGD | related to 12r |
| 22r | 32217 | 29800 | MLIRTTD | EAQNMQN | |
| 23r | 33380 | 32418 | MEDERLI | PSPCGGE | |
| 24r | 33602 | 33393 | MDKLYTG | FHYLKLV | |
| 25r | 34466 | 33612 | MKRAVSK | LEAPFNI | DNA binding phosphoprotein |
| 26r | 34735 | 34502 | MESRDLG | LNARRQN | |
| 27r | 35905 | 34739 | MNHFFKQ | RSLYTVL | |
| 28r | 37194 | 35905 | MDKYTDL | PEKPAAP | core protein |
| 29 | 37200 | 39248 | MENHLPD | IEAEPPF | RNA helicase |
| 30r | 41037 | 39229 | MIVLENG | RMGARPR | Zn-protease, involved in virion morphogenesis |
| 31 | 41374 | 42066 | MTFRELI | DSMASRS | late transcription factor |
| 32r | 42336 | 41731 | MRGHPAH | VAPREEL | |
| 33r | 42407 | 41997 | MASDASP | QPSSSRR | Glutaredoxin-like enzyme |
| 34 | 42410 | 43765 | MGIKNLK | PRLLKLR | |
| 35 | 43770 | 43958 | MVFPIVC | LPMLDIS | RNA polymerase |
| 36 | 43980 | 44534 | MREFGLA | AEPPWLV | |
| 37r | 45727 | 44537 | MESSKQA | TRAPPLF | core virion protein precursor |
| 38 | 45760 | 46557 | MTLRIKL | DRSLSCD | late transcription factor |
| 39 | 46567 | 47568 | MGGSVSL | YLLIVWL | |
| 40 | 47572 | 48303 | MGAAASI | TEFPPSV | virion protein, related to vaccinia F9L |
| 41 | 48352 | 48621 | MVRRVLL | LCLFSMD | |
| 42r | 49887 | 48634 | MEEKRGR | ARAMVCL | |
| 43 | 49917 | 50693 | MTNLLSL | TGAEAAP | core protein, DNA binding domain |
| 44 | 50719 | 51102 | MAAPTTP | VDVLGGR | |
| 44a | 51059 | 51511 | MDHEKYV | ATLSPGL | |
| 45 | 51584 | 52591 | MEGVEMD | RPLRGGK | polyA polymerase |
| 46 | 52509 | 53066 | MNRHNTR | SVSVVLD | RNA polymerase |
| 47r | 53523 | 53023 | MFFRRRA | GRRPPRP | |
| 48 | 53607 | 57473 | MSVVARV | EAAEEEF | RNA polymerase chain 1 |
| 49r | 58070 | 57528 | MGDKSEW | FVCDSPS | tyrosine phosphatase |
| 50 | 57700 | 58662 | MAAAPLR | ATSGVLT | |
| 51r | 59674 | 58673 | MDPPEIT | LLVTAIV | immunodominant envelope protein |
| 52r | 62089 | 59678 | MDSRESI | YMINFNN | RNA polymerase-associated tran-scription specificity factor (also called RAP94) |

(continued)

| ORF | from | to | N-term | C-term | Comment |
|---|---|---|---|---|---|
| 53 | 62198 | 62881 | MSSWRLK | KAAACKK | late transcription factor |
| 55 | 62909 | 63862 | MRALHLS | NSEQVNG | topoisomerase I |
| 56 | 63858 | 64271 | MDEALRV | FIRAAVA | |
| 57 | 64309 | 66831 | MDAPSLD | LYVFSKR | mRNA capping enzyme |
| 58r | 67266 | 66799 | MEPSAMR | DVQHVDL | virion protein |
| 58ar | 67803 | 67273 | MAGFSQS | TTCVPPQ | |
| 59 | 67915 | 68607 | MATPANA | FSFYSEN | Uracil DNA glycosylase |
| 60 | 68624 | 70984 | MAAPICD | IEDVENK | ATPase, involved in DNA replica-tion |
| 61 | 70994 | 72898 | MNSDVIK | EVSVVNI | early transcription factor |
| 62 | 72938 | 73507 | MSTFRQT | ASPAAKN | RNA polymerase |
| 63 | 73540 | 74211 | MRTYTSL | WGAAVTR | NTP pyrophosphohydrolase |
| 64r | 76120 | 74207 | MTSAHAA | VDPASIA | virion NTPase |
| 65r | 76749 | 76186 | MEGRARF | RFCNYCP | |
| 66r | 77698 | 76799 | MKTDCAS | KLKLLLQ | mRNA capping enzyme |
| 67r | 79343 | 77709 | MNNSVVS | AEKVTAQ | rifampicin resistance, virion membrane |
| 68r | 79816 | 79367 | MKRIALS | MALKSLI | late transactivator protein |
| 69r | 80529 | 79858 | MNLRMCG | AACSLDL | late transactivator protein |
| 70r | 80774 | 80529 | MGDNVWF | VLGLEQA | thioredoxin-like protein |
| 71r | 82815 | 80788 | MESPACA | NMCDVLC | major core protein |
| 72r | 83835 | 82834 | MDLRRRF | VDNTGTS | core protein |
| 73 | 83874 | 85583 | MEESVAV | LLNYGCG | RNA-polymerase |
| 74r | 85535 | 84402 | MDRLRTC | AEAAESA | |
| 75r | 88096 | 85574 | MVSVMRK | QEFYPQP | early transcription factor |
| 76 | 87759 | 88667 | MFQPVPD | SACRASP | |
| 77r | 88920 | 88642 | MRPCYVT | TRGTQTG | |
| 78r | 91652 | 88938 | MTAPNVH | AVSFDSE | major core protein |
| 79 | 91667 | 92674 | MTAVPVT | VRKLNLI | |
| 80r | 93466 | 92681 | MASEKMA | DLDGGMC | virion protein |
| 81r | 93761 | 93486 | MGLLDAL | RFSAASS | virion membrane protein |
| 82r | 94060 | 93788 | MDIFETL | DIELTAR | virion membrane protein |
| 83r | 94238 | 94080 | MVSDYDP | HFVHSVI | |
| 84r | 94508 | 94242 | MFLDSDT | DMPFSVV | |
| 85r | 95571 | 94498 | MGDTVSK | KTINVSR | |
| 86r | 96187 | 95600 | MESYFSY | EDLFFAE | virion membrane protein |
| 87 | 96202 | 97665 | MFGGVQV | GRDLAAV | RNA helicase |
| 88r | 97915 | 97643 | MSAVKAK | PLRDLAR | Zn-finger protein |
| 89 | 98251 | 99537 | MTSESDL | AIARAQP | DNA polymerase processivity factor |
| 90 | 99537 | 99974 | MIVAAFD | NYVLRTN | |

(continued)

| ORF | from | to | N-term | C-term | Comment |
|---|---|---|---|---|---|
| 91 | 100001 | 101140 | MLALFEF | LKELLGP | intermediate transcription factor |
| 92 | 101168 | 104650 | MEQALGY | SLFSPED | RNA polymerase b-chain |
| 93r | 106354 | 104795 | MESDNAL | GQHAAIW | A-type inclusion body/Fusion peptide |
| 94r | 107947 | 106400 | MEKLVSD | GRSGAIW | A-type inclusion body/Fusion peptide |
| 95r | 108256 | 107990 | MDENDGE | QTGYSRY | viral fusion protein |
| 96r | 108719 | 108300 | MDAVSAL | LFLKSIL | |
| 97r | 109679 | 108738 | MADAPLV | RELRANE | RNA polymerase subunit |
| 98r | 109861 | 109682 | MEEDLNE | MGQASSA | |
| 99r | 110830 | 110033 | MDVVQEV | ADSDGGN | ATPase |
| 100 | 110208 | 110417 | MRSWFWQ | PLTGMCL | |
| 100a | 110469 | 110651 | MRPKSVG | SGHTKPS | |
| 101 | 110915 | 111397 | MAHNTFE | KYFCVSD | enveloped virion glycoprotein |
| 102 | 111419 | 111913 | MGCCKVP | CMKEMHG | enveloped virion glycoprotein |
| 103 | 111949 | 112485 | MSRLQIL | RKLDVPI | |
| 104 | 112593 | 113450 | MKAVLLL | LNLNPGN | GM-CSF/IL-2 inhibition factor |
| 105r | 113323 | 112967 | MHASLSS | DETLTYR | |
| 106 | 113526 | 114152 | MEVLVII | GEFFYDE | |
| 107 | 114199 | 115236 | MPLFRKL | RDALDGL | |
| 108 | 115353 | 115787 | MACFIEL | TTFSSSE | |
| 109 | 115859 | 116551 | MSSSSSETT | TTGTSTS | |
| 110 | 116729 | 117523 | MACLRVF | CSMQTAR | GM-CSF/IL-2 inhibition factor |
| 111r | 117572 | 117114 | MAIAHTT | FRFRTPG | |
| 112 | 117423 | 118085 | MAATIQI | KRDGYSR | |
| 114r | 118968 | 118375 | MEGLMPK | RPISVQK | |
| 115 | 118508 | 119119 | MDSRRLA | LGDSDSD | |
| 116 | 119588 | 120202 | MRLILAL | PQMMRIG | |
| 117 | 120314 | 121231 | MAGFLGA | CKVEEVL | |
| 118 | 121380 | 123920 | MHLHKDP | LAFPSLA | |
| 119 | 121288 | 122256 | MANRLVF | RPMEIDG | |
| 120 | 122350 | 123924 | MENNDGN | RFLPSHK | related to 1r/G1L with Ankyrin-repeats |
| 121 | 123962 | 125566 | MDPAGQR | CSETDRW | |
| 122r | 125193 | 124591 | MSSSAAA | IAPDSRM | |
| 123r | 125689 | 123935 | MTAEASI | DPVYHKK | |
| 123ar | 123839 | 123297 | MPRTTSG | REQTEGL | |
| 124 | 125652 | 126170 | MANREEI | VRVLRRT | |
| 125r | 126121 | 125699 | MTAPTPR | AAYSLAR | |
| 126 | 126279 | 127769 | MADEREA | LACAMRK | related to 1r/G1L with Ankyrin-repeats |
| 127 | 127851 | 128408 | MSKNKIL | SYMTTKM | sheep-like Interleukin 10 |

(continued)

| ORF | from | to | N-term | C-term | Comment |
|---|---|---|---|---|---|
| 128 | 128520 | 130076 | MLTRCYI | RASGLAE | related to 1r/G1L wih Ankyrin-repeats |
| 129 | 130105 | 131700 | MVGFDRR | CGRRAPE | related to 1r/G1L, with Ankyrin-repeats (NT slightly changed) |
| 130 | 131790 | 133283 | MILARAG | PDAAALS | Kinase |
| 131 | 133246 | 133920 | MPPRTPP | RPAALRA | |
| 132 | 133972 | 134370 | MKLLVGI | RPPRRRR | homolog to the sheep VEGF (Vascular Endothelial Growth Factor) |
| 133a | 134418 | 134693 | MRKKAPR | ARTAPPR | corresponds to L7r |
| R1 | 134402 | 134992 | MMRSGHA | RMHRSEL | LTR-protein (corresponds to L4r), retroviral pseudoprotease |
| R2r | 134853 | 134419 | MCTVATF | SVAPSSA | LTR-protein (corresponds to L6, 134r), retroviral pseudoprotease |
| R3 | 135628 | 135897 | MTVHPPK | VLPPNSL | LTR-protein (corresponds to L3r), retroviral pseudoprotease |
| R4 | 136780 | 137112 | MSEGGRL | LLGLLFP | LTR-protein (corresponds to L2r), retroviral pseudoprotease |
| R5r | 137558 | 137022 | IRGFAGG | PQKVFRL | LTR-protein (corresponds to L1r), retroviral pseudoprotease |

SEQUENCE LISTING

<110> Bayer AG, Leverkusen, Germany

<120> Recombinant proteins of Parapoxvirus ovis and pharmaceutical compositions therefrom.

<130> LeA 35228

<160> 1
<170> PatentIn version 3.0

<210> 1
<211> 137560
<212> DNA
<213> Parapoxvirus ovis NZ2
<220>
<221> CDS
<222> (3)...(539)
<223> ORF: L1
<220>
<221> CDS
<222> (781)...(449)
<223> ORF: L2r
<220>
<221> CDS
<222> (1933)...(1664)
<223> ORF: L3r
<220>
<221> CDS
<222> (3269)...(2790)
<223> ORF: L4r
<220>
<221> CDS
<222> (2799)...(3851)
<223> ORF: L5
<220>
<221> CDS
<222> (2962)...(3753)
<223> ORF: L6
<220>
<221> CDS
<222> (3784)...(3122)
<223> ORF: L7r
<220>
<221> CDS
<222> (4341)...(4129)
<223> ORF: L8r
<220>
<221> CDS
<222> (4904)...(4428)
<223> ORF: 1ar
<220>
<221> CDS
<222> (6517)...(4970)
<223> ORF: 1r
<220>
<221> CDS
<222> (8042)...(6684)
<223> ORF: 2r
<220>
<221> CDS

<222> (9989)...(8070)
<223> ORF: 3r
<220>
<221> CDS
<222> (11195)...(10062)
<223> ORF: 4r
<220>
<221> CDS
<222> (11493)...(11227)
<223> ORF: 5r
<220>
<221> CDS
<222> (11802)...(12038)
<223> ORF: 6
<220>
<221> CDS
<222> (12358)...(12080)
<223> ORF: 7r
<220>
<221> CDS
<222> (13980)...(12364)
<223> ORF: 8r
<220>
<221> CDS
<222> (14826)...(14053)
<223> ORF: 9ar
<220>
<221> CDS
<222> (15080)...(15394)
<223> ORF: 10
<220>
<221> CDS
<222> (16838)...(15423)
<223> ORF: 11r
<220>
<221> CDS
<222> (19021)...(16847)
<223> ORF: 12r
<220>
<221> CDS
<222> (19704)...(19156)
<223> ORF: 13r
<220>
<221> CDS
<222> (20314)...(19736)
<223> ORF: 14r
<220>
<221> CDS
<222> (20401)...(22101)
<223> ORF: 15
<220>
<221> CDS
<222> (22125)...(22940)
<223> ORF: 16
<220>
<221> CDS
<222> (23003)...(23866)
<223> ORF: 17
<220>
<221> CDS
<222> (26908)...(23873)
<223> ORF: 18r
<220>

<221> CDS
<222> (26926)...(27213)
<223> ORF: 19
<220>
<221> CDS
<222> (27626)...(27216)
<223> ORF: 20r
<220>
<221> CDS
<222> (29754)...(27616)
<223> ORF: 21r
<220>
<221> CDS
<222> (32217)...(29800)
<223> ORF: 22r
<220>
<221> CDS
<222> (33380)...(32418)
<223> ORF: 23r
<220>
<221> CDS
<222> (33602)...(33393)
<223> ORF: 24r
<220>
<221> CDS
<222> (34466)...(33612)
<223> ORF: 25r
<220>
<221> CDS
<222> (34735)...(34502)
<223> ORF: 26r
<220>
<221> CDS
<222> (35905)...(34739)
<223> ORF: 27r
<220>
<221> CDS
<222> (37194)...(35905)
<223> ORF: 28r
<220>
<221> CDS
<222> (37200)...(39248)
<223> ORF: 29
<220>
<221> CDS
<222> (41037)...(39229)
<223> ORF: 30r
<220>
<221> CDS
<222> (41374)...(42066)
<223> ORF: 31
<220>
<221> CDS
<222> (42336)...(41731)
<223> ORF: 32r
<220>
<221> CDS
<222> (42407)...(41997)
<223> ORF: 33r
<220>
<221> CDS
<222> (42410)...(43765)
<223> ORF: 34

<220>
<221> CDS
<222> (43770)...(43958)
<223> ORF: 35
<220>
<221> CDS
<222> (43980)...(44534)
<223> ORF: 36
<220>
<221> CDS
<222> (45727)...(44537)
<223> ORF: 37r
<220>
<221> CDS
<222> (45760)...(46557)
<223> ORF: 38
<220>
<221> CDS
<222> (46567)...(47568)
<223> ORF: 39
<220>
<221> CDS
<222> (47572)...(48303)
<223> ORF: 40
<220>
<221> CDS
<222> (48352)...(48621)
<223> ORF: 41
<220>
<221> CDS
<222> (49887)...(48634)
<223> ORF: 42r
<220>
<221> CDS
<222> (49917)...(50693)
<223> ORF: 43
<220>
<221> CDS
<222> (50719)...(51102)
<223> ORF: 44
<220>
<221> CDS
<222> (51059)...(51511)
<223> ORF: 44a
<220>
<221> CDS
<222> (51584)...(52591)
<223> ORF: 45
<220>
<221> CDS
<222> (52509)...(53066)
<223> ORF: 46
<220>
<221> CDS
<222> (53523)...(53023)
<223> ORF: 47r
<220>
<221> CDS
<222> (53607)...(57473)
<223> ORF: 48
<220>
<221> CDS
<222> (58070)...(57528)

<223> ORF: 49r
<220>
<221> CDS
<222> (57700)...(58662)
<223> ORF: 50
<220>
<221> CDS
<222> (59674)...(58673)
<223> ORF: 51r
<220>
<221> CDS
<222> (62089)...(59678)
<223> ORF: 52r
<220>
<221> CDS
<222> (62198)...(62881)
<223> ORF: 53
<220>
<221> CDS
<222> (62909)...(63862)
<223> ORF: 55
<220>
<221> CDS
<222> (63858)...(64271)
<223> ORF: 56
<220>
<221> CDS
<222> (64309)...(66831)
<223> ORF: 57
<220>
<221> CDS
<222> (67266)...(66799)
<223> ORF: 58r
<220>
<221> CDS
<222> (67803)...(67273)
<223> ORF: 58ar
<220>
<221> CDS
<222> (67915)...(68607)
<223> ORF: 59
<220>
<221> CDS
<222> (68624)...(70984)
<223> ORF: 60
<220>
<221> CDS
<222> (70994)...(72898)
<223> ORF: 61
<220>
<221> CDS
<222> (72938)...(73507)
<223> ORF: 62
<220>
<221> CDS
<222> (73540)...(74211)
<223> ORF: 63
<220>
<221> CDS
<222> (76120)...(74207)
<223> ORF: 64r
<220>
<221> CDS

<222> (76749)...(76186)
<223> ORF: 65r
<220>
<221> CDS
<222> (77698)...(76799)
<223> ORF: 66r
<220>
<221> CDS
<222> (79343)...(77709)
<223> ORF: 67r
<220>
<221> CDS
<222> (79816)...(79367)
<223> ORF: 68r
<220>
<221> CDS
<222> (80529)...(79858)
<223> ORF: 69r
<220>
<221> CDS
<222> (80774)...(80529)
<223> ORF: 70r
<220>
<221> CDS
<222> (82815)...(80788)
<223> ORF: 71r
<220>
<221> CDS
<222> (83835)...(82834)
<223> ORF: 72r
<220>
<221> CDS
<222> (83874)...(85583)
<223> ORF: 73
<220>
<221> CDS
<222> (85535)...(84402)
<223> ORF: 74r
<220>
<221> CDS
<222> (88096)...(85574)
<223> ORF: 75r
<220>
<221> CDS
<222> (87759)...(88667)
<223> ORF: 76
<220>
<221> CDS
<222> (88920)...(88642)
<223> ORF: 77r
<220>
<221> CDS
<222> (91652)...(88938)
<223> ORF: 78r
<220>
<221> CDS
<222> (91667)...(92674)
<223> ORF: 79
<220>
<221> CDS
<222> (93466)...(92681)
<223> ORF: 80r
<220>

<221> CDS
<222> (93761)...(93486)
<223> ORF: 81r
<220>
<221> CDS
<222> (94060)...(93788)
<223> ORF: 82r
<220>
<221> CDS
<222> (94238)...(94080)
<223> ORF: 83r
<220>
<221> CDS
<222> (94508)...(94242)
<223> ORF: 84r
<220>
<221> CDS
<222> (95571)...(94498)
<223> ORF: 85r
<220>
<221> CDS
<222> (96187)...(95600)
<223> ORF: 86r
<220>
<221> CDS
<222> (96202)...(97665)
<223> ORF: 87
<220>
<221> CDS
<222> (97915)...(97643)
<223> ORF: 88r
<220>
<221> CDS
<222> (98251)...(99537)
<223> ORF: 89
<220>
<221> CDS
<222> (99537)...(99974)
<223> ORF: 90
<220>
<221> CDS
<222> (100001)...(101140)
<223> ORF: 91
<220>
<221> CDS
<222> (101168)...(104650)
<223> ORF: 92
<220>
<221> CDS
<222> (106354)...(104795)
<223> ORF: 93r
<220>
<221> CDS
<222> (107947)...(106400)
<223> ORF: 94r
<220>
<221> CDS
<222> (108256)...(107990)
<223> ORF: 95r
<220>
<221> CDS
<222> (108719)...(108300)
<223> ORF: 96r

<220>
<221> CDS
<222> (109679)...(108738)
<223> ORF: 97r
<220>
<221> CDS
<222> (109861)...(109682)
<223> ORF: 98r
<220>
<221> CDS
<222> (110830)...(110033)
<223> ORF: 99r
<220>
<221> CDS
<222> (110208)...(110417)
<223> ORF: 100
<220>
<221> CDS
<222> (110469)...(110651)
<223> ORF: 100a
<220>
<221> CDS
<222> (110915)...(111397)
<223> ORF: 101
<220>
<221> CDS
<222> (111419)...(111913)
<223> ORF: 102
<220>
<221> CDS
<222> (111949)...(112485)
<223> ORF: 103
<220>
<221> CDS
<222> (112593)...(113450)
<223> ORF: 104
<220>
<221> CDS
<222> (113323)...(112967)
<223> ORF: 105r
<220>
<221> CDS
<222> (113526)...(114152)
<223> ORF: 106
<220>
<221> CDS
<222> (114199)...(115236)
<223> ORF: 107
<220>
<221> CDS
<222> (115353)...(115787)
<223> ORF: 108
<220>
<221> CDS
<222> (115859)...(116551)
<223> ORF: 109
<220>
<221> CDS
<222> (116729)...(117523)
<223> ORF: 110
<220>
<221> CDS
<222> (117572)...(117114)

<223> ORF: 111r
<220>
<221> CDS
<222> (117423)...(118085)
<223> ORF: 112
<220>
<221> CDS
<222> (118968)...(118375)
<223> ORF: 114r
<220>
<221> CDS
<222> (118508)...(119119)
<223> ORF: 115
<220>
<221> CDS
<222> (119588)...(120202)
<223> ORF: 116
<220>
<221> CDS
<222> (120314)...(121231)
<223> ORF: 117
<220>
<221> CDS
<222> (121380)...(123920)
<223> ORF: 118
<220>
<221> CDS
<222> (121288)...(122256)
<223> ORF: 119
<220>
<221> CDS
<222> (122350)...(123924)
<223> ORF: 120
<220>
<221> CDS
<222> (123962)...(125566)
<223> ORF: 121
<220>
<221> CDS
<222> (125193)...(124591)
<223> ORF: 122r
<220>
<221> CDS
<222> (125689)...(123935)
<223> ORF: 123r
<220>
<221> CDS
<222> (123839)...(123297)
<223> ORF: 123ar
<220>
<221> CDS
<222> (125652)...(126170)
<223> ORF: 124
<220>
<221> CDS
<222> (126121)...(125699)
<223> ORF: 125r
<220>
<221> CDS
<222> (126279)...(127769)
<223> ORF: 126
<220>
<221> CDS

<222> (127851)...(128408)
<223> ORF: 127
<220>
<221> CDS
<222> (128520)...(130076)
<223> ORF: 128
<220>
<221> CDS
<222> (130105)...(131700)
<223> ORF: 129
<220>
<221> CDS
<222> (131790)...(133283)
<223> ORF: 130
<220>
<221> CDS
<222> (133246)...(133920)
<223> ORF: 131
<220>
<221> CDS
<222> (133972)...(134370)
<223> ORF: 132
<220>
<221> CDS
<222> (134418)...(134693)
<223> ORF: 133a
<220>
<221> CDS
<222> (134402)...(134992)
<223> ORF: R1
<220>
<221> CDS
<222> (134853)...(134419)
<223> ORF: R2r
<220>
<221> CDS
<222> (135628)...(135897)
<223> ORF: R3
<220>
<221> CDS
<222> (136780)...(137112)
<223> ORF: R4
<220>
<221> CDS
<222> (137558)...(137022)
<223> ORF: R5r

<400> 1
ggatccgcgg cttcgcgggc ggcggccggc tcccgcggcg gctgagccgc ggtgccgcga 60
cgaacgcgga ccaggagttc ctgcgggagg agctacggca gaggctggaa ctgctgaatg 120
ctttcgagga cgggcgtccg cgggaacgcg actccgcgga ggcggcaccc cgcagccgcg 180
agacctcgct ctggagtcag ggagtcaggg agtcgggagt cagggagtcg ggagtcaggg 240
agtcgggagt cagggagtcg ggagtcaggg agtcgggagt cagggagtcg ggagtcaggg 300
agtcgggagt cagggagtcg ggagtcaggg agtcgggagt cagggagtcg ggagtcaggg 360
agtcgggagt cagggagtcg ggagtcaggg agtcgggagt cagggagtcg ggagtcaggg 420
agtcgggagg tcagggagtc gggagtcagg gaaacagaag tccaagtagt acgggtgaga 480
caggagtcag gtggtcgggt gaccgcgccg tccgagtccc gcaaaaagtt tttagactct 540
gagagaggcc gacctgcctc caacggttcc gcggaaaagt ttttataaaa agttttcggg 600
agaggccgac tgccttccaa cggttcctcc tctcgcgtgc cgcgggcggc cgctctcccg 660
cgacggtccc gccaacgcgt ttacaaagtt ttaaaagttt tcgagagagg ccgacctgtc 720
ttccaacggt tgcgcgaaaa ggttctgcgg aggtttggaa gagccgcccg ccctccgaca 780
tcctccgaaa agttttcgca aaaagttttt aaaggtttcg cgaggagttt tcgagggagg 840
cgacctgcct ccgaggttcc gcgtaaacgt ttttacaaag cgtcggaggt gggggcgacc 900

```
tgcccttcct ctcctccgaa aagctcttga gagtgtggga gaggcggccg gccttcgcgg   960
tcgcgcgaaa aggttctgcg ggagttcgcg ggagctgacc cgccctccgc gcccctccga  1020
aaagtttttg cacgaagtct tttggcgttc tcgagaggac gcctaccccg acggtaacgc  1080
ggaacgtctc gggaggtcgg cctctccgct cccgcggtcg cggcggcggc ccgtctccgg  1140
aggttccgcg aaaagtttta taaaaagttt tgagggaggt accgacctcc taaagtttta  1200
cagagagttc tcgcaaaagt tttgggaggt cgacctgacc tcctaaagtt atcgaagagt  1260
tctcgcgaag agttctcgca aagagtttga gaggccgacc tgcctccaac ggttccgcgg  1320
aaaagtttta taagagtttt gagagaggtc gacctacctt ccaacggttc cgcggaaaag  1380
ttttataaga gttttgagag aggtcgacct accttccaac ggttccgcgg aaaagtttta  1440
taagagtttt gagagaggtc gacctactct ccgaggttcc gcggaaagtt ttataaaaag  1500
tttttacaaa gtttttgagg gaggtcgaca cctcggatcc tccgcctcgc gtgccgcggg  1560
cggcctccaa gagttttacg gaacgttctg gagaggagag gccgacctcc caaagatttt  1620
gcggaacgtt ttggagagga ggactggcct cgaaaagatt ttacaaagag tttggaggga  1680
ggactggcct ccaacggttc cgcgaaaaag ttttgcaaga gtttggagag aggtcgacca  1740
ccctccgagg ttccgcgaaa gtgtttgcgg agagtccgag agaggcagac actgcctgcg  1800
aaaagttttc gcggacggtt gagcgggtca ccgacctccg acagtttaca aacgttttac  1860
ggagagttag agaggcagca gaccgacctc cgaacagttt agttttacaa agttttggag  1920
ggtgaactgt catcgggaaa gttttacgaa gagttttggg agaggccgac ggttagccga  1980
gcgagcgcgc gagcgagttt acgttctctc gctcgtgtgc gcgttaactc gcactggttc  2040
cctctctaac tcggagtggg cgagcgagtg gttgactcgt cctccgctct cactctgagt  2100
ggtgataact gttaaccatt aactcgtcct ccactctctc actctctcac tctgagtgag  2160
gattgacttg ttaactcgtc ctccactctc tcgctctctc gctctgagtg agtgaggatt  2220
aactgttcaa ctcgtcaact cgtcctctgc ctctcatcca agactgagtg ggtagttgac  2280
tgctcttgtt ctcttactcc gaggggtgat tgagttagca acagctactc gttctcttgt  2340
tctctcacac cgagtgagcg agtgagcaac gttacttgtt ctctcacacc gagtgagcga  2400
gtgagcaacg gttaactcgt tctctcgttc tctcgttcta ttattccgag gagtggcgag  2460
tgaggtaacg gttactgtta cttgttctct gttcccttcc tcggtgagcg gtgcgtcaac  2520
ttgttctcgt gagtgagtac ggtcacttgt tctctcgttc tctacctcga ggagtgagtc  2580
aacttgttct cgtgagtgag ttgaccgtaa ccgttccctt acctcaagtg agtggcggtc  2640
gcttgttctc gtgagtgggt taaccgcgtt cccttaccgg agtgagcggg cgggcgataa  2700
aaataatcaa ttgactgatt cgctcgtgag cgagcgaagg gcggcggaca agggcgcggg  2760
atgctggtct aatctactaa ggccgattac aaaaacggat gggagaccgg gagggagagg  2820
gtcacagctc cgagcggtgc atccgcgcca gctggcggcg ccactcggcc gcgggccgcg  2880
gccgcccagg ccgcgttgta gccgcccgcc accgcgacgc aggtcagctc gaactccggc  2940
ccgagcgcgc gcacgtcgta gatgtgcacg gtcgcgacgt tcagcagcag cgcgcccttg  3000
cgcagcgagg cgaaggtcgc gtgcatgccg gcggcgagcg ggtacaccgg cgagagcgtc  3060
ccgccgccgt gcgcgaccac cgccatgtgc cgcccgtcgc cgccgacggt caggcaggtc  3120
accgaggcgg agccgttcgc gcggacgggg cccgcctcca cgagggcggc cggcagcggc  3180
ggcgccgcgg ggcggaagag cccgccgagg aggaagccca gcgccaccag cgcgagcgcg  3240
ccgagcagcc tgcgcggcga ggggtgcatg cttgttggct gcggtgttgg cgtctggcgg  3300
gtggaaggcg ggtgtggggt gtccgcggct gcggcgggag ggtctcgact gctaggcggt  3360
ccttttttcac tttgctccgt ggcgcctggt ccggggcaag ggctgcggcg ggcgcccggg  3420
cgggcgggcc gctaccccgc cgcgcggccc gcggccgcca gcgccgcggc cagccgccgc  3480
caccgcgggc ccgccgcgcg cagcagcccc gccgccgagc gccccgcgcc gccgcgggcg  3540
cgcgccgagg ccgcggcccc gcgccgcgcc agcagcagcg gcagccgcgc gtccagcggg  3600
ccgccgcggc gcagcgccgc gcgcagcagc gccgccagcg gcagccgccc cgccgcgtcc  3660
gggcccgccg cgcgcgcgcc cgccgccagc agcgcgcgca ccagcgccgg cgagggcgcc  3720
gcgcgcggac caggtgctcc acgagcaggg tggtgagcaa ggattctcga gaagtaggag  3780
tcatgtgtga cgacaaggag agacgttata ttaggcgcgt cctacttcac tttgaagatg  3840
gtgtaaagtg ttaaaacttg aacaccgttc actccaccac tgccgttacc gtgtcctgcc  3900
ccaaaagcaa ccacagtgct ttttccacca cctgttccaa atccgttcca aaagctccca  3960
tccattgttg ttagaacttt cagatgtttc tctaggttgt ttagttccac tgcaagtttc  4020
gaccattatc gttactggac atgctgttgg taatgagttt aataaccaat cataaaaata  4080
gttataattt gttataaagc taataaagta gcaaacactt taatgttata ttttgcctaa  4140
ccctccgtta acaccaccat taacaccacc acttaagctt ttactaccac cactaccacc  4200
tccaacacac attcttttct ctaaaggtcc ccaaattcca cctcctgaac ttggacgttt  4260
tacagcacct ccgggtgtac ttgcgtaccc tttagaagtt ccactgtgac tgtagatatg  4320
atactgtcct tctccaggca tgattaaagt gtgttgtaat tagtgttatc tacgcaactg  4380
tgcgagactc tcgaataaaa agaagctaca ttttacaatt ttgattagct gatgtaccac  4440
gctgtatcgc ggccaccaca agcacccgat ccagtagaac caaatccaga gtcgccgcgg  4500
tcagtgttgt ccaagcagtt aacctcttga actgctgggc acgatatgcg ttcgcatatt  4560
agctgagcta tcctgtctcc cttcttaacc tcaaagtcac tgtttccaaa gttaaacagc  4620
accactccga cgttgcctcg gtagtcttcg tcgatcacgc cagcgcccac gtcgataaag  4680
```

```
tgtttgactg caaggccaga acgtggtgct atgcgtccgt agcaaccaga aggggcgctt 4740
atcagaaggt cagtaaatac tacgcgactg caatgcgaag ggatgacaca gtcgtatgca 4800
ctacataggt ctaatcctgc ggcaccagga gatcctctgg ctggtatagt ggcgttttgg 4860
ctgaggcgaa caacctgaag agtttccgtg tggcagaact ccatggctag ggtggcgagc 4920
ggccgatcga ctacggggtg tacaatttac actttctcca gaaaaatcag gggcgggtca 4980
gcatggcgcg gcgcaagtcc agcagcgagt cgtacgacag gaagcacagg atggaggtca 5040
cgatctccgg cggcagggcg cacgggcaca tgaggccggc gatctgctcg gccagcgaga 5100
cgcgcagccg catcatgcag atcttgccga agagcgccgt cccgtagatg ggaactcgg 5160
ccgcgcgctc caaaaaggcg ttctgcacga agagcgcctt cgcgtcgtcc gacgcgcgca 5220
gcacgtccaa cagcgtcgcg tccgtgtggc agcgcaccgc gcgcatgctt gcgatctcct 5280
gctcgcacgc gcggatcacg gtcgcgtagt ccgccagcgc gcgctccgcc agcatgcgcg 5340
cgccctcgcc gcgcagcgcc agctcctgca cgcacagcag cgcggcctcc gagcgtcgga 5400
acacgtggcc ccattgctcg gatgtgatca gcgcgcgcgc gagcagctcc gtcggcggcc 5460
ggcgcgcgag cacggccgcc gtcgcgcgca cgttgttgcg gcgcagcatc tccgaaaccg 5520
cgcataggcc cgaggccgac atgtgctcga gctccgcgcc catgcgcacc agccggcagc 5580
aggcgccgtg gctgaacacc gccgcgcggt gcagcgcggt ctgcaggttg ttgttgcgca 5640
ggttcaggtc cagcccgcgc tcgagcacga agtccacgac gccgcgctcg cagctcccgt 5700
aggtcgccat gtagtgcagc atggtgttcc cacacgcgtc tacggcggcc gggtccacgc 5760
ctaggcccgt gagcgtgcgc accatgccct cggagatctt ggccgtgcgc gcgaggtggt 5820
gcagcgttgt gcgcccgtac gcgtccacga cgcacgcgtc cgcgcccgcg cgcagcatca 5880
tgtccacgag cgcggcggag acgccgccgg agcacagcag cgccgccagc ggcgtcaagc 5940
cgttgcagtc gcaggcgttt gggttcgcgc cgcgctcgag cagcagccgc agcacgtcct 6000
cgcggatcca ctggttcttg gcgtacacgt gcagcggcgt tacgccgtag gtgttgccct 6060
cgttcacgcg cgcgcccgcg tccagcagca gccgcgcgac ctcgagctcg gcgccgtcgg 6120
ggccgcagaa agccaggaag gaggagagca cgctgtcgca gacaacgacg ctggcgtcgc 6180
agaccacgtc cgcgcccgcc tccagcatga gcgcgaccac ctccggccgc acgccgtcgt 6240
actgcacgta ggcgtgcagc ggcgtgcggc cgcaggagtc cttggctttc acgtccgcac 6300
cggcctccag cagcacgcgc acgatctccg cgcactgctc gtgccgcgcg aagtgcacgc 6360
agaggtgcag cggcgtgcgc ccgtgctcgc cgcggaagtt cacgtctgcg tcggtggcta 6420
cgagcgcgcg gaccgtttcg aggtccacct gcccggactc caggtagcgg aagagcaggt 6480
ccgcgtgcgg gaccacgacg gactcccgcg agagcatggc ggcgtttaca aatattgaaa 6540
tctttttttca ctcatcttta tggcgctgaa cgcgcaataa gggtgagagt aaaaaacttc 6600
tacaaaaagc gtacaaaagg tacaaaaggt aaaaaaggcg gggcggggac gggctggggt 6660
gctgcgagct gaattggcct ctacacaggg acgccctcgc cggagccggt gagccggtag 6720
ccggcgccgg cgatcatggt caagcgctgc acgagctcgt tgcgcttgac gccggcctct 6780
gaaacgcaca ccatgtggtg gatgtaccgc tcgatgcact cgcagcgcgg gagagtggag 6840
tcaagatcgg atgcgagttg cagaatgtca tcccagagct cggagaactt gctgtacagt 6900
tctcggaggt ctctctccat tcgagccgta agagagtcag gatgcggtgt tccttcggga 6960
gtctgagcga acaccgcgaa caggctggtt atgccgtgtt ctagaataga gtggttccgc 7020
gttaatgccg cagacaaggg tcgtcgtccg cgcaacgact ggcggcagag cgctgtttgt 7080
gccgcaccgc ccattcctct ggcgatcgca tccaccgacg cagtgatcat ctgcgcgccg 7140
acgtcattgt agcgcgcgtt aaactcagta atcatgatta cgagattgca gatttcatag 7200
tagcactttt ccaagtcgac gcgcagtttc acgatctggt tgacaatctt gcacgccttt 7260
cgccgcgtct ccgccacgtt ggcgactcgg acttgcgctt cctggtcgat ggacggcgga 7320
aacacttcaa acccaaggtc gcacagttca gcggtgggga ctagcgtcac gatgatgtac 7380
tccgcatcgc cacccacttg cggcaggaag aacaccgacc gcgcggcggg aacgaccaga 7440
acgtcgcctt cctgcatgtt ggtttttaga aacttagtgt tgttcacgga gatgccggcc 7500
atgccctcgt ttttgacaca tattatggtg acgtacgcgg cgaccgtggg ggccatgtgg 7560
tggcgcatgt accactcgtc gtgcttgagt ttcagaccgt gagattcgcc aacctcgaag 7620
tgcatgttgg cgtctctgac gtagcgcgag aactcgctgc gacagattcg cgcgggcgcc 7680
cggtggaacg tcgactcgaa gagactgatg gctgtccatt cgcccacatg agtgaccacc 7740
gaagaagtgt tttcgatccg agtctcgaac accgagtcca cgagcaccgg acagttggtt 7800
ccgggcaccg tcagcaccaa gggccgcgcc tccacggggg cgacggacga agccacggag 7860
tcggtgtccc cgtacccgta gtcgtcgtcg gagtcgccgc ctccgtcggc cccgtcgcgc 7920
ggcctccgca gcggcatgca gccggcggtg ggaacgcact ggtttcggcc acggccgaag 7980
cggccaaaca gtctcgccag ggctgacatc cttggacggc cacaccaaaa ccaaaaaaac 8040
atattttatc agttatttgt cgattttcac cggctcaccg agggcaggac ctcctggatc 8100
ccggacaccc ccgccaggca gcgggccgcg cgctcgcgca cccaaaagcg gtcgtagccg 8160
tgccggagca cgaaggccgc cgtggcgtgg cagtccacgc gctcgatgaa gccgtggacg 8220
gcgcggcgcg cgtagctcgc cgcgaaggcg cggaccaccg ccgagcagcg ccccgagggc 8280
gagtcgtccg tctccagcgc cagcggcatg ctcgcgatgc gcgacatcag gttggaggtc 8340
tgcgggatgt tgagctcgcg cgtggcggtc atctgcgcct cgagcccggc cttgagcacc 8400
tcgtcgcagc ggccccactc cagcgcgcag accacgcgga tctcgtaccc cttgagccgc 8460
```

agcgcggtct cgatgtccac ggaggtgagc accgcgctga agcgcaggcg ctcttcgtcc 8520
gcggggtcga agagcacggg gatcttaacc tccgcgctgc gcgtgacctc gcagagcgcg 8580
atcgcgagca gcccgcgcgt gagcttgctc accatgcgcg gcttgcccac ggggtacagc 8640
tggctcgcga cctcgcgcag cgggtacgcc agtcggaagc agcgcgcgtc cgcgggcacc 8700
gggctcgcgc ccgtctcctc gaggaagagc gcggcctcaa ccatgttcag cgcggagaag 8760
tgcaccgggc aggcggcgca gccgcgcgcg gcgttcgcga gcaccatctc gcgcagcccg 8820
cggaaggccg ccatgtcgca ggaggggaag atgcgcgcga gcgcggcctg gtgcgcgagc 8880
gccgcgtccg agagcgcctg cgcgccgcgg cggcgctcct cggcggcggc cgcgctctcg 8940
tccgcggaga ccacgtcttc gggcacgtcc acgcagacgc cgccccagaa ctcgcagtac 9000
tcggagaaga gcgtcgcggg cgcaaagcgc gcgaggtcca cgaaggcgac gcggttgccg 9060
agcctggaga gcagcgtgtt ctccgagatg cgcgtccagc ccttgccggc gagctccatg 9120
acctgccgcg tgtcgaagag ggagctgtag aagccgtaca cggtgatgtt ttccttgcac 9180
gtcgtcagcc acatgaggaa gtcgcgcacc accagcttcg cgcagtctcc ggagaacacg 9240
gggccggcgt tcgtcgcgat ggagttcagg cgcacggtgc cgtcactgcc gaagcggtac 9300
acgaaccagg cggccacgct gttgccagag ggcgtgtgaa cgtgtggctg cgcccaggag 9360
tcggcgctcg cggcggtgcg cacgtcgtgc gagagcacct cggtgtcggg gcgcgagtag 9420
gtgctggggt ctttgatcca gatggcgtag ctgcccacgc agcacacgtt catgaggtcg 9480
agcagcgtct gccggcgcag cggcgtgccg agccggcgca cggcgtcgtg cgagaccatg 9540
cgcaggtcgt agaggcccac gtccgagagc cactggttga gctcgtccat ggacagggcg 9600
tcgcgggggg gcgggctgtc ttcgaaggcg gcgcggagct cgggctccgt ctccgcgcgc 9660
tgccgcagga tgtccaggaa ggggctggag gagtcgggga tgtagcagtc ggggtcgtgc 9720
ctggacacta tagcgaaccg ctgcgtcgcg ggcggcgggg ctagcgcgtc ggcgcgtgcg 9780
tcgatgaagg tgcacgatat acgcacggac ttgagcgagg ggaggacgac tgcggcggcg 9840
cgcgcgccct ccgcgtcgaa gatcatcgtc tttccgtccc tcgcctttgc gagcgcgtat 9900
tctccaggca cgaggtccgt cggcggcggc tcgtcccagg cctgccggtc agggacgccg 9960
ccgcacacct ttccccagaa ccccagcatc ctccaaaata cctataagga cggccaatag 10020
cggggcttgc gggcgttcgg accttccgcg ctttaatttt aatttattgg cttgcagaac 10080
tccgagcgcc agtcccgctc gaagaccgcg gacaggtcct tgacgatgtc gcccttctcg 10140
gcgttcacgc tcacgaaggc gtggtagcgg tagtgcgtgc cgtcgaggtt ggcgaccgtg 10200
aggtgcgcga aggtgtcgtc cacgatgagc agcttagtgt tgttcgcggc gtcgtcccgg 10260
ccgggtacca cgaacttgcg cacggacatg tccacgctgc cgacgccaaa gtcgtcgagg 10320
ctgcgcgcgg ccgagaccga aagcgggtcc gcgttcttcc actcggtaat gatcacgcgc 10380
acgcgcacgc cgcggttgat ggccgcgcgc agcagcgcgt caatgatctg cggccagtac 10440
tccacggcgc tggcgtgctt gatcaccggc accatcgaga gcagcgagag gtcgatgctg 10500
ttcttggcgt tctcgatgcg gtgcagcacg aggtcctcgt cgagcgtgcg gtagaagcct 10560
aggaagcgct ccggcgagtc cgagaagaat acgccgcccc cggagtggtc gaggtggaag 10620
ttcgtggccg tgggcgtgac gatggcgcag cagagccgcg tgaacggcac cttcggctcc 10680
acgatcatgg agtagaaggt gttgtagcgg ttcatgaggt cccaggccag gtgcttgttg 10740
gtggagtaga gcccgaggtt cttgatggtg gacacggacc cgcccgtgag cgaggcgctt 10800
cccacgtacc agtgcccggc gtccgagagc cagaagctgc cgagaaggtt gccgacgccc 10860
tccttggtgg acaccttgac cttgtagtag ttgacgcccg cctcgcgcag ctcgtccgcg 10920
tccttgtcct tgctctgcac gtccacgagc agcgtgacgt ctacgccctc cttggcgagc 10980
gtgcagagct tgtccttgac gtcgacgccc tccttggtgg agctcaggtt gcagcagaag 11040
ctgcagatgt acaagaactt cttcgcggac tcggcgatag cggtgaagca gtcgagggtg 11100
ctcatgttgc cctgcgccaa agacgccacc tctgcgggca gcgtctccac gacgcggcag 11160
tcggcgccca gggggatgga ggagaacggc cacatttatt tatctcacaa aaataatagg 11220
gcttcaggga aagtctttta gcaggcgggc gagttcttcg agttcgctta ggagttcttc 11280
catttcttcg gaagtcagca actggagctc ggacttgatt tgaatatctt cgaggaaacc 11340
gtctagcatg ttcgccatgt cttccgggga gcactgcgcc acatcttcgg ggacaggatc 11400
gggtgtgggc attaggtctc cgcttacttg aacgtcgtcc atcatcctgt cgatgaggtc 11460
ttcgacttct agacggggtc cgtagatcag catatttggt gatggaggta gtttaaggtg 11520
cgagagttag tgttatacga ccgccaacgt gtgtttatcg cgcgtacatt ttcaataatt 11580
aacaaactcc ccttcctgcg cctgctcgag aagcagctcg tccagctcct cctgtcggcg 11640
cgcggccacg cgtctttccg cgaagagtac catcagctcc agccccacgc cgcacagacc 11700
caggacgccg aacaccaccg ccgccgagat cgacagaccc agcagcaccg acatcctcac 11760
gcgggcatcc ggctatttaa tcgttctgga aacgtattaa tatgggcgtc gtcatgtgcg 11820
ggtgtctgtt tgtgtgggcg ggctggatcg cgcgccgcgt gcgcggcctc tgcgcggcgc 11880
tgcgccagag ggtgtcgcgc gacaagggct acgtggccgt catccagacc tgcgacgacg 11940
actacttcac agaggaggag ttcgacgacg gcaagcaggt ggtcgcgctc ctgcgcgacg 12000
tctcgcgcgt ggttgccgcg cccgcgggcg tgacggaata agttaggata aggagtcgag 12060
gggagaaaaa cagcggtcac actataaact cgcgcgaggc cgattttgac gtgctcatgt 12120
ctggaagctc cgctttctgc agcgcggagc ggcacacgaa gcacacttcc gtgttggtgg 12180
gagttatgca gtggacgtgg tagccgtgcc cgcacaccat gacttggaac ggacacgcgc 12240

```
cgggacaggc cgcgtttatg catccttccg gcgagcgctt gttgcagatg tagcacacgt 12300
ctgagcacgc cagcgtgcag gacacggcca gcttccactg cttaacctta acgggcatgg 12360
ctagttgaac acgaccatgg gcgagtcgcg agcctcgagt cggggggttca gggcaaaccg 12420
tttcacgccg tcaacggttc ttctctttgc aattttctct ctgcacaggc tcgtcagcgt 12480
catctcggcc aggcgcgcgt cgttgcctag gtgccgcgcg gcgtcctcga ccgtcacgcc 12540
cgtcttgccg gcctcgtcca tgagcacaat gcataccagg tgcgcgctag agcatatgac 12600
ctcctgctcg cgcccgccgg cagcggggat ggttagctcc gcgcgcccga aggccgccag 12660
cggcgccacg tcgtaggcag tgtctgctcg ggcgagcgcc gactccacgg caccgcggag 12720
cgactccggc ggcgtcagcg cggccagcgg caccggcgtg ggcacggtgt acacgttcac 12780
gggcatgagc accatctccg ggtcgtggtg gccgctctct tcgccgtcgt gctccatggg 12840
ctgcggcggc ggcagcagcg ggagcagcag ccgtccggac atgagccggc gcacaaggtc 12900
gttgagcgcg gacgaggcca tcggcgggta cagctccatg gccagcttca gcgataggtg 12960
cttctcgagg ttgacgccgg tgtagacgct cttcacgatg cgcgcgaagg ccacgcgcgc 13020
gaaggccgcc agctcctcgc gcggcaggcg ctcgatgtag gagagcagca tgtcggtgtc 13080
gcacggcggc gccgcgacca ccgcgccgta gagcgccttg cccgagagct tttccagcgc 13140
ccttgcgtgc aggccgtggg tcttgagcac gtccacgtag ttcacgtaca ggcagagcgc 13200
gcgatcgagg ttgctctccg cgacgtgcgt ctcgatgcac tccacgatga gcgggcccat 13260
gcggtccttg atgaggtcta tgagcccgcc gtaggcgact cgcgcgctca tgaggcacgt 13320
gcggcagtac gccatcaggc cctcgaggtc cgcggctatc acgtcctcga ccacgttcgc 13380
cacgacgcgc tgccagagcc gcaccttgct cacgttctgg tgccgcacca tgtccacgag 13440
ctcgtcgtac gagccgccgg gctcgtgcgc gcgatcgacg atgcacctcg ccatggtgcg 13500
gctctggcgc atgagctcgt tcgtgaagcg cacgcacgcg tcctcggaga agagcgcgct 13560
caggcaggag tagcagcggt ccgcgacgag gtgcgggaag cggcactcca cgacgccgcg 13620
gccgatccgc agcacgcact cgccgtacat ctcgtccatg gcctcgcgca gacagtcgtc 13680
cagcacgtcc gcgttgtgcg cccactggat cacgcagagg tagggctcga tgttctcgcg 13740
cgcgttttcc acctcctgca ccatgtactc gagcacggtc atgtcctcgt ggatgtcggt 13800
gcccagcatg cgcccgggcg gcagccagct cttgcgcgcg atcgcctctc gcaggcacgc 13860
caccgccgtg aaggtgttga cgcggagctt ggtcagcagc cgcccgcagtc gggagatgtg 13920
tgccacggag aggtccatct ccatggcctg ggcgatgagg cgcgtgagtt cctcctccat 13980
ggcggcggct ccgcgggcag atatacgcga acaacggtaa gccgtgctat ttcatttttg 14040
gacaaaaagc tagtcgtcga cgcgcatgtt gtcgaggttc cggcacagcg agagcacgtc 14100
gtcgcgcgcg cgcctccggc gcagttgatt gttcgcgcgc cgcgcgtccg cgagcgcctg 14160
tctgtacatc gcggagtccg cgtacccgtg cagcggcgag cgccgagcgc cgggcctcgg 14220
gctcgcgcgg cgcgggagcg gcgttggcgc gcgcctcgag cgccgcgcga agtgcgcctg 14280
catggccagc aggcaaccga acggcaccat gtatcggtcc atgaggcact ggctggccgc 14340
ggacggctcg cgcgggtgca tcacgccgcc gcccacgtcc tccatgacgt cgcgcagcac 14400
gcagcgcagc atggtctcca tgctgcgctg cgtgaaccgc accggggagg cgtcgacgta 14460
gaagccgtcg gccacgaagt agagcgcgtc cagcccgccg agtttctcgc cgagaccgac 14520
gaagagctcg tccacgtgcc agtccaccac cgaggccttg aagagcacca cgtgccggat 14580
gtcgtgcgag cgcgcgagct cccaggtgtc ctcgccgatg ttgctggcgt cgatgcggcc 14640
tgtcatgcgc acgctcacgc acggcgtcat cccgttcttg tagcagaact ggcgcgcgag 14700
ctcctcctgg cgtacgatgt cgaccatgct ctccatgaag gaggtggaga gcagcatcgc 14760
gccgcgcgcg gcgcgggtcg cgttttcgtc cacctccact tccatcccgc cgccgatcct 14820
aatcatctat cgtatttaaa ttttcggcgg agcagacacg cggctgctcg ctgcgcgatc 14880
gcttcagccg cggcggcgtc acgcacgcgt tgcggcggcc ggcacgcacg gacgaccgcc 14940
ggggctgttc gctgagcgag cgccgcggcc gcgtgacgcg acagtcgcag gtgggttgcc 15000
gggagtcgct cgcgcgcctt cttcgcattt cgccggaacg ccgcgtttat gtaggggatt 15060
atattttcaa cgtaactaaa tggacggggg cgtgcacaaa cggcctttca tcgtgaacgt 15120
ggatggcatg ggcaaggtgc tcgtgctccg gtacttgcgg atgtgcgagg tgcccgaggc 15180
caagtgcgag gggtcgcgcg cgtcctgcgt gctcaagatg gaccctcccc gctcacccag 15240
ctgcgagcgc aggccgtctc tcccgccgtc ccccccatgc cccatgcgca cgcctcccgg 15300
gtcgccgctc caggctccct tgatgcgcac gcagatgctc caggggctgt tcgacgctgc 15360
caaaaacaac ggcgagcaga tgtgccgccg ccagtaacct aggctgcgca gtacgaaagt 15420
tagtgcgtga tcacgttttt tgcaatgtcg atcacgccgt gcgtgcccgt cttgcgctcg 15480
cgctccacca cgctagtcac gggccgcgcg tccgagacta gcgaccccag catcgagcgc 15540
acggcgccct ccgcggcggg gtggcgcgtc agcagcagga acatcacgat gtgcgcggag 15600
acgccgcgcc ggctcagatc gtgcacggcg tcgccgtcca tgagcacggt gttcgagaag 15660
tacgtgaaca gagtgttgtc tcgcaccagg aaggccgagt tcgagacgct ctcgaagtcc 15720
acgatctcgt cgtcctgcac gcccatgtcc aacagcgtct gcacgagcgc gggctcgtcc 15780
aggaacacca ctgcgcgcgc gaacccgcag tccagcgcgc gcgcgtccgc ctccaacacg 15840
cgcgaggcgc cgccctccgg cggcaggaag gcgcagggca gcggcgtgcg tccgtccgcc 15900
ggcgcctccc cgagctcctc gagcgcgaag gccagcagcg tctccatgcg cgcgcgcgcc 15960
ttgtcgaagt tgtccgcgag gtcgcggatg cggtctgtct gcgagaacat cttcagcatc 16020
```

gccatgagct gcacgaaggg gtgcagcacg tatatgttgt ccacgagcag cgtgggcagc 16080
gcgcgcagcg tggcctgccg cacgttgaag ctgtccagga tgtgcccgcc ctcctcgtcc 16140
tgcagcacca cgtagttctt caggtagggc acgcgcagca gcacggtctg ccgccccgtg 16200
acgaagtata tcaggaaggc gaggttgatc aggaacgggc gcgcgttcgt ctgcaccatg 16260
tcgatgtcgc cgtactctat ctcggggttc agcaggtgca gggcgtacga gccgtagcac 16320
acgcaccgct tgttgtgtcg gcgcaggtgc tccttcacga gccgcttgac cacctccacc 16380
aggtccgagt gcttgtgccg cgccatcggc gcggcctcct cgggcggcgg cagcactgcg 16440
tacgagttga gcgcgcggct ggcgagcgcg cgcgcgcggg gcgcgtccac gcgccgcacc 16500
gccgcgttga ttgcaggcgt cggcgtcgtg agagagccca gcgtgcgcgt gaactcgctc 16560
acgatcacgc tctgcagctc cagcaccgtc aggatctggc ccagcttctc caggcgccgc 16620
tgcctcgaga agtactcctc gatgcgcgcg gcgatctcct tctcggagcc gcctagcttc 16680
ttgaagaagc gacgacgact ctttacaaca agagagagaa aaagcttcct atcgaagttg 16740
aggacgcggg tcatgttgcg gcgctgcgcg cgcaggagca cgcagcgctc catggagggg 16800
cgcgagccga ggtactcttc gatcacgggt ggagccatga cagctatttt ctgaacccgc 16860
gattattgta cagcgcaagc cgcgcgcaga cctgctggca cagcagcgtc gtgtttcgca 16920
tgcacacgcg cgaggactcg atcgtgcgcg cgtccggtgc ccaggcgcgc agctccatca 16980
gttcctgctc gacgaagtcc acgggctcca cgaagcgctc tgcgcagagt ccgtccgtga 17040
acgcgttgac gatctgccgc acgagcacta ccacgtccac ctgctccacg aggcgcacgc 17100
ccatggcgac gtgcacgaag aggcagcgga agagcgcgtc catggccatc tggtggtccg 17160
agcagggccc gaccgcggtc ttgcagccca gcgcgaagcg cccgatgccg cggtactgca 17220
ccatctccga gggcgagaag gagagccgct ccatcttgag cacgggcggc gggcccgccg 17280
gcagtccgcg cgcgaggtcc agcaccggcg tccacccggg cgtgaacatg tccgggatca 17340
ggaagagccc gtagctggcc atgcgcgcga tgtcgaaggc gtggtccacg accttgttca 17400
cggcgctgtc cgcgcggttt acgcgcagcg cctgcaggat cacgtttccg gaagcgtgcc 17460
gcgtgatcgc gaggtccgcg gtcgcgtacc cgcgcaggcc cggcaccgcg tacgcggtca 17520
gacacacggc caggcgcgcg ctgtgctccg aggagaagat ctcctgctcg tagccctcct 17580
cgggctcctc gcactcgcga gggcgccgca cgtcctcgac cgagcgcagc cgcatctctc 17640
cctccgacac cagacagcca agcgactccc tcaccgccgg cgcgagcacc tccgtggcgc 17700
agagcgcgtc gtgcacgcgc ttgagtgtgt tcggcttcag cgcgtagccg aagagcagcc 17760
gcgtcatccg cgagcccgag aacgcgaagc ggcgcacgta ctcctccgcg agctcggggc 17820
ggtcgttgat ccacgaggta gagaagacgt ggtcggaggc gaaggggtct gcaccaaccg 17880
cgagcagtgt ggacagaggt acggtgtcga ggaagtccac gacgtcgggg aagttctggc 17940
gcacgcaggc ctcggcgacg cgtctggtgt gcacgcacat gtcggtgacg ggcacccggt 18000
ggccggactc cacgacggac acgcagacgt cctcggtgac ggcgtccacg ggcatggtcc 18060
gcagcagctt gccgagcacg tcgccgaacc cgccctcgag cgccttgcgc cacacgaacc 18120
ccgcgtcgaa cttcccgggg aagtccgcga tcaccgaaag ctccgcgtgc gagaggttgt 18180
ccacgttgag gtaggtggcg gcgtccacga agatgggccc gaaggcgccg gtgtcggaga 18240
cgcggtctct gaggtagtcc ctggcgtagt ggaggtactc ccgcacctgg ccggcgcgga 18300
tgcgctcgag cgcgaaggcc ttcatggtct cggagcagag cacggagtgc cgcagcccgt 18360
ccagtgtgcg ccgcacgtcg tagacgccgc gcatctgcgc gagcatctcg acggcgtccg 18420
ccggcgtcgc cgccgcgagc cccgggttca ctggaggtat cctgtgttct gcgagcatgc 18480
gcttgaggaa acagaggtcc agcggccgcg tggtgtacag cgcggaggcc atctcggggc 18540
gcgtctcgac gatgtcctcg atcatctcgt ccgtgaatgc cgcgttgatg ttgtgcacgc 18600
tgcgcgcgtt cacgtgcagg aggatgtcgc ccacgttgtc ggggaatcgc tcctcgatga 18660
gccggacgtc gtcctccgtg atgttcatgt agggaataca gcggcagagc agcgcgtagt 18720
ccgcgaactg cgcgatgtag ggcgtgtgga actcgatgtg tctggcgaag agcgcgccgc 18780
agcgccgccg cgagagctct tcgagcaggt cctcgggcgt gacgtgctgc gggcggaaga 18840
ggtgcaggtg cgtggggtgc tcggcggcca cgcgcgcgta cagccgccgc gggaggtgtc 18900
tggggtgcac gccggcgagc acgagatcca tggcctctga ggtagacagt gcggcgaacg 18960
cgcgctcggt gccgcccgcg gcgacagcgg cgccgacaaa tctcttgagc agctgcagca 19020
tcgcgtgttt gggctttcgc ggaaggcgct tattttaatg ttattggcgg tggccggtgc 19080
gagataaaaa ttagaactga tgccgcagtt gttgatgata tgatgattgc gctggccggc 19140
gcgagataaa aattagaagc tgatgccgca gttgttgatg aggatggtga gtgcgctgga 19200
gcaagcggtg tggcgcgcta gcttcttgct ggccccgtcg gccacggcaa cgacctttcc 19260
ggatatcgtg atggtgcagg tgaagcgcgg acagtgatcc tctccgccag cacgcgtctc 19320
gcagaactcc agaggtctgt gtgtcatcat gcagaactcg ttgaccgcgc tgaccgggtt 19380
aagacttttg aggcgtatca cggcagactg agtcatgatg tcgatgtcgc cgccgaaaag 19440
cgtatcgcac ccagcctcgg tctccatggg ctcggtgtcg gagttttcgt cctcctcggt 19500
gggcgcggag ggcgcgcact ctacgaacca gcggggcggg tttccgtcct cacagcaaac 19560
ctcgtccgag tccagcaggc ggtacagctg gcggtttgcc tcgtgtttgg atatgccgag 19620
ctccttcgcg atttgcttgg ccggcagctt gtcgtcggat tttctgagaa gctcgaggat 19680
cagagacgcg cactcgcagg ccattgtggc gatttacggg gcgtgcgttt ttttaggatt 19740
ttggcttgcc tttcttttcg cagaacttgg gaggattgaa actcttttgg caatttttgc 19800

aggcgtactt gatcaagggc ggctcgtccg ccgagcgcgt ctggatcatc atcggcatgg 19860
tgttcttgct ctggcacgag gggcagggca ggttgaactt ctcgtcgagc acgttgaagt 19920
acccgctgta gtcgtggtcc ggcacctcct cgatgtcgta gggcacgcgc gcggccacgc 19980
acttgaccgc gaagagcagg taccgcagcg cgtcgtgctc cgcgccgctg gtcgcgcgga 20040
tctgcgcgca caggtccgcg tagtcctcgt tcgcgtccac ctccaggctg cgcttgttct 20100
tgtacgagag ccggttcttg gcgtccttcg agtactcgat gccgatgttg tgcgcggggt 20160
caaagttggt ctcgtcggtg ttcgaggtct tggtgttcac gatgttcttc agcgcgaagc 20220
gctgcgcgca gtccagcgcc catcgcgcga tgcgcgcggc ctccgccgcg tcggtgtgct 20280
tcgccgcgag gtcgcgcagc cggtcttcgt ccatcgcccg attttaggtt gggtatatta 20340
tctcaattcc gctcttccgc gggccgcggg cgcgcgcccg cggcaaatta ggcgttacaa 20400
atggacttcg tgcggcggaa gtacatgata cacgccatcg accgcaacct cgacttcatg 20460
aaggccgagg tccagcagaa ggtctccatc ttctccctcg ggcacgtgct cgcgctccac 20520
tacctggtca ccgcctttcc gcaggcggtc atcaccaagg acgtgctcgc gagcacaaac 20580
ttcttcgtgt tcgtgcacat gtcgcagcgg cacgaggtct tcgacgccgt gctcaaggcg 20640
gccttcgacg cgcctcagct ctttgtgcgg gcgctctcgc ggcacttcga ggccttcgtt 20700
gccgccatcc gggcctaccg cgcgacctgc gcggagctgc tggccgacgc gcgcttcatg 20760
gaggtggctg cgcgcgcggc cgagctcgcg gaggtcattg gcgtgaacca cgacatcgcc 20820
gcgaacccgc tcttcgcgga cggcgagccc gtgcgcgacg cggagctcat tttcgcaaag 20880
accttccgca agaccgagtt ccgcgccgtc aagcgcctcg ccgtgctgcg gctgctggtc 20940
tgggccttcc tcgtgaagaa ggaccttggc ggcgagtacg cggacaacga ccgccaggac 21000
ctgtttacgc tgctgcagaa ggccgcgggg cccgtgcgcc acagcgcgct cacagagagc 21060
atccgcgagt acctcttccc cggagacagg cccagccact gggtctggct gaacgcgcgc 21120
gtggccgacg acgcagaggt gtaccgcgac cggcccgcgc gcacgctcta cgagcgcgtg 21180
ctcagctacg cgtactcaga ggtcaagcag gggcgcgtga acgccaacac gctcaagctc 21240
gtgtaccggc tcgaggacga ccccgacatc aagggtctgc tgctgcagct catctacgac 21300
gtgcccgcgg acatcgtcgg cgtcgtggac tccgcgaacg aggagtggcg gagctacttc 21360
gtgagtctgt accgcgagaa cttcgtcgac ggacgcacct tcacctcgga cgcgcgcttc 21420
cgcgacgacc tcttccgcgt ggtcgccgcc gtcgatcccg acttcttcga gcccgagcgc 21480
atccgcgagg ccttcagcgc agacgcgcgg ctgcgagagc gcttcacgga catggacctc 21540
aacaacgcct tcatgtcgca cctcatctac gactccgtgg accccgacgt cgccgccgcc 21600
gagcgcgggc tcgcactgcg cgtgcacaac gaggactccg actacttcat ccgggagtac 21660
aacacctacc tcttcctcag cgagaaggac ccgctggtgc tggaccgcgg ggcgctcacg 21720
cggctctcgg acgtccccgc cgagcgcttc cgcgacctct tcagcgacag tgtgctgcgg 21780
tacttcctgg acgcgaagct gggcacgctc gggctggtgc tcgaggacta ccgcgaggac 21840
gtggtcgccg ccatgcttcg gcacctgcgc cgcgtcgagg acgtgtcttc cttcgtgacg 21900
tacgccgcgc gcaagaaccc cgcctgcgtt cccggcgtcg tgcgcgcggt cgtgagcaac 21960
ttcaaccccg cggtggtcgc ggccatgcgc cccttcctgc gcgagcacat gacgcgcgtg 22020
gacgcgctgc tggacggaat gccgcacctc tcggaggccg accgtcggta catccgccgc 22080
gtggtgctgc agggccgcgc ctgattcgcc gtcaataaat cgcgatggtg gacagcggca 22140
cgcacgacgt ggactccgcc gcgcaggagc gcacgcccaa ccagcagacc ttcttcacca 22200
aggggctcag tccgctgatg cgccacacct acatctacaa caactacgcc tacggctgga 22260
ttcccgagac cgcgctctgg agcagccgtc tgggcgacta ccgcgtcacg gacttctacc 22320
cgatctcgct gggcatgctc aagaagttcg agttcatgtt ctcgctgctg gcggaccccg 22380
gcggcgcctg ccccgcgtac gagcccaagc tcaacaccga gttcctgaac cgcggctcct 22440
tctcgggacg gtacgtgaac cccttccacc gcttcgcggc gctgcccgag cgcgagtaca 22500
tatccttcct gctgctgagc tcggtgccca tcttcaacat cctcttctgg tttaagggcg 22560
agaccttcga cactgccaag cacagcctgc tcggcgccgt gtacaccacg cccgagcggc 22620
acatcgagct cgcgcggtac ctgcggcgca cgggcgacta caagccgctg ttcagccgcc 22680
tgggcaacga cgacacctac tcgaagccct tctctgggtt cacgcgcatc agcaacccca 22740
cgcccatcgg gcggctgccg ccctcggact tcgagacgct ggccaacctg agcaccattc 22800
tctactacac gcgctacgac ccggtgctct gtttcctggt cttctacgtg ccggggctct 22860
ccgcgaccac gaagatcacg cccggcgtgg agttcctcat ggagaagctc tcgctcgcgc 22920
ccgagaacgt ggtgctgctg tagcctcaaa cataaaatat aggcgccttt gatcgcactg 22980
cttcagttca gacagagcta agatggcttc ctacatcagc ggcgctagcg ccagcgcgaa 23040
caccgcccag ggcggcgatt ctcagtaccc acagtactat tatcacacac gcacctccca 23100
aggcgacatc cgcgacgaaa gcgaaggttg cttccacacc acggacgacg agcacttgga 23160
tctgtccgac gactacctcg gcgatggcgc accacactgc ggacacagcc acaaccacag 23220
tcgcagagat ggagatcggc accgccagcg cgcaccgcgg ctctatgagg acccggtgcc 23280
cgcgaacatc atggtgccca cgctcagtct agagcagctg ctggaggaaa cctcggtcgc 23340
ggggggcctt ctcggcggca ggacagagag ggacgtggaa cagctcctgg aggagttctc 23400
cgcactctgt cccggggacc agatcaccgc gctgcgctgc atggcggcct cctttaccg 23460
cgacgcgctg ttcgcgccgt acgcctgcat gcacctcatc gccagtcgga tgcgcgtgca 23520
ctacgcgcgc gaggtcgtgc acgtggccga ggacctcgcg gacgcgatgt ctgcgaacag 23580

cggcgtctgc ttccggcggt accgaaagcg cgtgctagag gacatgctcg cggaggagat 23640
gggcgtgtac aattacctcg cgcgcgccaa cgcggacatc tgcgaggaca acctgctatc 23700
ggccgtggag acgctgctgc ggcgcttccg tcggatgggc tgctaccgct ctctgtgcat 23760
gctcaagatc ctcgcgctgc agcacgagga cctggccggc ttcatccgcc gcagcataag 23820
aaaaacctgc aacttggcac acgcgcgcac gcacacggtt tacgtgtagt taccctgtaa 23880
agacgggctt gctcccgaac aagcgctcga agaagagcgt gcacatagcc ttattgtcca 23940
gcaagttgac tatctctgta cacagcctct tgaagtacac ctcgtacatg atccgctcgt 24000
ttttatccag tctgaaggtc ttgtcgacca cgcgctcgta ggacttcacg ttcgcgatcc 24060
ggcgccgcca ggggccctcc tcgcacacgt acgcgaagaa gtagcgctcg ccgatctcga 24120
tggcctccgc gttcgccgcg ttgtaccgcg tcactagcgc cacgttgggg ttgtcggggg 24180
acttgaagtt cttgtggtgc gttcggctca gcaggaacca gtccagcggc atgctgcgcg 24240
cctcgaactc gaaggtgagc tcgtcctcca gcgagcgcag gatctccacg cccacgttcc 24300
cggagccctc ctccgccagc gcgcggcaga gcatgtcctt gtacttgcgg atcatgagct 24360
tgtggaaggg cgccacgtcg cggcgcgtct cgctggtgcc cttgctcacg cgctcgctgc 24420
cgccgccgtc gctcaccgca aacttgatcg tggtgtactt cttcttggac tgcatgatca 24480
ggttgcagta caccgcttcg aactccacct tgaagttcgc gaagagcacg tgctcgttga 24540
tcacgcgctc cagacagcgc cccacgcgcc gcgagaacgc gatgtcggag gcgcccacct 24600
ccaggaacac ggagtcggtg tcgccgtaca cgctgcggaa gcccacgcgc tccgtgcgct 24660
cgccggccac cgccgcgtcg atctctagct ccgcggcgcg gcccgcgaag gcctcgtcgc 24720
gcagcagcgg gttgtccggc gccgccgcca gcgacagccg cgtgccgcac accgacgcgc 24780
cgtctagcgt gcgctccagg tacgcgatca tggtgcgccc gatggccgtg cagctcttgg 24840
ccgaggcgta cgagaagagc gcgctgttgc ggaagcccat gagcccgtac acggagttgg 24900
ccgtgatctt gtacgtgtac tgcatcgagt tgtagatctc gcggtccacc gcggtctccg 24960
cggccttcat cagcttcttg tacttggcgc gcgcgtccag gaaggagcgc agcagcatcg 25020
ggatgatgcc cttggcctcg cggtcgaaga tggccacctc ggcgacgagc tccggcgagc 25080
gcggctcgca gggcaccgcg atgtaccgcg gcgccgggaa catccgccgg acgtccacgg 25140
ccgcgacctc cgcgtcgagc cggttgtccg agacgaccac gccgacaagc gtctccggcg 25200
acaggttcgc gtagatgcac acgttcgggt acaggctgtt gtagtcgaag atgagcacgt 25260
gcttgttgtg catcttctgc ttgggcgcca tcacgcggcc gccctcgtag aagaacttgg 25320
acttcgtgtc cgcgcgcacc atcaccgtgc ggttctccag cagcagcttc atcagcgggc 25380
ccttgatgca ggtgctcgcg cggtactcga agaccacgct ctgcggcagc aggtacgtgg 25440
acgcggcggc cgcgatcttg gtctccacgc cgtagtgcga ccagaggtag aggcagaggc 25500
aggcgtcgtg caggcagtac cgcgccatgt ccagacacac gtccagcgag tagttcgcgt 25560
acatgtccgc gaggctgacg tcgtccttgc cgaaggccag cgtgacgcgg tcgccgggcg 25620
cgcgcgccgc ggggtccgcg aggtccacgg tgaagccgtc ctcgtcgacg cgtttgtgca 25680
gcacgcggca cacgcgctcg tccacggtca cgtagttgcc ggtgctgagc acgcgagcga 25740
acacggccgc gttcccgtcg gcgtcggtgc tgcggtcgcc gcgaaagcgg accgcgtccg 25800
gccgcgcgtc ctccacgacc gcggtgcagt ggaaggcgtt cttggatatg gcgtccagct 25860
tgtaggagtc cagcttctcg gtgcgctgga tgaaggcgta caggtcgaag tagatggtcc 25920
cgttgttgtt gttgatgtgg aaggtggtgc tcgagacgcc gccgacgccc ttgtggctgg 25980
acttcgtgcg ctcgtacacg cagaagttga ctgtctcggt cccgtccggc agccggaagc 26040
ggatgtgctc gcccgtgagc agcgacagcc gcgagtccag gtaccgcagg tcgaagttgt 26100
ggccgttgaa ggtgaccacg aagtccagcg gcatctcgag caggcgcttg gccacgcgca 26160
gcagcgtcac ctcgggacac agcgtgacct ccgcgtcgaa cttcacgtcc gccgggtcca 26220
ggcagaccgg gatctcgcgc cgtgccgcct cctcgaggtc cgcgtcggag agcatatcgg 26280
agttcgtaag cgtgaatcgc cgctccgcgc cgtccttgtc caccacgcag aagctgatgt 26340
gcgagacggc gttcttgaag acggaaggaa actttttctc gaagtggcac tctatgtcga 26400
ggaagagccc cgagcgcgtc acgttgaagc gcgggatctt ctccgcgaag cacgcgccgg 26460
ggtcgtcgca gtggaagcag ttgctcccca ggtcgcgcag cagcgcgggg tccacgcggt 26520
agcacccgtc agggtcgatg tcgtgcgcca cgaagaacca ggacacgttc agaaagtcgg 26580
acatgaagac ctctggcggc gcgagcttgc gctcgctggc caccagacac agctctatct 26640
ccgagcgctg gcgctccgga atcttcgccg agcgcgctac gatctcgtcg atgctgacca 26700
cggacatggg cccgagcgcg cgcgtccacg ccagcggctg ggcgatgtcg gccaccgcgt 26760
ccgcgcgcac cacgtagtaa aagtgctgca cgaagcgcag gtacacgacg gcgttgtcgg 26820
cgcggcgcgc cttgaggaag aggaaccggc tgtcattgcc gcggttctcg aaccagttca 26880
aacatttcag ctccatttca aagagcataa taacatttca tttaaatgga gcctcgcttc 26940
tggggccgcg ccatgtgggc ggtgatcttc atcgtgctgc ggcgcttcga ggagcaccgc 27000
gacctcgagc gctgcaagcg gcagctgtac gtgatctgct ccacgctgcc ctgcatcgcg 27060
tgccgacgac acgccaccgc cgccatcgag aaaaacaacg tcctctccag cgaggacccc 27120
aactacgtgc tcttcttctt catcaagctc ttcaacaacc tcgccttcga cgacagatac 27180
aagatcgacc ccgcgaaggt gcgcccgctc gtctagagca tgccctcgta cgcgcgcgag 27240
ttgtccgagt acacggtcac cgcgatgccc tcgcgcggcg tcacgtgcac gtgcatggag 27300
tccgtgatca cgaagcccac gcccgtgacc ggctcctcgc ccgcgtcgtc cgtgaagagc 27360

agcccgtggt tgaagaggta gaactcgttc tctgcgagcg aaagccgccc gcggtcgcag 27420
aggtagtaga agatgtcgta gtcgcgcacg gccagcgtga acgtggactc gctcaccacg 27480
atgtacttgt ccagctcctc cagcacggac gcggccgcgc ggcgcgcggt ggcgcggcac 27540
tgcgtcgggc actcgcacgt gtctgcagag tacaggatgc gcgttcgccc cgaggcggtc 27600
tcgagaaaaa cgttaatcgc ctccatcgcc cagaagcgac tcgaggatcg cgagcaccgt 27660
gcgcagcacg agcccgatgg tgcagaaggg aacctctccc gactccgagc actcgcggat 27720
ctccgtctcc acgcggtcgt gcacttttat ggagggagcc gtcgttccag tggcctccat 27780
cgcgacggac accaccttgg ccacgaactc gcggatcttg ctcatgcgcc ggagcacggt 27840
cacgcggaag aagacggccg cgagcaggta ctcggccacg cagctcacgg cgatgagcgc 27900
ctgcgcgtgc ctggtgttga gcacgtgcgc gtcgggcacg aagtccgaga tcttcaggtg 27960
cgagagccgc acgagcgcgt tggtgtcctt gacgccgtcg caggaaatgt tcgcgaagat 28020
gagcttctcg tagtcgagcg cctcgaccac gcgctgcgcg tccatgtgcc gctcgcccga 28080
gagcgcgcgg ctcaaagggc cccagcacac cgagcccgcg aagcaggggt ccaccacgcc 28140
gtgcatcgcc agcagcgtca cgtcggccac cgccgcgagg atggccgcgt cgtcgagctc 28200
gttcgcgggc gtcgcgcccg tcagggacgc gctgcgcagc gcggacccgc ccggcgccgc 28260
gtgccgcgcg cagaactcgc acccgcagcc cggcggcagc ggcggcttcg agagcagact 28320
tatgagcccg cccacgtgcc cgtgctcggt gatcagaagc gccaggatgc tgtcggtgct 28380
gccctctatg gcggctgtgg ccgcgctaga aggctctccc gtgacctgcc atccgcagac 28440
aaccttgagc atcttgcgct tgagctcgtt gggcgcctcc gaggccagca tcgtgcgcgg 28500
gaacgtcgcg ttgaggcgga agtcctgcag cagcttttcg agcgtcgcgg tcttggcgtg 28560
ccgccctttg cgccactcct cccccaggtg ccacatgagc tcctcggccg tgtccagcgt 28620
cggcgagacg cgggccttgg gcacgcgcgc cgcgctgcgc atcagcggct ccgaggcgcg 28680
gaagctgccg cgcgcgtgca ggcgcatgga cgcagacgag gaccgcatcg agggtctctg 28740
gtggaagctc gtcatcgtga agcgccgcgt gagcggacct acctcgtcgc gcgactcgtc 28800
gaagtccggg tcagggtccg tcgtgtcggt ctcggcgctg tgcgtgctgg gcgcgctgct 28860
gcgggcgctg cgcgtgctgc gcgtgctgcg ggtgctctgc gtgctgaagc tgcgcgagca 28920
ggagtccgcc gtgtccgctt cctcgtagtg gaagtccacg tgctcctccg gcagccggcg 28980
cgagcgcgac ttggagatgc cgaccatctt gtccgcgccg accgggcgca cgcagagcgc 29040
catcgcgccc tcgcgcaccg cctgcgcgaa ctcgcggctg gcaaccatgc tggggttgag 29100
gaacttgatg atgttgaagt atggccactc gcagacgagc cgcgcgcagg tcgcgacgtc 29160
gtcgacgctt ctgagggccc tgttcagcgg ggggatgttg ctgccgtcgg ccagtgcgac 29220
gaggtcctcg gggagatctc cgagcttggg aatcatctcg ttcacgagcg cggggtcgat 29280
ggcgtggcag acggtctcta cctcggtgcg cgagagcttg agctgcgcgc aggccgccca 29340
cggcgcgcac gtgagcgcga acaccgcgga gactcggccc ttgccgacca tcgccacaac 29400
ctcgggctcg tagaccaggc cggccttgag cagagcctcg agcacctcta tgtcgtcggc 29460
ggcgagcagc gcgcgcctgt ggaagcacac cgcgggcatc atcttcttgc agatgccgcg 29520
ggtgctcttg agggccgtga taaggtcggg gagcctgacg tgctgcggct ggaagaacat 29580
gacgttggcg gggctcgcgg ccaccgcctc gtcgtacacc gacatcggca ggtcgccgtg 29640
gagcccgcac ggcagcaggc tcagcagctg cgcgcgcgag agcttctcgg cgcagaagtt 29700
cttcttggcc agggcggccg ccgcgtcgcg ggccttcttg ggtataaca acatggcggg 29760
ctttaaacac gaaacaaaaa tccgggttgt aacatttcaa ttttgcatgt tctgggcctc 29820
ctcgcagagt ttctccaggc cgccggccac gatggcgtcg acgaagaggt cggcctcggt 29880
gaagcggtgg ttgccgcgca ccgccaccag cgcgttctcg gtgaccacca ccgacagctg 29940
ctgcgcagcc gtgcgcaggt cgaagtgtcg gcacgcgagc ccgtggccca gagtctggtc 30000
cagcgccgcg agcacctcct ccaggctctc ctcgcggtgg ttgtagaacc acatcagcac 30060
gaagtaggcc acgtaggtgt agaggtagtg cgcgaccgcg cgggcgcgca ccagcggctg 30120
gttgcagcgc gcgaaggcca ttccgctggc gatgaggtcg tcgtccagcg tggcgtagtc 30180
gccccagttg agcgcgctga actgcacgct gtagaccgcg cgcacgaacc cggactcgag 30240
gatgtcgatc tgcgacggcg cgccccaggt caccagccgg tacacgatgc cgcgctgcat 30300
cagccgcatg aggtcgccgc cgacctcgcg caggcggtgc gtcagcgagg cgaaggccag 30360
cttccgctgc gtgtactgca ggcccacggc cacgcacccg tccgactcca ccagcacgag 30420
cttgtggtcc gtgcagccgt cgctgcgcag gccgccctcg cgcgccatca tgtcggtgac 30480
gaacatgtac ttgcgcgcgc gcggcccgac caggatgcgc ttcttgcctt ccatgcgcag 30540
caccacgtcc tctagcagcc gcgtgctgtc cacgcgctcc acctgcgggt ggatggtggg 30600
ctggtagttg tacagcagcc gcggggacca gttgtaggcg aacgcgaaga ggtgcgtgtc 30660
caggagagag atggggaaga cgccgccttc ggtggcgcgc cggaggatgg cgagcttggt 30720
ctccgcgggc agcaggctcc cggtcaccgc gccgaagaac atggggtggt tgcggaactt 30780
gagcgcgtac gcgctcagca cctcctctcg cgagaatatc gagtccgcgg ggttggagct 30840
cgcgggcagg agcacgtcct ccacgctcag cacctcgtcg atgaagggca gcaccatgtc 30900
cacgttggag gcggtgaacc actccatgtc cacggcgttg cgctccacga tcacccggat 30960
cgtctcctcg gatatgttct cggcgaaggc gctctgcagc tcgatcaggt tctcggtggc 31020
gtcgatgctg agcccgaggc gcatgccctg ttgcagcacg tcgttgatgg ggttcacgta 31080
catggccacc gccatgcacc cggccggctg cacgcgccag aggttggagt acgcggacac 31140

gtccgtgatg cgcagcgtct ccagcacgtt gtacatcgcc gcgcgcattt ccagcgtgtc 31200
cacgtacacc tccgcgtgct cgaagatgat gtcgagctcg aacgcggaca gcggcagact 31260
cacgtagatc tggcaggcgt caaagagttc ctgcgcgtac tcagagaagc acgcgtacgc 31320
gatcacgccc gcgcggttga cgatgtagtc cgccttgaac atcttcgtga ggtaggcgta 31380
cagcgaccgg caggccacgt gcggccttag ctcgtcgagc acggcgtcca gcacctcgcg 31440
gtgctgcagc acggagggt gcaggaactg cgtgaagtcc accgcggtct catccatgaa 31500
gtcggggatg gtctcgacca ccaagcggtg gttccgcacc gcgcggaagc cggtgttcat 31560
cggcgcgatg ctgtgctggt cgtggacctc cagcaggatc tcgtagccga tctctcggca 31620
gctcagcgcc gcgcgcgcct ccgtcacgct cgcgttccgg aagaactgcc gcaggtgctc 31680
gtccgtgcgg cagagcgtga cggattgggg gatgcgggaa aggtcgcaaa gagggctgtg 31740
gacggagatg cgccgcagcg cgtggtctac gaactcaaaa ccgcgcctcg acatcgtgaa 31800
gtcgcggagg gcgtacatgt tgtaggaaca gaggcggaag aggcagtcta tgtctagcat 31860
gttggaaacg cagtacgcgt gtctgtggag gtgcgggagc atggcgggca cgacctcggt 31920
cgtgttctgg agcatggtgc atacgaggtc gggcgctgtg aggtcggggg tgacgatgag 31980
gatgcaggag ctggagaact tgctgagctc ggaggccagc cggtgaaggt tgtagtcgtg 32040
gcgctggctg aagttgctgt ttatcgtgcc cgtgaagccc atgagcgccg ccagcgtcag 32100
gtcctcgcgc tcgatggccc agatgtctag gccggaggct atcatgcagc gcaccagcgc 32160
ggcggcgcgg tcgctcgtgg ggtagctcat ggtgctgtcg gtcgtgcgaa tcagcatggg 32220
ataatgcttc atttttacgg tcggggggttg cggactgtgg ggcgcacagg gcctgcgggc 32280
ggctcgtgcc ggtccgcggc gttcgccgaa cgcaggaacg ggcccatgcg cgcccaggcc 32340
atccacagcc ccgccgtcag cgccagcagc cagacgaata ccacgatcat cttttatgta 32400
gctggaactc gcgctcactc cccgccgcac ggcgacgggg agagcccaga gccgagctcc 32460
atgcgcgtgc tctgcacggt gagcgactcc acgagcttgg acacgttcat gcgcgtgttg 32520
tcgggcacca ggtgcgcgag gcgcgcgtac acgtcatcgt gcatgcgctt gctgcagcgg 32580
tccaggctcg cggagagcgc ggagactagc gcggtgtgct tcgcgtacac gaagtcgccg 32640
agacacacgg tctcgagccc gagcacgtcg gcgctctccg cgtccttgag cgccacgaat 32700
atcttctgct cctcgcgcgt catcgagcgc atgaggtagt cgtgcagccg cgagcgcgag 32760
atgagcccct gagagatctg cgggctgcgc atgaagcgcc ggcgcatcgc gcacagcagc 32820
tcctcgtcga cgacgtacat gctgtccttg atggagctct tctcgtcgat cacgagcaga 32880
ccgtcgttgg cgaccacgtt gatgaaatcg tccacgtgcc gcgcgtctat gtcgtagcgc 32940
gtgccgccgc actcgatgtg cgagggcggc gacttgagcc ggttcgcgag cgccttcacg 33000
tcggagacgt cgatgtacag cgaggactcg cgcgggacgc agccgaggat gcgcgtctcg 33060
agcggcgtga ggatgagcac gcgctcggcg ccgtcgacga gcttgccgtc gtcgggggaa 33120
aagaagttgt tctccacgat gctcgagacg aggctggcga gcacgccgtc gcggtaggcg 33180
ccgagcgcaa actcctgcac aaagggcgcg tgcagcaagt ccacgggaat gcgcatctcc 33240
acgcggcgcg cgaccgactt cttcttctgc aggtgccgcc ggtccaccat ctcgtccacg 33300
atgtccgata tgcgggagca gaggtacgcc ttgaggacgt tggcgtttac cttgttgaag 33360
atgagccgtt cgtcctccat ttaagctgct cagacgagct ttaaatagtg gaaacacagc 33420
agcacgccga tcgccgccgc tatcaggccg attagaaaaa cggtggtcca ggggacgccc 33480
ttgggcctat cgcacgccgg cttttcggtc attacggtgc gcacgatgtt taggaactcc 33540
tcgaagtcct cgtccgagtt ggagaggaag gagccgaaga cgccggtgta cagtttgtcc 33600
atttactact agatattaaa cggcgcttcc aactcctcgt cctcgaagcc cgcgccaggc 33660
tcgacgacgc ccaggccgcg cacgtcctgc tcctcgttga acgtggtctg agtctcgctc 33720
atgcgcacac acgtctgctc gccctcgaga ccgagcacgg tcagcgagca ctcgcgcggc 33780
atggtgatct tcttaaccgc gaaggtgact ttgccctcgc cgcccgagcg gtagaacacc 33840
accggcgcca ggatgagcgt cgccatctgc gcgtcgcggg ttgcgaggtt ttctatctct 33900
cgcgtcagcg gacatatctg cggctgggtg tcgtcgtcgc tggtgaactc caggagagcg 33960
ccggtcaggc ggttgagata cagacatccg gacttaaagg tgttgtcgat ggccgtttcg 34020
gtgttgaagt tgcgcagcga cggcggaacg cgagcgccgg ttttgatgtt gtcgtatatg 34080
ttctccagca gctggtagag cagcggactg gcgctcacgg gcttgaggcg accgaagtac 34140
ccgctgtcgt tctgccgctg catgtcggtc ttcttgctcg ggtagatctt aaactcgccc 34200
ttcacgacga tgagcggcga gacgagcttg gatgctagac tttcgacgag acacacgttg 34260
atgttggagc agctcgggta ctgcgacgga gttagagtca cggcctcgat gaccttggtt 34320
tggctcgacg agagcgactt agcgaagttg atcgcgtcgg tgcacgacat cgcctggttc 34380
tcgccgaacc gcctggcgga cgctgcatcc tcctgctgag gagcgcggtt agacgcgacg 34440
gtggttttgg atacagcgcg tttcattatt gcggcgattt taaagtacgt gtatactttc 34500
agttttgtcg ccgagcgttc agcgcctgca tgcagaggaa gtacaggatg atggtgcacg 34560
ggatcgtggt cagcagcgat acgaagtcca tcactgtgag gacgcgcagc gccccgcgcg 34620
agcggatgcc cagcgagggc gcgccgcggc gcgcgatggt ggccccgttc gtcaccacta 34680
ccagcagcat taggatggtc gcgcccacgg cgacgcccag gtcccgcgac tccatttata 34740
gtacagtata gagcgaccgc gtcacgaact ctcggctggc caacacgcgt ccgtcgggcg 34800
ggtgtccgcc ggccttcccg cggaactccg ggacctcgaa gctggacttc gtcacgcggt 34860
acgtgtactt gccgcgccag accaggtttt ccttctggaa gacgccgtcc atggtcacgc 34920

```
ccgccatgaa ggcgtccttg acgatgacca gcaccgcgtc tagcttgcgc ccgttgatgt   34980
gcgtgacgaa gtccgtgccg ctgcggctcg cgcagcggat gtccacgccc gagggcaggt   35040
ccaccacgaa cacgaagcgc ttcggcgcgt agagcaccag gtccgaggac ggcgacgccg   35100
aaggcgccga ggggaactgc cggtggtcaa aagggtgcac cacgcccacg atggacgtga   35160
cgcggtcgtc cgggaactgc gtcgcggcgc cgccgccgcg gtgccgcgtg accgtgcttc   35220
tgcccacgtc gtcgcagacc acgtgcagct ccgacacgat cggcagcagc gtggccagca   35280
tgcggtcggt ctctgtgcgc gtcgcgcagc ggtacgcgat cccgcagtgc gcgtcctgcg   35340
tgcgcccgaa gaagagcacc agcacgctcg cgtcctggtc gaagggacac acggccatca   35400
cgcccaccgg cggcggcccg tggcctgcgt acgcggagga gaactcctgc acctcgacca   35460
cggcgtcctc gcgcgcctcg ccgggcacca tcgccgccgc cggccgcagc gcccgcacgg   35520
tctgcttaac cgcacgcgcg gcggaggccg cgctcggcgc gactacgcgc acggccgcgt   35580
gcgcgcccgg cggcggcgcg gttccggcca tccagcccac cggcgagaag aacacgtcgc   35640
agacgtgcac gcccgcggcc tgcagcgcgc gcgcgagcgc gcgcacggcc tcccactcct   35700
cgcgaaaggc gctcgcgacc gcgagcgcct tcagcaccgt gtccacggag ttgacgggct   35760
tctggaagag gttctcgttg ttgtagatga actcggggag ctccacgggc actgtgaaca   35820
gcctaatctc gtgcgcgccg ctgggcgtga gccgcgtcgc gggcttgcgc acgccggcgc   35880
cgatctgctt gaagaagtgg ttcatggcgc cgccggcttc tcgggctccg gcgggagcag   35940
actatttatt cgggaggtta tcctttccga aagcacctgc acggacttcc gcgtccagcg   36000
ctccatcttc atgtactcct tcatgccgtc gctgagcacc tcgacggcct ccagcttggg   36060
cgctgtcggg tcgaagagga tgctcttgag cagcgtcatc ttcttgtccg cgaggaagcg   36120
gaagtaagtg tagatgcagc gcagcgcgcg gaagttctcc gggtgcttga tggtgcacag   36180
gatcatgaag atgcaggtga acatgccgca ctcggactcc atgagctggt tgacctcgag   36240
gttgatgcag ccgcgccgcg ccttgaagtt gtccacgaag aagcgcatga gcacgtccac   36300
gtcgcagttg cggttgtcca ggtccgcggt ctcggcgttc acgttgaagc cgtccgagaa   36360
ggagtagaag tagaagtact tgcaggggtg gaactccgag ggctgttgc cgccggagtc   36420
gtagaaggac acgagccgcg agacggtgtc gaagatgcag cacttccagt ggaacatgta   36480
gcagaagccg aacatcacgt agcgccgccc ggcgcgctcg atcttgtcct tgagcgtgag   36540
gctgaccatg ttgcagcgga agcggtccgc cttttcgtgg atggccgcgc cgttgaggaa   36600
gttcaggttg aactggccca ggtacgcgac ctcggtgccg aacgcgaagg gcgccaccag   36660
actctggatg ctcacgttgc tcatccaggc gctgcggtcg ggctttatgg cgatgggcac   36720
taccttggtg ttcacgcccg tgctgacgcc cgcgcgcgcg aggtcgtcca cgttcagcgg   36780
catctgcgag aagtccacgg cctcggacac cttctcgcgc aacgaggcct tgaagaagaa   36840
gcccagcggg accttccact ccagcgcgat cgcctcgcgg aagccgtagc gacccttgag   36900
gctggccagc aacgcggtct tctgcgcgac ctcgtccttc tcggtgtccg gcggcgcggc   36960
gtcgatgagc ccgcgcttcg cgaagtccag cagcgccgcc agcgggatgc acgagacgcg   37020
accggccgtc gcggattcgt cgaagcgccg caccacgtac ccgttgcagt tggtcttgaa   37080
gttggacacg tccaggtgcg cgctgagccc caccaccgag tagatgtggc acagaaggtt   37140
ggtgaacccc agctccggga ttttgctcac cactaaatcc gtgtacttgt ccatttatca   37200
tggagaatca tctgccggac atgctgatgt ttcccaactg cgtttctgtg tttccctttg   37260
agtactcgct ggaggacgtg ttccgcctcc ccgaggagcg acggcgcgcg ttcgccatgg   37320
ccgtgttccc gctctccaag caccgctgga ggggcgcgcg gctccagcgc gacgagcgaa   37380
gcgtgtggct cagcgtcgag gaggaccgcg ggcgcgcgct ggacgagcgg aactgctcct   37440
ggctctcgga cgtggccgcg cgcatggtcg acgacgaggg ccgcgcggtc acgcccgagg   37500
cgtacgcctt catgcgcgcc gcgcccggcg cgcgcgtcgc cgagctcgcc gcggacgcgg   37560
gcgtgctagc gggccttgtc gccggcggca acgcgctgcg cgtcttctcc tcggagtcca   37620
cgcaggcgcg cgagggctgg aaggcgcgca gcgtgggcgt gctcggcaac gcggcgccgc   37680
tggcgcccgt gccgctggca tcgctgcgtc cggaagtgca gcgcgagctc ttcgccgcct   37740
ggatcggccg ccgccccgtg gtgctcacgg gcggcacggg cgtggggaag acctcgcagg   37800
ttcccaagct gctgatgtgg ttcaactacc tcttcggcgg cttcgagcgc ctggacgccg   37860
tccgcgagtt cgcggagcgc ccgctcgtgc tctcgctgcc gcgcgtcacg ctggtgcgcg   37920
cgcacaccgc gacctacctc gcctcgctgg gcttcggctc ggccgacggc tccccggtct   37980
cgccgcggta cggcgccatc ccggacgccg agcggaacac ggccccgcgc gcctacgggc   38040
tcgtggtggc cactcaccgg ctcacactga ctgccatccg ccgctacgac acggtcgtag   38100
tggacgagat ccacgagcac gaccagatgg gcgacatcgt ggtcgcggtc gcgcggaaac   38160
tgggctcgaa catgcgatcg ctggtgctta tgacggccac gctcgaggac gaccgcgcgc   38220
gcctggagga gttcctggac cggcccgcct ttgtgcacat agagggcgac acgctcttcc   38280
ccatccgcga ggtctacgtg aagaacacgc aacagccgcc gctctcgcgc aagtacgcgg   38340
aggcggagct gcagaacgtg gcgcaggcgc tgggcacctt cgtccccgag cagggaaagt   38400
gcggcatcct cttcgtagcc acggtggcgc agtgcgcgct cttcgcggag accatcgagg   38460
ccaagcaccc cgggctgctg gtgcgcgtgg tgcacggaaa ggtgccctcc gtggccgcgg   38520
tgctcgagga ggtatacgcc gcggaccggc ccgcggtgct ggtttccacg ccgtacctgg   38580
agtccagcgt gaccgtgcgc accgccacgc acgtctacga cactgggcgc gtgtacgtgc   38640
ccgagccctt cggcggccgc gagaccttcg tctccaagtc catgtacacg cagcgcaagg   38700
```

gccgcgtggg ccgcgtggcg cccggcacct acgtgcgctt cttcgacacg cggctcgcgc 38760
tgccgctgaa gcgcatcgac tccgagttcc tgcacccgta cgtgctttac gcgcgcatct 38820
tcgggctaac gctgcccgac gacctgctcg tgcagcccag cgacctcgcg ctgctgcgcc 38880
gcaccgagga gtacgtcgac ggcttcggca tcagcctctc gcgctggacg cagctgctgg 38940
accggcacta catgcacatg gtcgagtacg cgaaggtgta cgtgcgcggc gggcgcctcg 39000
ccgccgcgct ggacgccttc gagcgcaccg gcgtgatgac gcacgaggcc accgaggcca 39060
tccgcgccgt ggacatgctc gcggccgtcc taaacgtgcg caagtccaag gaccgctacc 39120
gcgcggagtg caaggtgctc ttcgggccct tcgcgggcaa aaagttcgtg gtcgccgggc 39180
ggcgtccgcc cggctcgcac gtgctcatgg tcacagaccg cgtcttcatc gaggccgagc 39240
ccccattctg aggaccacct tcttggagac gcccgagaag tcgtcggcga cgccgcggcg 39300
cgccaccaca aggcagtacg aggtcacgtg cgggcagcgc gcgatgcagc ggaaggcttc 39360
ctcctgcgac agcgagaagg cgaacacgta gaaggtgtgc ggggacttca gcggcgtgtg 39420
gtccatcgag tagatgacac cgagcttctt catgcgccac ataagcgcgt tgatgtggtc 39480
ggcgcgcagc gcgcggccct tgagcacgcc gcagacgaag ctcgagcagg ccacgacgtc 39540
gtagcgcgtg ttcctgccga agaccaggtg cggcgcgccg gcggcgcgcc gcgcggccgc 39600
gcgattctcc acgatgtcct ctatggagcg ctcgctcgca aagaagtcca ggaacatgta 39660
ctggtaggcc acggccgggc gcgacttgct gaacttcatg aaggcgtccg agtccatgat 39720
ggcgtccatg tcctcagcgg cgagccggtg ctgcagccgg atgccctcga aggtgtggaa 39780
gagccgcgcg tccgcgtgca tggacagcgc gagagtgacg aagttgagaa ggtccgcgtc 39840
gccaaagcgc acgagcacgt taccgggcgt gcgcgtcttg cgcatgagcc gcgcgggcgc 39900
gccgtcgttg tggctgcggc ggcgcatttt gtcgccgggg gactcgggcg gcaggtcgat 39960
catgaccagc cggtgccgct gcgcgtcctc ggcgttgaag atcgaggacg tgaagcccgg 40020
gtacagcacc acgcagtcgc gctccgagat ggcgtgcagc acgtcgcgct tgagcccggc 40080
caccagccgc tccgcgttct cgacgaagta gttctcgtag tccaggatgt cgtgcgccat 40140
ccaggggaag ttcaggtacg cgttcatggc gtagtcctcg gcgtcgaagc agatgcgcgt 40200
gtctggcgtc gccgcgatcg gaaggtcctt gatgccgcgg agcagcccgt cgtagtcgga 40260
ctcgtccacg aaggagagca ccacaaagag gtcctcgccc acggtttcgt agtcgaagag 40320
gtggtaaagc tctcttagcg ccagcacggc gagcgcgttg tccagcgagg cgtgcacgcg 40380
cgccaggatg ctgtagaagg gcgtggccat catcacggcc ttgccgccct cgcaggcaac 40440
ggcgcgcggg aaaatgacct ccggcgtgcg cggcagccgc ccgaacgtcg cgttcagcag 40500
cgcgaccgtg gccgcgtcgc tctggcgcag gaacactacc accgaggggc ccgagatgct 40560
gagcatgcgc tcgcgcatgc gcgctggcag gtccggcgtg gtcacgaggt ccgcgaagcg 40620
gccgccgttg tagaggtcgc cgccgccgag gaaggtgagc acgtcgaagc agtgcagcac 40680
ctcgttgcgg aagtagtact cgttctcgag ctccttggcg tacgcgcgta tgtccacgtt 40740
ctcgaagttt gttcgcagac cgccgccgtc gaagaaccag gacgcgagct cgcggacggc 40800
gtccgcgggc ctgttgcggc ggctcttgca ccagaagctc atgtagttgc gcgaggtgga 40860
ggcgttcgcc aggaagaagc ggtggtcgaa ggagatgagc acatgctcga gcaggtgcgc 40920
gagccccagg accgcgccca cgtcgcgccc aaaaccgaag tttgatatcc ccaggtagac 40980
gtcccgtttc atagacggcc tcaggaacac cctgacgccg ttttccaaca ctatcattct 41040
ccggtattta cttacccaaa agtagtatgg ggagaagtgt ttgaacgtcc cctcgccttt 41100
ttaaatcaaa agtagacttc tcgcgcccgt gcgccaccgt cacgcgcgcg cggcgcgagt 41160
ccataccggc gatcaccgcg ctgctctgcg gtgcgtccgg ccgcgggaag agcacggtct 41220
cggagatccc gtccagctgc gcgtcggtgc gctgccgcca cgcgtgcgcg tccgcgagct 41280
cgcgcacggc cagctgcatc ttgttcgtcg gcaggaacgt gaacacgtac gccgccgcca 41340
ggaaggctgc gaagagcacg aactcaaccg cccatgacat ttagggagct gattttgttc 41400
cacgcggcga cgcacgtcgt gacgggcgac cccgaggcgc cgcggcgcgc ggcctcgctg 41460
tgccgcggct tcggcgtgga cttccgcgcg attcacgcgg agttcgcgcg gcggtacccg 41520
cgcaccgcgg ccgccgtgga gcgcgcgcag ccgctgcccg aagtcgatgc cgcctttccg 41580
ccggacgcgc gccggcaagt cgtgcggctg cgcctcgagg ctgcggcgct ggtcgtcaag 41640
gagtcgcgtg cgctatcggc ctccatgcgc ggcgtggcgg tggtcgacgg ctgctgcgtg 41700
cgcgtgtgcc gcgccaacga cgagctgcta gagttcctcg cgcggcgcta cgaccccgcg 41760
gtctaccgct acgcggaggt gccctcgccg agcgtgcgcc cgggctcgaa agtcttcgcg 41820
tgcgcgggcc gcagcgtcac ctttgcggcc gcgcaccgga gccgcatcac ggccaaccgc 41880
ccgctgcgcg tggtcgtgac cgaggcctgt gtggacggcg tgctcgcgcg cggcgccgcg 41940
gaggtcttcg accgcggctc cggcgtgctg ccccgcgcgc tgcgcgagat cttctaccgc 42000
ctcgacgagg acggctgtcc cacgggccag acgccaggct tcgcggacag tatggcgtcg 42060
cgcagctgat ctatgtccac ctttttctcg tcgatctgcg ccacgaccac gaaactgcga 42120
atgtccacag cggccatggt cttggccacc gggtcgtact tgaggagcag cacgtactcg 42180
ttgccgaagt gctcggtgac ctcggtgatg agccggtaca cgcccatgcc gagcacgttc 42240
accgcaccgt ccttggcgaa gagcgagagg atgttcacgc acttcagctc catctcgccc 42300
tcgaggcgcg cgagcatgcg ccgggtgacc tcgcatactg aacaaagagg cttacctagt 42360
aagataagcg ttagcttagc cgcggtcggt gacgcgtcgg aggccattta tggggatcaa 42420
aaacttaaag gcgttgctgc tcagccacgg cgcgctgacc ccgcacgagc cgggcggcga 42480

```
cgagcgcttc cctgccgtgt tcgtggacgg cttcagcgtc atgatgacca tggcgtactc 42540
gtgcgcggac gaagacgagt tccgcgcggc cgtcgaggag cgcgtgcagc actggatgag 42600
cgtgtccgag agcgggcgga tcgtggtctt cctcgaccgc ggcgagattc cgatcaagca 42660
gccgctgcgc gaccagcgcc gcaaagccac gcgcgaccgc gccgcgcgcc accgcgagtt 42720
catcgccgcc gcggaggcag acgcggcggc agaggccgtt ggcgcccgcg aggacaaaca 42780
ggaggacgag cacgcggagt tcgccgagga gatccgcgct gagaagcagc taaagctgca 42840
gcgcatccgc ttccagctca gcatcgccaa ccacgaggtc gttaagtcgc tgatagagtc 42900
cacgctcgcg cgcgctggcg atgccgtgga gatcgtcttc tgcgacggcg tcgacgcgga 42960
gatggtcatg tgcgcgcgcg gacgcgccga ggccgagcgt cgcgggcgct ggccgctgct 43020
cgtgaccacg gaccaggacg cgcttttgtt cacgtccacc gatcgcgacg agaagatagt 43080
gagcaccgtc tccgcctgct acgcgttcag gcccaccgag acgaccgagt acctgtgcaa 43140
acttgcggcg ctggccaacg gctgcgactt cttccccggg ctcggcggca tatgcgtgag 43200
tgtggagtcg ctgcgccgcg ccacgctttt cccggaattc tccgtgcgca acgccgccgt 43260
gagtctgtgc acgcggccca tgcggctgtc cacgcaggac gcgctggagc cagaggccgc 43320
cgccgaggtc gtggaattca tcaggcggta cgccgccggc gacgagcgca tctaccgcga 43380
ggtgccgccc ggcgcgtgct gcggacgcgc gtttgtgcgc ggagcgctcg cggccgagtg 43440
ggccgacgcg ctgccggcgg ccacgggtct gagcgtggtc gcggacatga tcgcgtgtct 43500
gcccgcgcgg cgggaccccg cgcccgagga ggtagagcgg ctgctggcgc tggaggcgcg 43560
cgcgcgaggc gcgcgcgtca cggatgcgat gctcgcgcag actgcgcagc tgctgggtta 43620
cggcgcgagt gcgggcgccg acggcgcctc cgccttcgcg gtctcgggcg ccaagggcct 43680
gatgtgtcgc ctgcgcggca cggccatgtt cttcaacgcg gagtacgtgg aaattgaaag 43740
cgaacccaga ctgttaaagc tgcggtagca tggtgttccc gatcgtgtgc tcaacgtgcg 43800
gccgcgacct gtcgcacgag cggtttctgc tcatcgtgcg acagcggccg ctaaaggttg 43860
ttttgcggac ggtgcgcaac gtctgctgcc gtataaagtt gtctacacaa atagagccgc 43920
accggaacct gacggtgctg cccatgctcg acataagctg atttttcttt tccgctcgta 43980
tgcgcgagtt cggactcgcg gcgcgcatgg cccgcgccat cgaggacgtg tgtccgcgcg 44040
gcgcggtgat attcgtatcc agcgccgcgt ccatgaccga ctgcctgaac ccgtcggtgt 44100
tcaagcacgc ggcgatatac gcggggcgcg tggaccgcgc gccgctgccg ccgccctcgc 44160
cggtcccggc ggaggccgtg acggagccct gtgcgataga cgccatagcg ccttacggcg 44220
cgcgcgtggt cctgctctcg gagctgctgc ggagctgcgt ggccgttcag gcctaccgcc 44280
tggcagtccc cggcgccctc gcgctcatga acctcgcggc cgacgcggcc ttcgagctcg 44340
tgggcacgcc ctacggcttt aacagcgacc gaacgtactg cttcaagctc gttgccgact 44400
gctttgctag cgtgggcgtg acaacgaaga ccaggcgcat catgggtcgc gacgtcgtgc 44460
tcagccagga cttcctggag agcggcatgt ggaccaaggt gctggactcc gccgcggagc 44520
cgccgtggct ggtctagaac agcggcggcg cgcgggtccc gagcacgggc cgcgccacct 44580
gcagccgctg ctgcagcgcg cggcactgcg cctcggcgtc ggccgtctcg gcggggtcga 44640
cgggcgtcgg agttgcggag gtggtcctga acggctgcgt gttcaccgag acgcggatgc 44700
gctccttgca ggagcgctgc tcgatgcagt tggccagcat cttcatcacg tgcaggtact 44760
ccagcaacac gaacttttcg agggtgatgc cgtcgaaggg cgacgacccc accacgccca 44820
gcgggctgga caccgcgccg tcgagcacct cgccgcggga ctccttgcgc gcgcgctcga 44880
gcaggtcctc tgtccgagcc accacactgc cgaagtcggc ggccgcgggg gcgggaacag 44940
gcgcagcagc gctgtccgcg tccgccggca tctcctcgat cttgagaccg gccgcgaact 45000
ccgaggccgc gtgcacgggc gaggcgccgc gccgcaccat gaagtcgcac agacgcgata 45060
gcgcggagga gcgcaccggc atgtcgagca ggcgctcggc ctccatctcg gcgaccgagt 45120
cggcgcacgc gtccggcgcg cccgcccgca cgagctcgtc gcagcacccc gcctccttca 45180
tgagcgcggg catgagcttg tactgcgcca tgttcaccag cccgtacttg agctcgagca 45240
ggtccgcgag ctcggaggcc atgggtcggt tttggtgta gatgacgcgc tccacggcct 45300
ccgccatgtc cacggcctgc atgagctcgc cgacgagcac gctggccacg agcgtggcca 45360
gcgtgacgcg cacggtgggc acgcagaccg cgaagaagga ggtggagtgg gtgaagcgca 45420
tgagcgcgcc gtgcagacgc gcgaggtccg cgctgttgcc cgcgtgcacg aagcgccggc 45480
gcagccgcgc cagcgcctcc acaaggtcct ctcgcgtggt cacgcgcacg ttcgcgatgc 45540
acaggtcgtg gatcgcgttg gcgatctgcg cgcggcgctg cggcgagctg ccgggcagca 45600
gccgcgcctt ggcctcgacg tcgacggtgc tcgagagaca gccgcaggcg gcgccgcgga 45660
cgacgaactt caacaacgac tcgaacacgc gcgcgcccgc gcggggcgct tgcttggacg 45720
actccattta ctttaaataa tttacgagat caaaataaaa tgactctgcg catcaaactc 45780
gagaagctca agcagatcgt aacttacttc tcggagttca gcgaggaggt ctcggtgaac 45840
gtggacgtcg gcgatggcct catgtacata ttcgcggcgc tgggcgggtc cgtgaacatc 45900
tggaccatcg tgccgctcag cgcgagcgtg gtatacgacg gcgatgtcag ccgcgtgttc 45960
aacctgcccg tgctcaaggt gaaggcctgt ctgtgcagct tccaccccga ctcggtggtg 46020
agcctggagc ccgacctcga ggacaacgtg gtgcggctct cgagccacca cgtggtcagc 46080
gtggactgcg acaacgagcc cgtggcgcac cgcacgaaca ccgccatctg cctgggcatt 46140
aaccagcgca agtcctacgt gttcaacttc cggcgctacg aggagaagtg ctgcggccgc 46200
accatcgtca acctggacct gctgctgggg ttcatcaagt gcatccacca gtaccagtac 46260
```

atcacggtct gcttccgcga caagaagatg gtgctgcaca cgcccgggaa ggtggacaac 46320
ttcttccgcg agtactccat gaccgagtgg gcgcccgacc tcgagcgctt ctcgttcaag 46380
atccccatct cctccgtgaa caaactccgc ggcttcaaga agcgcgtggt catgttcgag 46440
tcgcgcgtgg tcatggacgc cgacgacaac atcatcggca tgctcttcac cgaccgcgtg 46500
ggcatgtacc gcgtgaacgt gttcatgtcc tttcaggacc ggtctctttc atgcgactaa 46560
atactcatgg gcgggtcggt gagcctgccc tcgcgggacc tgccgccgcc ggtgcgcacg 46620
ccggagatga acatcgtgcc cgagcgcgac ctcgcggaca cgatggcgcg cctctccacc 46680
gcagacccgc cgcagccgct gggcgtcggc gacgacgcgc gcatggccgt gctgaagacg 46740
accttccccg agttcgcgat atcgcggccc gcgacgggca tgctcgccgc gcagcgaatc 46800
aggtacgacg gcgacccgcg cgtctgctgc ggcgggttcg ggatctcgca ttactgggag 46860
aagggggcgc gccgatcgaa cgtcgcgttc gagggcgcgg cgctgcgcac ctgcgacccc 46920
acgcgcttcg acgcgggcgc gtgcgacgcg ctgctcttcc gcgagtgcgc cgccggcggc 46980
gtcgacgcgg acttctgcgc gcactggatc aacgcggccg tgacgcggcg cacggaccga 47040
cagtcgcgcg cgcggctgaa cgacatgttc gtgcgcgatt gccaaaacga cgccgcccgg 47100
cctcactgcg tggcctggat ccgcgcgatg cgaagcgcgc gcgcgacggc ggacgacggt 47160
ctaatagacg ccgtgctctc ggtgcagagt cccgagttca agggcaagca catgcgctgc 47220
agctacccct cgccggccac tctcgccatg gccgcgaacg tggacgagcc gcgcgagtgc 47280
tgggaccccg agtgcgtggc cgggaacgtg gacttcatgc taagcgataa ctacacgaac 47340
ctgggcttgt gtcggctctc gcgctgctcc atcggcgtca cacacctgcg gattgacgcg 47400
cgttcgcggc tgcgcatgcg gtgcgccggc gcgcttgccg ggctcacgaa ggcgcccgtg 47460
aaccagactg tcgtcgtcgg cgacaacctc gcgcgcgcct tcgagccgcg cgtggaaacg 47520
ctcagcgtgt tggcgctgtg cgtggtgtat ctgctaattg tctggctcta aatgggggcc 47580
gccgccagca ttcagaccac cgtgaccacc gtcagcgagc gcatccgcaa cgagctcgag 47640
cagagcgcga gcgctagcgc gaccgccgac tgcgacgtca ccatcgggag tctgattatc 47700
cgcaagaacc taggatgcag cgtttccgtc cggaacatgt gctcggccaa cgccggcgcg 47760
cagctggacg ccgtcatgaa ggccgtgagc agcaccttca acgacctctc gtcggaccag 47820
aaggcctacg tgcccgggct gctcacggcc gcgctcaaca tccagaccac ggtgaacacc 47880
gccgtcaagg acttcgagac gtacatgaag cagacctgca cggcggacgc ggtcgttcac 47940
aacaaaatca agatccaaaa catcgtcatg gaagagtgcg cctctctgcc agggagtccg 48000
gccacgcacc tggatttcgt gaacaccggc acggccgtgg gcaactgcgg cgtgaaggcc 48060
gtgatggacg tgctcgcgaa ggccagcacc accgtgcgca acgaccagga ggccggcaag 48120
ggctaccaga ccatcatcat cgcgatcgtg gtcgccatcc tggcggccat cttcgcctgg 48180
tacgcgcggc acatgctatt catgtccacc tccgacaaaa tcaagctcga gctcgccaag 48240
aagcccgtgg tgcactggac cacctacctg gacaccttct ttacggaatt tccgccgtcc 48300
gtctagatac gcgcaacatt gaaacattat atccacctct caaacggcgg tatggtccga 48360
cgcgtcctcc tcgagcgcgt ggacggcatc gtcgagcact cgcgcgcaga ccgacgctac 48420
ttggaggcca ttcagcgaca cctcgagggg tctacgcccg ggctgcggca gatgtggcgc 48480
ttcctctacg acctgctgct gacggtgttc gtcgtcatgt acatcgtctt ccgcctaatc 48540
gtgcgcaacc ccggcatctg cgccatcctc gcgctcgcgg ccgcggtgta ctacctgttt 48600
ttgtgtctct ttagcatgga ctgatggcga tcacagacag accatcgccc gcgcgcgcgt 48660
gaccagctcc ggcgccgcga agacgtcctg caccgggaag tcgtcgatct cgaacacgga 48720
gccgtccgcg gaccagatca cgcgcacgtt gtcgctcacc gagacctcgg tcagcgtcac 48780
gcccagcaca accgcgtcgt tggtgctcac cagcaccagc gcgccgggct ccgcgcgccg 48840
gtgcagcggc ggccccgaga ctgagcgccg ctgcacgcgg aacatgtccg cgaactgctt 48900
cgagagcaag tccaggtggt tgcggatgat ccactcgaag aagtacgcgc aaccgccgcc 48960
gccgcacagg aagcgcgaac ccgcgggcat cagcagccgc acaacgtcca tgtagcaggc 49020
ctgcggcagg ctcgcgcggt acagccgcgt cttcggcgag agcaccacca ggctggaggt 49080
gctcatctgg aagaccagct ggctaacgga gacggtgagc gtgcacgcgg gcacggaaac 49140
cacgtccagg cagatgtcgt ccagaaagat gctccgctgg tagaggtggt acaggatggc 49200
cacgatctga aaggccgtgg cgtcgctgat ggcgcagggg cggtcggcgc agcgcatctg 49260
cgcgcaggac cagcccccga aggactcgaa gcagacggtg agcatgcccg tgctcggaca 49320
gtgtggcgag cgccgacaca ccggaaagcc cacggccttg cggcagcgca ccatggtcga 49380
gagctctatc cagcagcctg cctcctcctc gcccatgccc atggctaccg gcgtgaaggc 49440
cgtgacgtcg tcgcagatgc gccgctccag aaaccccacg cccgaggagg ggtgcgcggc 49500
cggcggcgag gtgatgcgcg ccgggacgcg gctcggagcg ggctcgggag gcgagctgcg 49560
ctcgacccgg gcagtcgccg ccggccgcga tgccctgcgc gcgggcgcgc gctcgcgcaa 49620
cttgtttgac ttgctggcct cgtcgctagc gtcatcgaag cggtcgttcc tgtcgccgcg 49680
gacgtccgcc tcgtcgcccg tcggctgcgc ggcgggcgac gtgccgtccc gcgtacggcc 49740
cgcgttcggc gcgaatgtca cgcgccggtg cacgtacggc tccgtagagc ccgtgggggc 49800
gccgcgcccg cgcccgcggc ggaaggcctg ccgggacgcg ccgaagcggg cgaactcccc 49860
cttcgcccgg cccctttttt cttccatgat atttatcaca aaaaaaactt ctctaaatga 49920
ccaatctgct ttcgttggtc gacccggagg acctggcctt ctgcgccggg ttcccgtcct 49980
tcgacgagac catgctcgtg atcgcggggg cgcgagtgcg cttcccacgc tcgctgctct 50040

```
cgctcttcaa cgtggtgccg cgcaccatga cgcgctacga aaccgagctc gtgggcaccg 50100
agatggtggt gggcgccgtg ttcaccaccg cgtacaacgt ccgccgcaac ctaggcctcg 50160
gcgaggagcc cgtgaccatg cgcgacatcg agaagtactt cctggactcc gagaacgagg 50220
tgctcacgct catcgtgcac aacaccgact tttccgccat gagcggcgtg cgccggcgcg 50280
gcggccggcg catcgccaac cccgtcatct tccgcagcgg gtccacgccg ctgctcatcg 50340
tgatggagtc gcgcaagaag accaacatct accgcgagcg caccgcggag caggccaacg 50400
cctcctacag ggaggtcggc tcctcgctcg cgctggtcac tcggtacgcg ggtctgcagc 50460
tggttgacgt gcacacgccc agctccgtgc taacggtctc cgccgtctac ggcttcaccg 50520
aggacaaggg gctcaagaag ctgggctccg acaaggagct cgcggactac cagtccacgc 50580
cgctcaccga ccccatccgg ctcagcgact tctccaatat attcgacggc gtcaagaaga 50640
gcatccagct cacgaacgtg cccgtgccct ccaccggcgc cgaggccgcg ccgtaggctt 50700
tcatgcgcga taaatcggat ggcggcgccg acgacgcccg cggtgcacct cacgccggtg 50760
ttcgtggagc ctacgatcgc gcactcgctg ctgcgcgcag agtcctacct cgcgatcgcg 50820
gtccttgagc tcgtgctcgc gctcgcgctc gcgctcgtct tcttccgcga cgagctaggc 50880
tcgctattcc gctgcgcgcc gcgagcgcct tcgccgctgg acgcgtacct gcaggcgagc 50940
ctcgtctgcg acggcgacgc gctgctgatc gagctgcccg agggccgggt gccggcgctc 51000
gcgctggacg ggcggcccgt cgcgttcccg gggtgcgaga gccttttgta ccgcataaat 51060
ggaccacgaa aagtacgtct tgtcgatgtt cttggaggaa gataactcct tcttctcgtt 51120
cgtcgccgcg ctgtccgatg acgaggcgct cggcgccgtg cagtccgctg ccgccctcct 51180
ggacttcctg ctctccgtgg tggtccgcgg caaggagaag ctcgccgccg cggggcacca 51240
ctacgactcc atcgcggacg gacgcgcgcg cgccgcgttc gagttccgag acctgcgcga 51300
gctggcgcag ctcttcgacc ggcggccctg cggcgtccag gaccgcgtgc gtgtgcgcga 51360
cgggcccgcg cgcgccttcg tggacgcggc actggggctc atgcgcgagc gaggcttcga 51420
cggcacgcag gccgcggagc gcgcgcgcta catcgcgccg aacgatctgc ccgcgctggg 51480
ggcaatatcg gccacgctct cgccgggtct ataacgtaaa aaatattagt aaaattctga 51540
aggtccgtgt gtttcgcggg cggccaacaa accagtcgct taaatggagg gggtggaaat 51600
ggacaagccg ctcctctact tcgacgagat cgcgggcgcg cgcgactacg acgcggcctt 51660
cgcggagaag cacgagccgc ccaagatccc cggccgcgga cagatgaagc tgctggtctg 51720
cgagctcgtg tttctcaacc ggctgcacct gcacggcatg ctcgacggca gcgtcatcgt 51780
gtacgtgggc tccgcgcccg gacggcacat ctgctgcctg cactcgcact tccaggagct 51840
cggcgtctcg cttaagtggg tgctcattga cgggcgcaag cacgacccct gtctctcggg 51900
gctgcggaac gtgaccacgg tgacgcgatt cgcggacgag gcctacctcc gcgagctgcg 51960
cggcgagctg cggcgcgcca agatcgtgct catttcggac atccgctcca accgcgtgga 52020
cacagagccc accaccgcgg acctgctgcg cgactacgcg ctccagaaca ccatggtgag 52080
cgtgctcaag cccgtggcct ccagcctgaa gtggcgctgc cccttcccgg actcctggga 52140
gaaggacttc tacgtgccct gcggcaagga gatgctgcag ccgttcgcgc cgccgttctc 52200
cgcggagatg cggctgctca ccgtgtactc ggagacgcgc ccgaagctgc gtctgatcac 52260
gctcagcgac gcggtcaact atgaaaagag gatgttctac ctcaatagcg tggtccgcca 52320
gcgcgtaatt ctgaactttg actatcccaa ccaggagtac gacttctttc acatgttctg 52380
tctgctctcg tcggtggtgt gctcgtgcga atttaaatcg cccaaagaga aggtgctgag 52440
cctgcagaac cgcttcttcc gcttcctgcg catcccgccc tccatcacgc tcggctgcg 52500
ccggcacgat gaaccgccac aacacgcggt acctggccaa gatcctctgc ctaaaggccg 52560
cggtaagaag cgaccccttc gcggtggtaa gtagggacac cgtgcgcatg tacgacatcg 52620
aggtcgagta cggcgacctc gtgacggtgg tcaccgtcac gcacaaactc gagaccagcc 52680
gcaccgtctt ccaggtcttc aacgagacct ctgtcgcgta ctcgccgctg ccggacgact 52740
acggcgagcc catcgtgctc accacgtaca tgcagcgcga gcacaccaag ttcccgctct 52800
ccatgctcta catcgacgtg gtcgcctcgg acatgttccc cacgtttaag cgccccaccg 52860
aggaggaggc cgcggtggtc gcggccatgc agcgcgtggg cgggcgccgc gagcccgtgc 52920
tcaagctccc gcgcatgctg gacaccgagc tcgtgtgcaa gatactgcac ctgcccgagc 52980
acccgctgcg cgtggtgcgc ttcctgcgcc gaaacatgtt cacgggcgtg gaggtcgccg 53040
accgctcggt gtccgtggtc ctcgactgac gaagggcagc acggtcagcg aggccgccgc 53100
caccaagcac agcggcagcc acgcgcgcgg gtccgccacg ggcacgaaga cgtgctggtt 53160
caggtacttc gcctggaagc gctccgcggt ggagtccacc ttggacccgc aggcgttggt 53220
gaggcgcacg accgcgtccg cgacgcgcac gtccccgagc gatatcacgc agtcagagac 53280
gttgcacccg gcgatgtttt tcttcagcgc gcgcggcagc agcgcgtccg cgcgcttgca 53340
gggcgcgtac cagcagtagt agggcaggcg cgtgtcgcgg ccggtgtcga ccacggcctg 53400
gctgggcttg aggcacgcgc agcgctcgtc gtccgggtgc gcgtcgcaga aggcgtaaat 53460
ctcctcgtcg ggcgcgtccg gcccgggcgc ggtcggcggc gccgcgcgac ggcggaagaa 53520
catctctgaa aaaatacttc gaccagaaaa cgaccaccga tcttatttca aagataaaaa 53580
tactattaat acgcactcgg agaatcatgt cggtggtggc gcgcgtgtcg tacagcctgt 53640
actcgcagag cgagataagc gccacggacg tggtcatcag ccagttgaag aacgacgagg 53700
acctgggcac ggtgaaggac ccgcgcctgg gcgcctcgga cgggtccata tgccgcacct 53760
gcgggctcac ggagatggag tgtttcgggc actggggcaa ggtgcgcatc tacgagtcct 53820
```

acatcgtgcg ccccgagtac atccccgagg tggtgcggct gctcaaccac ctctgcgtgc 53880
gctgcgggct gctgcgctcg cgcgacccgt acacgacgga cttggccgcg ctcagcgtgc 53940
acgagatgcg caagatgaag gaccggatga tgtccaagaa gaaggcctgc tggaacagca 54000
agtgtctgca gccgtaccag aagatcgtct tctccaagaa gaagatctgc ttcgtgaaca 54060
aggtggacga gatacccgtc cccaacgcgc tcatctacca gaagctgacc tccatccacc 54120
gcaagttctg gccgctgctg gaggtgttcc aggaccccgc gaacctgttc tacaaggagt 54180
acatgcccgt cccgccgctg ctcatccggc cggcgatcag cttctggata gacaacatcc 54240
ccaaggagac caacgagctc acctacctgc tgggcatgat cgtgaagtac tgctccatga 54300
acgccgagga gcaggtcatc cagcgcgccg tgatcgagta cgacaacatc aagatcatct 54360
cctcgaactc gagcagcatc aacctctcct acatcatcgc gggcaagagc aacatgctgc 54420
gcagcttcgt ggtcgcgcgg cgcaaggacc agaccgcgcg ctcggtcatc gggcccgact 54480
ccgcgctctc ggtgtgcgag gtcggcatcc ccgactacat ccggaacacg ctcacgcaga 54540
aggtgttcgt gaactacctc accagcaagc gcgtgcgcgc gctgttcgag gaccgcgcgg 54600
tcaagttcta cttcaacaag cggctgcgcc agctcacgcg catcaaggag ggcaagttca 54660
tcaaggacaa gatccacctg ctgcccggcg actgggtgga gatccccatg tccgagggca 54720
cgaacgtgat attcggccgc cagccctcgc tgcaccgaca caacgtcata tcctcgaccg 54780
cgcgcgcctc gcccggctac accatcaaga tcccgcccgg gatcgcgaac tcgcagaacg 54840
cggacttcga cggcgacgag gagtgggccg tgctcgagca gaaccccaag tccgtgatcg 54900
agcagagcgt gctcatgtac ccggtgacta tcttcaagca cgacgcgcac ggcgcgccgg 54960
tgtacgggtc catccaggac gagatcgtgg ccgcgttctc gctgttccgg caccagaacc 55020
tctcgctgga cgaggtgctg aacctgctcg ggcgctacgg gcgagacttc gcgccggagc 55080
ctggccagaa gaccttctcg ggcgccgacg tcttccgatt catgataggc gcggacataa 55140
acttcaaggg cgtgctcgag aacgggcgcg tggtggcgcc gaacgtcgac agcgacctcg 55200
tggtggccat gcgcgcaacc tcgctagcgg ggctgatcgc ggactacgcc acgaacgtgg 55260
agggcgtgcg cttcgtggac atggcctcct acgtgtacaa gcggtacctg gccatctacg 55320
gcttcggcgt gaccttccgc gacctgcgcc cggacccgag tttggttcgc cggctgcacg 55380
cgctgaacac cgagaagata gagcagatca aggacgcgta ctcgcggtac ctgcaggacg 55440
tcgcggacgg gaagctggtg ccgatggcgc ccgcggacga ggccgacgcg ctggactcgc 55500
tgctggccaa cctgaccaac ctcaacgtgc gcgagatcaa cgagtacatg cgcgagacgc 55560
tggagcgcaa ccccgataac agcctgctaa agatggcgcg cgccgggtac aaggtcaacc 55620
ccacagagct catgtacctg ctgggcacct acgggcagca gcgcgtgaac ggcgccgtcg 55680
ccgagaccaa gatatacggg cgcgtgctcc cgtacgcgtt ccccgactcc gcggacccgg 55740
aggcgcgcgg ctacatcatc aactcgctca tgaacggtct ctccggctcg cagttctact 55800
tcgcgatgct ggtggcgcgc tcgcagtcca cggacatcgt ctgcgagacc tcgcgcacgg 55860
gcacgctcgc gcgcaaggtc atcaagaaga tggaggacac ggtcgtggac gggtacggac 55920
agatcgtgag cggctcggta ctgctcaagt acgcggccaa ctacgcgaag atcccggggt 55980
ccaccaccaa gcccgtggag ctgctcttcc cgcacgagag catgacctgg ttcctggaga 56040
taagcgcgct ctggacgaag atccggcacg ggttcgtgcg catgcaccgg cagcgcctgg 56100
ccaccaagat cctggcgccg ttcaacttcc tggtcttcgt gaaaccggcg ccctcggagg 56160
cggaggcgct ctccgcgcgg gacctgtacc acatgatcca gcgcgtgatg aacgacgtgc 56220
gcgagaagta cttcttctcg ctggcgaacg tggacttcat ggagtacgtc ttcctcacgc 56280
acctgaaccc ctcgcgcgtg cgcatcacgc gcgcgaccgc cgagctcatc ttccgcaagc 56340
tgtaccagaa gctgaacgcg ctgctcggcg gcggcacgcc cgtgggcatc atgtccgcgc 56400
aggtgctctg cgagaagttc acgcagcagg cgctctcgag cttccacacc accgagaaga 56460
gcggcgccgc gaaggtgaag ctgggcttca acgagttcag caacctcatc agcatgagcc 56520
gcaaccacac cgagatagtg gcgctgaccg cgccgagcgc ggacaagctg atgccgctga 56580
aggtaaactt cgagttcgtg tgtctgggcg agctcgtgcc cgagatcgag acccggccct 56640
cgggacggcc ctccgtgcac cgcgtggaca tcacggtgca ccgcctgcgc atcaagcgcg 56700
cgcacctgac cgaggtcctg gtggacacca tcatcgagcg cttcgtgtcc ttcaacgtgc 56760
tcgtgaagga gtggggcagc gacatgaccg tggagggcga ccgcgtcacg tacacgctgc 56820
tgctgcgctt cgtggagccg gagcagctca acttccacaa gttcatgctg gtgctgcccg 56880
gcgccgcgaa caagggcaag gtgagcaggt tcaagatccc gatcaccgag accacggtct 56940
acgacgactt cgacgccgcg cgcaaggcct accgcatgaa catcgagctc atgagtctga 57000
aggagctggg gatattcgac ctcgaggacg tgaacgtggt ccccggcatg tggaacacct 57060
tcgacatatt cggcatcgag gccgcgcgcg ggcacctctg cgagagcatg ctggacacct 57120
acggcacggg cttcgactac ctgtttccct cctgcgacct gctcgcgagc ctgctctgct 57180
ccgggtacga gcccgagtcc gtaaacaagt tcaagttctg gaacgcgagc gcgctgaaga 57240
aggccacctt cggcgacggc cgcgcgctgc tgaacgcggc gctgcacaac cgcaccgacg 57300
cggtcgcgga caacagcagc tgccacttct tcagcaagac gccctgcgtg ggcacgggct 57360
actacaagta cttcgtgaac gtggagatgt tcatgcgcat ggagcgcgag atccaggcgc 57420
gcgtggcggc gcgcaagatg gaggagatcg aggaggccgc cgaggaggag ttctaggcgc 57480
gacggcgcct tactttgcga ccgtgtcacg acgacacgac acggttagga cggcgagtcg 57540
cagacgaaca tttttatgag ctggtagcgg aagttggcgt tttccaggaa ggcgccgcgg 57600

aggtcccgga tctcgtagta ggttttgagg aagtacacga agcgcgcggg ctgcgtcata 57660
gtcgggttct ccgcaagccg cttgtgcatc acgtacccca tggcggcggc gccgctgcgg 57720
ttgacgccgg ccacgcagtg cacgagcgtg ggcttctgct cggcctcgag gcgcgccagc 57780
agcttcacga gcgcgggcat gatggaagcg atgttcgtcg tgtcgtcgtc tctcagcgga 57840
atgtggtacg ccgttatccc cgcgggcgtc gagtacttgg acatggtcat gttaaccaga 57900
cacttgaagt cgacgccgga gtcccccgc agcacggcgc gcgcgtcctc ggcgctgccc 57960
aagtacacgt ggtccgtgag ccgcgtcatg cccgagggca gggccagcgg cggccccgcg 58020
cgcgtgcacc gcagcaggag cctggcgtac cactcgctct tatcgcccat atttatttat 58080
atgatacaaa tggcagacgt cacaacactg acggccaacg gtctgaccct ggagttcgcg 58140
cgcgagcgcg ctctgcgcag tctgcgcgcc gcgcgcacct ccacgctggt gttcttcacg 58200
ctcacgctcg cggcctcgct gttcgtgctc tggctgcagc taaccgagtt tcccgtcttc 58260
gaggagctcg gcaagtacgc gcgcatcaag agcgcggtgc ggtcctggcg cccgctggtg 58320
gaggctaaga cagagatcga gtccgacctc ggccggcaga agaccgccga ccggcccgag 58380
ctcttcgagt tcaggtgcgt ggacttcggc aagttctacc tgccggtgag gtacagcccc 58440
acgaccttcc tgccgcaagc cgtgcgccgc ggcgcgggcg atggctggat ggtgcacaag 58500
gcggcggccg tggacctcgc cgcgcagcag ttctgcgagt ccgtgctgcg gcaccgcgcc 58560
aacaacgtca tcacatgcgg gtcagagatg atgcggctgg tgggctacag cggctacttc 58620
gaggacgacc actggtgcgc cgcgacgtcc ggcgtgctga cgtgaacgat cacacgatgg 58680
ccgtgaccag cagcccggcg atgaaccaca gcagccgcga gttcggcagc agcagcacga 58740
gcaccagcag gtacgccagg atgaagatgt cgaccacgtc cacgtcgaag agccccatga 58800
aggagaagag cggcgtggtg aggaagtaga tggcgccggg ccagaagcgc gccagccacg 58860
tggcgagcag cgaccacagg gagggcgcgc cgctgagccg cgtcttcacc tgtatgtagt 58920
actcggggta gaccacctgc tcggcgccgg agagcaccac gcgcgccaga gagagccgct 58980
tctccagcgt gaacacctcg gtgagcaggc cgctgcgcag ccctccctcc ttgatgatcg 59040
cgtcgtagag cttcttcatg ccgccgacgc tgatgatgta ggcgtctagc gagacgtcgt 59100
acccgccggg gtagaccatg agctcggggt cgccggtgcc ggggacgttg gtggccagcg 59160
cgccggtcat gtaggtctcc ttgagctgcg tcatgtacca gccgttcgcc ttcatcgcct 59220
cgatgagcgg cttcaccatc tcgggcttgc ggaaggtcat gtcgttgtcg accaccagga 59280
tgaagtcatc gtcggagtac ttggtgggga cagtgccggc cgatatgctc tcccagaggt 59340
tgaggtggtg cgctgcgcgg cgctgcatct ccttcggaca cgtggacttg cacatgtccg 59400
tgaagaagtg cgggtagtct ttggagtcca cgtctttcca ttccaccgcc ttgagcacgt 59460
ggtcgccctt ggggtgcggc gcgggaggag atggcttggg tgccggcgcg ggggccggtg 59520
cggggggctgg ggcaggagag ggagcaggcg cgggttgagg cttgggcggg tcgtcggcga 59580
ggcccaccag gtacggcagc gtggggaaca cctccttggt cccgcggcct tcggcaaccc 59640
cgattatgta ggccgtgatt tcgggtggat ccatttagtt attaaaatta atcatataca 59700
actcttttat ggcggctatg gattcggcta tccagtcctt gaccgagccc acgatgcccg 59760
ccaggaacag gaagaaggcg aactccaggt ccacgcggtt cagagagtcg ctgaagtaca 59820
cgaagacgtc gctgtccggg aagaagctgc gccggaacat gttgtacccg ttgaccttgt 59880
gcgcgacgtg ctccgcgctc agcagcgtct cgtcgaaggg gtacgggtcg ctgaagcgga 59940
acacgtacat ggccgggttt gcgtagtagt acttcatggt gtttgtgacg aagaggctcg 60000
ccagcgagat gatgattttt ttcttctcga tctcgatctt gatgtggtcc tcgaagcgct 60060
tcatgttgta ggcgttggtg tcgtgcacgc ggatgagcac gcgcgagtcc gacatgatgt 60120
cctggaactc cgcgcgcgcg tcggggctct cggcgggcgt ctccgcgggc cgcgccacct 60180
ccgcgcacac cgtcggccta gcgcgcggcg gcgtgcgcat gggccgcgcc cccacgcgct 60240
gcgaagcgaa aaactccacg gcgcgagcct cgcccgcgtc cgcgtacgac tccaccaggt 60300
agttgcggct gcgcgtggtg cggccgatgg tgttcagccg gtgcagctcc gcgaccagcc 60360
ggcggtagtg cgcctccagc tcctcgggca tgatggaggt gtacacctcg gtgagcagca 60420
tcacggtgtc gaagtcctcc ttgccgcaga cgcgcgtctt cacgaggaag tggtgcacag 60480
ccgtcgcgat agagagccgc agcgtggact cggtgacctc gacgctggcg tccttggtct 60540
tcttcgcgct ccgcgaggcc atgaacgaga cgaggaagtc cgcgctgctg ttgagcacga 60600
tgaccagcgc gacgatgaag ttgaggttca gcgtcttcgc ggactggaac agctcggtgg 60660
ccgacgcgtg cacgtcgagc aggttcgcgg agagccgcag gaagaacacg ccgcgcttga 60720
tctcggccgc gaagcgacgt tcgtactcct gccggcgcgc gttgatcgcg atgaggaagt 60780
tcaggatgag ccggttgatg ttgtacttca cggcccaggt ctgcgtcttc atgatggtgt 60840
cgaaggacat cacgatgttg aagatgaagc gctggctgtg cgagaagtag ctgtagggct 60900
cgctgaggaa gatggacttg ttggtcgcgg gcaccaccac gcccgcgcgc gcgccggacg 60960
cgtcggtgtt caggtccggg atgttcatgc cgcagatgcg gcagtaggcc atgccgtcct 61020
caaagtacac gaactcctcc acgaactcgt tgatcttggc gaagtagtcc acgtccacgc 61080
gcatcgcgac cgcgagccgg atctggtgct cgcagggcgg cgactcgaag cgcacccccct 61140
cgccccagcc cggcggctcg cgcacgacca gcgcggtgcg cgaggccggg cggaacttgg 61200
cgtcgcgcgc gttgagcagc gccgggaaga ggtcgcagag gtgccggctc gagaggaaca 61260
cgtacttgta cagcagccgg cgcgcgtccg cggccatggc gtccacgaag gcgcggcccc 61320
actccgcgac cgcgggctgc tcctccgcaa agttgttcgg gtagaccttg tccgtggccg 61380

```
cgaggaacac cttcttcacg tcgaggaagt cgcggatcac gatggggacg cgcgcgccgt   61440
cgagctcgta catgaacacg tagcgcaggt tgagcttgcg ccgcgagacc gggatgccga   61500
tgtgccgaca caggtacgcg aactcgaggt acttcttcga gaagcggatg cggtccaggt   61560
tcttggagac gtactgcagc atgttgcgca tgttgaaggg gatctcgcgc acggcgggct   61620
ccgcggcgtc gtcgaaggcg gtgcgcagat cgctggtgcg ctgtacgacc acggcttcgc   61680
cggtggcgtc gtcgtgcacc agcacgttaa cgcgccgctg ccggatgacc atgtcgaagg   61740
tgttgaagaa catctcgtac atgctgtgcc gagtgtcgtc cgcgatgcgc tcgcccaccg   61800
agaggctcgc ggtggcgtcg tcacgcacct gcttctcgaa cttgtacccg atgtaggaga   61860
atatcgagat cagcgtggcg tcgtcggcgt cggggttctg ctccatggtc gcgaagagca   61920
ggcggatgtc gtcctccgtg atcgcgtcca cgttgtacag gttgaccacg aagatggact   61980
tgttctcggc gatgaagtcc gtgtaggact tggtggccgt gttcgggtcg cgcatgtacg   62040
cgcggatctt cggcacgatg ctcgcgagga tggactccct ggaatccatt taaggacggc   62100
aagggcgcgc gagaccgtct caaaactgaa atcgtataaa ctcttaaaaa atcggtattg   62160
aaagtacgca ccaccaaata aagcgtcgag gtcgggcatg tcttcgtggc gactcaaaat   62220
gagcaagtgt tcaggttcca gcagcgtcca gactctcgag gatctgcgta atcgtcttcg   62280
ctccgaggcc ttgggcaacg attgccaaga gccccgcgac gacctcttcc ccagcggcga   62340
ggagtgtctg gacatcgacg ggccctgccc ttgcgatgag gcggagcagg agatcgacca   62400
ggagcagttg cccgtgcccg aaaccgtgcc cgaaccgccg gccaagactc ctaagcgccg   62460
accagtgaag aaggataagg cagataaggc agataaggac aagtcgacca gaggcgcaaa   62520
gaaaccgtgc ccttcggacg acaaggatga cgagctcaag agcaacgacg tcgacaacaa   62580
cgaagagtcc ggcgacacag acggcggcgc gagcgcccga agccccagcg acatcgacaa   62640
cgtggacgaa atggacgact ccgacctcat ggtggcgttc tccaccatcc tcgcagactt   62700
caaggacctt acccaacgag tgaaagctct ttcgtccgtg ctcacggacg tgcaggcggc   62760
cggcatacgc aggagcttct cgacgctcgg caaggctctg acggaggcgg cccacatcgc   62820
caacaccgga tctaagccag tcactgcgcc tcgcaagaag aaggccgccg cctgcaagaa   62880
gtaggcgcac taaatagcga ggctcggtat gcgggcgctg cacctgtcag acggcaaact   62940
tttttttgac aaggagctga cgcagccggt ccccgacgac aaccccgcgt acgctgtcct   63000
tgcgaagatc cggatcccac cgcacctctc ggatgtggtc gtgtacgagc aggacctcga   63060
gtctgcgcag cagggcctca tcttcgtcgg gcgcgacgcc aagggccgaa agcagtactt   63120
ctacgggcgc ggacacgtgg agcggcgcac ggccgtccgc aacgccgtgt tcgtgcgcgt   63180
gcaccgcgtc atgaacaaga taaacgcctt catcgacgac cacctcgcct ccggcagcga   63240
ggccgaggcg cagatggccg ccttcctgct catggagacg agcttcttca tccgcgtcgg   63300
caagacgcgc tacgagcgcg agagcggcac cgtgggcatg ctcacgctgc gcaacaagca   63360
cctcgccgag gccgagggcg gtgaggagat ccgcgtcgcc ttcgtgggca aggaccgagt   63420
cgcgcacgag tttgccgtgc gcgagggcga gcggctcttc gcggcgctgc gtcggctctg   63480
ggacccgggc gcgcccgaca ggctgctgtt cgaccggctg agcgagcgcc gcgtgtacac   63540
cttcatgcga cgcttcggca tccgcgtcaa ggacctgcgc acctacggcg tgaactacac   63600
cttcctgtac aacttctggt ccaacgtgcg ctcgctggag ccgcgtccct ccgtgaagtc   63660
gctcatctgc acctccgtgc ggcagaccgc cgagacggtg gggcacacgc cctcgatctc   63720
gcgcagcgcc tacatggcca ccgcggtgct cgagctcgtc agggacggcg cgttcctgga   63780
cagagtcgcc gccaccgaca cgctcgacga cttcgtggac atcgtcgtgg actatgtaaa   63840
taactctgag caggtaaatg gatgaggcgc tgcgcgtggc ggcgcgcgtc gtggacgggc   63900
tccggccgct ggacgtggcc gtgtgtctcg cgcagctgcg cggagccgcg cccgagcgcc   63960
gcttcccggc gctcgacgag tgctccggcg aggccttcct ggactttgag ttcgccggcg   64020
gggacgtggc gtcgcggtac ctctccgcgc acacgcgcga gctccgtgcg gcggagcggc   64080
gcgagcacat ggccgcgatc gcgcgctgcg tcaccgaggc cgacctggcg ctcgcagacc   64140
gcccccgggg caaggcgcgc gcggcgctgc gcgtgtgccg caaccgcgag aaagtcgcgc   64200
gcttggcgag gctgctgcgc gacgccgaga gcagcggcgc ggacttcgcc ttcatacgcg   64260
cggccgtggc gtagcaaaac gtaaaaacaa cacattccct aaatcgccat ggacgcgcca   64320
agtctcgact gcatgctcgc cgcactcgcg gcgaaggcgg cctcggtgga ccgaggcgct   64380
cccgaggacg aggtgcacca cgaagtggag ctcgtgctcg tggacccgcc gctgtccacc   64440
ctggccgcca cgctgcgcct ggcctcggag acggagtcct tcatcctctt cacggtgacc   64500
gcgctcgcca aggaggaggg caagctgcgc gcgcgcgtgc ccatgtcgcg cgtcgtcggc   64560
ctggacgtga agaacgtgca gctggtcaac gccatcgaca gcatcgtctg ggagcgcaag   64620
gcgctcgtgg aggagaccgc gctgcaggaa ggctgtctgc tgcgccactc caccgagcgg   64680
cggcacctct tcgtggacta caagaagtac ctctcggcca tccgcgtgga gctggtaaac   64740
cgcgtgcgcg tgcgctccaa agaagtcgtc gcggacttca agttcaagta ctttctgggg   64800
tccggcgcgc aggccaagag ctcgctgctg cacgcactca accaccccaa ggtgcggccc   64860
tcgcccacgc tggagttcga ggtcgtcccc gcgggcgagg ccgtggacga ggccgccgtg   64920
ctcgcggagc tgcgcgccgt ggcgaaggcg ctcttcatgg cgcccaccga cgccgtcttc   64980
ctggcgccgc cggccgagat gccggtgcgc acgctcatgc tgcagaagca ggagatcccc   65040
gcgctagacc tcgacggcct tttcgcggtc tccaagacgg acggcgtctc cgcgagcgtg   65100
tgcgtggacg aggacggcgt cttctgcgcg ttctcgcacc tcgcgtacac catccggtac   65160
```

ccgctcgcgc gcgaagtgca gggccggtac cggctctggt gcgaggccgt gcggcccgtg 65220
ggcgagcgcg tgtggtccat gttcgtgctg gtcgtggagg agcctgcggg cgatgaccgc 65280
gtcgcggccg tggccggcgc cgtggaggcg ctgcgcggcg tgtgtgcacg cgtcgagttc 65340
aaacctaagc gcgtggacgg gcccttctcg gcgacctccg agctggtgga gcacatcaag 65400
agcgcgctgc agacggagcc agagggcgtg gtgctcttct acgcgcgcgg agagaagtcc 65460
aagcgcgacc tcaaggtcaa gcgcgacaac acggtggacc agaccacgaa cgtgatgttc 65520
cggtacatgt ccagcgagcc catcgtcttc ggcgagggct ccaccttcct ggagttcaag 65580
cggtacagca acgaccgcgg gttccccaag gagtacggcg cggggcgcat cttcctgcgc 65640
gaggacgtgg tctaccacaa caacatctac tgcatcgagt tcacgaagac gcacctggag 65700
gtgggcctcc gcagcgtggt cgtgcccgtg aagttcatcg gcgagttctc gcaggagggg 65760
tacctgctgc ggccgcgcct ggccaaaacg gagtgctact tccgcaaccc ctcattctac 65820
gggaaccagc actcggtggt gctcgagcac actcgcgacc agctgctctc ggtgggggac 65880
gtgttcgacg agagccgcat ggccgccgtc gggcagacgc tggccaacga cgccttccgc 65940
ctgaaccegg acacgcccta cttcaccaac cgacgcacgc gcgggccgct gggcgtgctc 66000
tccaactacg tgaagacgct catgatatcg ctgtactgct cgaagacctt cctgaacaac 66060
gccgagcgac gcaaggtgct ggccgtggac ttcggcaacg gcgcggacct ggagaagtac 66120
ttcttcggcg agatcgcgtc catggtggcc acggacccgg acgcgcgcgc gatcgagcgc 66180
gccatggagc gctacaaccg cctcaacgcg gggctgaagt cgcgctacta caagtttaac 66240
tacatccagg agaccatccg atccgagacc tacgtggaga gcatccgcca ggtcatgtac 66300
ttcgggcgct tcaacatcgt ggactggcag atggccatcc actactcctt ccacccgcgg 66360
cacttcgcca cggtgatgcg caacctgcgc gagctcaccg cgcccggctg caaggtgctc 66420
atcaccacca tggacgggga cttcctgtcg acgctctccg agaagaccag cttcgtgatc 66480
aaccgcaacc tgcaggagag cgaaaacttc atgtcgatcg agcgcgtggc cgatgaccag 66540
gtcatggtct acgcgccctc gaccatggcg cagcccatga cggagtacat cgtgcgccgc 66600
gcggacatcg tcaagctctt cgcggacaac ggcttcgacc tcgtggacca cgcgaacttc 66660
gagaccgtga tccggcgcag ccgccgcttc gtcgagggcg tctcgcggct ggagacgcgg 66720
ccctccacca agaacttctt cgagctcaac cgcaacgcgc tcacggagat ggacagcacc 66780
gacgtggccg cgctgctaaa gatctacgtg ctgtacgtct tcagcaagcg gtaggcagaa 66840
ccagggcgtc gattccgcgc ccgcgccggc gcggaaggcg ttgaacagct ccgccagcca 66900
ggctgcggtc tcgcgcgcgt cgatcgggcc gccgtcgtcc ggcggcggct cgcgcgccgc 66960
gcgcaacacc agcgtctccg cggcggcag aggctccaga gcctcgaaga ccgcgcggct 67020
cgggaacagc gcgcgcatca tgcgcgcgcg gtggccgaac accgccttga ccgcgcgcag 67080
tgccgagcgg ttgtccagcc gcagcgctcg gtcaaaacga tgcacgcgcg cgggcgcgcc 67140
gcggtggtcg cgctccacga gcacgtgccg ccacgccagc gccgcgccga cgcggtccag 67200
gctgggcgcg agcgccacca ggcttttcag cgcatgtaaa tctccgcgca tggccgacgg 67260
ctccatttac tactgcggag gaacgcacgt ggtcgcggcc gcgccgggcg ccgcgcttgt 67320
ggtgctggac gcgcccggtg cggcggcggc ggccgcgccc gcggggcagc gcgtcttctt 67380
cgcccagtac ggcctcgaga agcgggccgg cggcccgatc acggcgcggc tgcgccgctc 67440
cgggttccgc ggcgccgcga acgcctgggc ctccgtggcg gacttcgagg ccggcggccg 67500
tccctccgcg tggacgctgc gcgcggagga ggcttcgcgc gtgccgctgc cgacggacgc 67560
ggcgctggtc ctggcctggg gcgcgcgcga ggagccgctg cgggcgtgcg tgctggcgcg 67620
cgcggcagac gcggaggcgc cggtgggcgc cgcgctcaaa gaagccgcct tcgacgcgcg 67680
ggcgccggcg gccgcgctgt tcgcggcgct gggcgcgccc gcgctcgcgc ccccgctgcg 67740
ggcgcggcta gtggcgccgc cgggcgcgcc gccgcggacg cggctctgcg agaacccggc 67800
catgctgcgc gcgttcgcgg tgggctggtt cggcgcgcag ctgggcgagg cctccgaaaa 67860
tgaaaaggta tttgccgcct ttgataaggc gaggtcgtgt ttggacgacc gctgatggcg 67920
acgcccgcga acgcgcccgc gctgctcgtc gcggcgctgc gacaccgccc gtaccgcgtg 67980
gagtaccacc cggactggga gccggtcatc gagacgctgg tggacgagta cgacgcggtc 68040
gcgccctggc tgctgcgcga cgcgacgagc cccgagcccg agcgcttctt cgcgcagctg 68100
gcgaagccgc tggcggacaa gcgagtgtgc gtgtgcggca tcgacccgta cccgcgcggc 68160
ggcaccggcg tgcccttcca gtccccggac ttcagcaaga agaccatccg cgcgatcgcg 68220
agctcggtcg cgcgcacgac cggcacgcag ggctacgcga actacgacct ggacgcggtt 68280
ccgggcgtgc tgccctggaa ctactacctc tcctgccgcg agggcgagac caagagccac 68340
gcgatgtact gggagcgcat ctcgcggctg ctgctgcagc acgtggccaa gcacgtgagc 68400
gtgctctact gcatggggcg cacggacttc cagaacgtgc gcgcgcgcct ggacgtgccg 68460
gtgacgctgg tggtggcttc ccaccccgcg gcgcgcgacg ggcagttcgc gcgcgagcgg 68520
gccttcgagg tcatcaacgc cttattggag ctcaacggga agtctcaagt ggactgggcg 68580
cgaggatttt ctttttatag tgaaaattaa tccgtggtcc taaatggcgg cgcccatatg 68640
cgataactct cacgtgttcc tcctcaagcg cctgggcgtg ccgtcttcct gccggcgctc 68700
ggaggacccg cgcttcgtgg agatcctgac tcccttcgag ctctcaaact acatcgagcg 68760
gcacccggga tgctgcctct tcgagacgct gcgcgacgag gaggactgct ccgtcgtgcg 68820
cgtcttcgcg gacgtggaca tggacagcgt gctcgaggag gaggacttcg tcgcggcgct 68880
ggaggacctc atcgtggagc tcgcggcctt cttcgaccgc ttcgcgagcg gctcctgcgg 68940

caccgtgccc ggcgaggtca agcgcgccat gctcgcgaac ttctcggtca cgcgatccac 69000
ggccgagcac aagaccagct tccacctgat cttcacggag acgtacacca cgctggacac 69060
gctggtggcg gcgaagcgcc cgctgctgga cctgtgccgg cgctcggaca acgtgctgct 69120
gcgcgcgctg gacacggccg tgtaccgccg cggcgcgacg ctgcgcgtgg tgggcacgcg 69180
caagacgccg gagtcgagcg cggtccaccg catgcagtcg cccgacgacg acatcaagga 69240
ctacctgttc acgttcgtgg agctctcgga cgcgagcgtg tacttcgagc tcgcggagcg 69300
cgagcagcac acgctgagca ccgtctgctg ggagacctcc tacatcccct tcggcgacgc 69360
gatgcggcgc gtgtgccagg cggtggtcaa cgacatcgtg aacctccgcg acatcaccga 69420
ggacaacttc ctcgacacgc cgctggtcat cgactacgcg acgcgctgcg cgctgtgcaa 69480
gaagcccaag cacaagcacg cgcaccacat caccatgggc aacggctgcc tgcgcctggt 69540
caagggcggg aacgcgcaca gctgcaaggt caagatcatc cagctcgagg gcaaccggct 69600
cttcacggcc gcgcagatca tcatcgcgtc cgaggtcgtg aagctcaccg agcgcaacga 69660
ctacatcgtg tggctgaaca actcctggcg cttcagcgcg gaggagtcgc tcatcaccaa 69720
gctcatcctg gacgtgcggc actcgctgcc cgcggactac gccaacgaca tgctgtgtcc 69780
gcgcaagcgc aaggtcgtgg agaccaacat ccgcgacatg ctcgtggaca tctccgagac 69840
ggacacgcag tacgacaagc tgcccttcac gaacggcgtg ctggacctgg ccacgggcga 69900
gttcctcacc ggcgaccgcg cgaaggcctg cgtgtgcacg gtctccaccg ggtacgcctt 69960
ctcgcgcgag gagttcgcgg ccgcggcgga ctcggaggcc atgcgccggc tggtcggcgt 70020
catcgacgac atccagccgg acacgcccga gaacgccgat aaccgcgcgc tgtacgagcg 70080
cgccatgtcc agcgcgctct gcggcgccac gaagacggtc atcgtcttct tctacggcga 70140
caccatgacc ggcaagtcca cgagcaagcg tctgctcatg tccgcgctcg gcggactctt 70200
catcgagacc gggcagaccg tgctcacgga cgtgctcgac aagggcccga acccccttcgt 70260
ggccaacatg cacctgcggc gcgcggtctt ctgcagcgag ctcccggact tcgcctgcaa 70320
caacgcgcgc aagctgcgct ccgacaactt caagaagctg accgagccct gcatcgtggg 70380
ccggccctgc ttctccaaca agatccacaa ccgcaaccac gccaccttca tcatcgacac 70440
caactaccgc ccggtcttcg accgcgtgga caacgcgctc atgcgccgcg tggcgctggt 70500
gcgcttccgc acgcacttct cctcgtcggc cactcgcgcg gccgccgcgc acaacgtcga 70560
gtacagcgcg gtcaaggaga tggacgagag cctggacacc aagatccagc gcaactactt 70620
ccgctacgcc ttcctgcgcc tgctcgtgca gtggttcggc aagtaccacg tcccgcaggt 70680
ctcgctggcg cccacgcccg acgcggtccc cgacttcgcc ttccaccgcc gcgtggccga 70740
gctggtggtg gccagcaacg acgcgcaccg ccgcgcgatg gagtcgctgt ccaagctggg 70800
gtacgtgctc gtgggcggca acgtggccat gcccgcggac gccttccggc agcggctggc 70860
cgcgcacttc aacgcgcgcg tgcacggcgg cgacatagac gccttcatgt tcaagcacaa 70920
gaaggtcgtc aacgtaacgg aggagtacgt ggagtacgta ttcatcgaag atgtcgagaa 70980
taaatagacg ggtatgaact cggacgtgat aaagctgttc gtcgggcacg acgagtccgt 71040
gcccggcatc ctgccgcacc agctcgcgac cgtggacttc ctgatacgcc gcgttctaga 71100
cgacaacgtc agcgtgcttc tcttccacat catgggctct gggaagaccg tcatcgcgct 71160
gctgttcgcg atggtggcct cgcgcaccaa gaaggtgtac atcctggtgc ccaacgtgaa 71220
cgtcatgaac atattcaact acagcatggt catggtcgct aacctgttca acgcgccctt 71280
cgtggccgag aacatattcg tgtactcgac gactagtttt tattcgctaa actgcaacga 71340
cggcgtcata aactacaacg gcctcggcaa gtacgagaac tcggtcttcg tggtcgacga 71400
ggcgcacaac atcttcggga acaacaccgg cgagctcatg atggtgatca agaacaagac 71460
gcgcgtgccc ttcctgctgc tctcggcctc gccgatcacg aacacgccgc tcacgctcag 71520
cagcatcatc agcctcatgt ccgagaagga cgtggacgtc ggcgacatcg tggtgcaggg 71580
caagaaggtg ttccagatcc tgctgaacga gcacggcgtg cgcgtgatcc gcgaggtgct 71640
caaggggcgc atctcctact acgagatgcc ggacacggac atgcccgagg tgctctacca 71700
cgggcgccgc ttcctggaca cgcgcgtggt ctactgccgc atgtcgcgcc ggcaggagga 71760
cgactacctc accgtgcgcc ggctctgcaa caacgagatg ttcgagaaga acatgaacaa 71820
cgtgtccatg gcggtgctgg gcccgctgaa cctggtgaac aacctggacg tgctcttcca 71880
ggcgcaggac aaggacctgt acccgaacct gcgcatcagc aacggcgtgc tctacgggaa 71940
cgagctcacc aagctggaca tcagctgcaa gttcaagttc ttcatctcga aggtgggcgc 72000
catgcgcggg aagcacttca tctacttctc caactcgacc tacggcagcc tggtcatccg 72060
caacgtgatg ctcagcaacg ggtactcgga gttcggcggc tcgcagagca acaatccgca 72120
caccacgccc gacgggcgcg ccaagacctt cgcgatcgtg accagcaaga tgaaggcctc 72180
gctggaggag ctgctcgagg tgtacaactc cgcggagaac aacgacggtg gcaagctcat 72240
gttcctcttc tcctcgaaca tcatgtccga gtcctacacg ctcaaggagg tgcggcacat 72300
ctggttcatg accatccccg acaccttctc gcagttcaac cagatcctgg gccgcgccgt 72360
gcgcaagttc tcctacgcgg acgtggccgc gcccgtgaac gtgtacctca tggcggcggt 72420
gtactcggac ttcgacgagg acatcgtctc gctggaggac tacagcgtgg aggacatcaa 72480
cgcgctgccc ttcgacgtga agaagctctt ctacctcaag ttcaaggcca aggaaaccaa 72540
ccgcgtgtac gccatcctgc aggagctctc ggacgcgtac tccgcgcgcc cgcacccgca 72600
gctcgtggac gtggtgctgg gggagatcgt gcgccagttc ttcgcacggc actgccgcgt 72660
gcccgccgag gacgccgcgc tcgtggccgc cgtcgaggcc gttctcggca cgcgcgaggc 72720

agcggccgag tacatccgcg cgatagtgga cggacacttc ttcgtgacca acaagacctt 72780
cgggaagtgc ctgctcttcc ggcacgagcg cgacatcgtg accgtgccct tcgagctcga 72840
gcacgacccc ttcgcgtggg cgatcaactt ccgcaaggag gtcagtgtgg tgaatatata 72900
acggcaaaca taaatagaaa gactgtcctt ttgcgcgatg tcgaccttcc ggcagacggt 72960
gtacctggcg gtgacgctgc agccgcacga gctcacgctc gacttccgcg gcaacgtcgc 73020
ggaggcggtc atgcgcgagt acctctacaa ggagaagggc gggctcatgg ccaccgacat 73080
cgaggtctgc ctcggaaacg agatgccgct ggggcgcatc gtgaacaacg cggttgtggt 73140
ctcggtgccc tgcaacgtga ccttcaagta ctaccgcgtc ggcgacaccg tgagcggcac 73200
gctcaacgtc gaggacgaga ccaacgtctt cgtggactgc ggagacctca tctgccagct 73260
cggcaagagc tcgggcggcg tgaccttcaa cgagtccaag tactgcctcg tgcgcaacgg 73320
agtcgtctac gagcacggca gccgggtctc ggctgtgctg cgcgaggcgc gctccggacg 73380
cgagtccgcg ttcgtgttct ccgcagtgct gctggacggc gtccccgccg aggagaagga 73440
cgagaagaag gacgagggcg agaaggccgc ggaggaggag acgcccgcga gccccgccgc 73500
caaaaactag cattattggg ccgcgcgaac cttcgataaa tgcgcacgta cacgtcgctg 73560
ctctcgaagc tgctcaagag caaccggcgg ctcgggagca cgcgcgtctt ccgcgacccg 73620
ctgcagcaca tcagcgcgac cgcctttgtg caccggcgca tcgaccggca ccggcgcgtc 73680
tccatctgcg ccgtgctcac caccaccgac gggctcgtgg tcgcgtgccg gcgccggtac 73740
tcctttttgt cctccgagct cgcggagacg cgctcgcccg cgcggcgcgt gctgctcgca 73800
accaagcacg cggacgctct cgcgcgcctc ggcgccgcgc gcccgcgcga cgacgtcatg 73860
tttccgggcg gcgccccgct gtccggggag tcgccgctgg cgtgcgtgct gcgcgaggtc 73920
gaggaggaga ccgggctgcg cggcgaccag gtcagcgtgg acgagcggct gttcgtgcac 73980
gccttcatcg acgacctggt ctcgggccgc gacttcgacg cgatcatctt cacgggcgca 74040
gtcgcgcttt cgagcgcgga ggtggcgaag cagttccggc ccaacgacga ggtcaagggg 74100
ctggtcttcc tgcaccccga ggacgcggag ggcgtgggcg tgatggcgcg gctggcggcg 74160
ttcgcgcgct gcgcggcgcg cctgcgctgc tgggcgcgg ccgtcacgcg atagaggcgg 74220
ggtccaccac gtacacgagg cgcccgccgc tcacgcgcac ggtgggcggg tcgcccagcg 74280
cggtcaggaa gttcccgtcg tcgtcgaaga ggcgcccgcc gcgctcgagg aagcccttgc 74340
gcaccgtgac cagcgccgtg gaggtggagt accacacgct ctgcccgtcc gcgagccgcg 74400
ccgcgcgcgc gggcccgcgc gcgtccgccg ggcgcgccac cagcgcggac cagccggagt 74460
cgtcctccag cggcgcgaag tccgtgaagg cctcgcgcac ccactccagc gagcagcgct 74520
tgagcacgcg gaagagctgc gtgaactgcc gggacttgtc gcggatgagg gccagcaggt 74580
cctcgtccac ggtggcggcg ccggagtcct ggcgcgcgac cacgaagtgc acgttcacgt 74640
agcggcggtc gggcggcgtc atctcgtggc tgttcaggcg caccgcgcgg cccacgatct 74700
ggcgcagcga ggcctcgttc caggtcatgt ccaggatgaa gatgtcgttg atggagagga 74760
agctgaggcc ctcggagccg ctcagcgaga acacacagac cttgatcttc tcgccgtcgg 74820
tgttgtcgca ggcgttgaag gcgtccacga gcttggcgcg cgtgtcgcgc gtgcgcgagg 74880
agaactccac gctggagacg ccgaaggcgc ggaagtagag cagcagcatc tcgatgccgg 74940
tcacgttgac gaagggctcg aagaccagac acttgcccgg cgaggccagg atgcgcaggc 75000
agacctcggt gtacttgcag ctgcgctcgc gcagctccgc gagcagcgag acgtccgcgg 75060
aggtcatgcg gtcgccgctg acgggcgcgc cgctgcggaa gagccgcatg gccgcctccg 75120
agaagacgcg gtccttgacg gcgcgcgcga agtccaggaa gagcgcggcc acggcctcgt 75180
cgtactcctg cttggagagc acggacttgt cgggcgcgtc ctcgaaggcg aaggtggccg 75240
cgatgcgccg gtacacgcgg aagaccgcgg cgccggactt gcgctccatg gcggccgcgc 75300
ggcggtaggc ctcggtctgc ttcgcggtca tgtccacgta catcatgcgc acgcgcttgc 75360
gcgcgaaggc ggcggagccg tcgacgtcgt cgaagatgga ggcctcgttg gtgactaagt 75420
acgagcacag gccgccgagc ttgtccacga ggtcctcggg gttcgcgagc gcgccgccgt 75480
tgaagagcgg cgtctgcccg accacgccgg ggcgcagcag gttcacggcc atggagaact 75540
ccttgacgct gttcaccacc ggcgtggccg tgaggcagag cagcttcccg cggcccatgg 75600
ggatgttctt cgcgaggtag ttgtacaccg tgcgcgcggg ccgctggcgc ccgtcctcct 75660
tggtcagcga catcgagatg aagttgtgga actcgtcgat gaccacgcag acgcggctgc 75720
tcgacgaggc ggtcttcatc agcgtgaaga agcggtggtg gaagcgcggg tcgtcgtagt 75780
tgatgaaggt gcacccgggc acggcctcgg gcgcgaagcg catcatcgtc gaggtccagg 75840
gctgctccac gagcgccttc ttcacgagca cgaccaccgt ccagtccgtg aagacgtcgc 75900
gcaggtgctt gagcacgtac accgcggtca cggtcttgcc cacgcccgtc tcgtggaaga 75960
gcagcagcga gtgcatgctg tccaggccca ggaacacgcg cgccacgaag agctggtagt 76020
ccttgaggcg cacggactcc tccacgccct gcatctcgga gggcatgtgc gcggtgcgcc 76080
gcagcgcgta gtcgatgtag gccgcgtgcg cgctggtcat ggcgacggtc ggcgctcctt 76140
ttacggggtc tgtcgtctat ctattgtcgg cgcgggtctg atttaggggc agtagttaca 76200
aaaacgtttc cgctgctcgg cgcggcgttt ggaggagcgg ttgcggccgc ggcggcgcag 76260
gcgcgcgcgg cgcgtcttcg tggtgcggtg gccgaaccag cgccggtgca tgaccgggtg 76320
cgagaccgcg gccgcgcgat ccgcgctcat gcaggttgcg taggtgcggc acatgctgcg 76380
cagcacgcgc cgcgtgcgcc gctccacggc gtcgagccgc ctcgcgacga tgggaaagag 76440
ccggcgccag ccgcgcacgg cgaagagcgg gcgctcgcag accgggcgcg cgagcgcgtg 76500

gtaggcgccc agcagccgcg ggtccagcga gcgcacgtag gtctccacga agccgttgcc 76560
gaagacgatg gcctgtgcgc atagcgggtt cgtcatctcc ctcttggagg cgatggcgtc 76620
gcccacgaag gcgcgcacgc cgcagtgccg cagcaccagg cgccgccgcg ggaagtgcag 76680
gtgcgggccg agcgccgcgc gggcggcggg gatgtgcagc cgcggagaaa aacgcgcgcg 76740
tccctccatg gcatctaagc gctccgtctg ttttcagtta tatcgccgcg ggcggctact 76800
gcagcagcag cttgagcttg cgctggctct cgttctcgat gctcttggac tcggaggtca 76860
tgctctcgta gagcagcgag tgcgtgacgt agagcgcctc gtacacgcgg ctggcgaagg 76920
ccacgaagcg gtccacgaac tcgctctcta cggggtcctt gagcacgcgg aagggcacgg 76980
ccagcgcgtc gcgccaggcg gcggccttgg tgcgctcgcg cacgtgcgtc acgaaggcgg 77040
cgatggccgc gcgccgcggc tcgctggcga ccatgacggc gctgtccttg agccagctgt 77100
cgctcacgca cttgaagagg cgcacggtgc cgaagaggct gcagtacacg cgcagcgcgt 77160
gcaccacgtc ggtgccgaag agcgtgggca gcttgagcac cacgaagcgc tcctgcgtga 77220
tctcgagcag cgggcgcatc accgcgaagg tgatcgcgtg gtagtcagcc acgtagaggt 77280
tgttctcggt gaggtggttg ttggagcgga tggcgccgcg ctccttgcgg tagagccggt 77340
tgcgcgcgct gaggtcgagc acgaccgcgt cggccctgcc gcgcgctctg gagcgcacgc 77400
tggtgatgcc gtgcgcctcg agcaccttct cgacgtcgcg ctcgtcgatc atgaggtcgt 77460
gcgtgtacag actcagcatc tccgtgggca tgcggttgat gtcgttcacg cgcgagcact 77520
gcaggaagta gttggtcccg taggccaggc tgggcaggtg cccgacgccg agctgcaggt 77580
ccagcgcggg cgtcgagtcg aaggtgggca gcgtcacgct gagcccctcg cggatgctgc 77640
ggcgcacggc ctccaccgcg tccatggccg atttattgga cgcacagtct gttttcattt 77700
cgcggctact gcgcagtcac cttctcggcc acgatccccg cgtcgtagct gagccggtac 77760
acctcgttgc acaccacgac catctgccgc ggcacgtaca tgagcgggtt gtgcgcctcc 77820
atgtgcgcgg tggtcacgcg caccgccagc ttgtccttgc ccctggagac gttggagttc 77880
agcgcggtgg gcgagaagaa ggtgctgggc gtaaagttga actgcagcgt gcgcacgccg 77940
ggcgtcttgc cgaggatctc gccgaagacg cgcgagactg cgctgttctc cgagtacagc 78000
acctcgttgc cgaagcgcac gtccatgcgc gcgatgacgt cgatcttgtt cttgaagtcc 78060
acgcccttga ggaaggggtc ggccacgaag aggtccttgg cgcgcgcctc cggcgagcgg 78120
ttgtcgccgt tgtacacgtt gcgctggcag gtccacacgc ccacgggcac ggaggcgtcg 78180
ccgatgttca cggagtggat ggcggtcgtg aagcggatgc gggaggtcgc gcggctgtag 78240
gcccccgtga tggcggagaa cttcttggac atgttgtaca cgacggagtt cttcctggtg 78300
gcgaacacta ggatgttcgt gtgcaggaac acgcgcatgc ccacgggcac gtcgtcgatg 78360
cgcacgaaga cgtcggtgtc ctggatggac acgacgcccg acgggggcac ctcgactatc 78420
tccgcggtct cggggaagcc ctcggggtag cagttcgaga cgatcaccat gtcctccagc 78480
aggcgctcca cgaaggccat cacgaagtcg ccctcggact gctggaagcc ggggtacgat 78540
atgaagcggt tgttggcgtc gctgagcacg ggcttcatgt acacggacag ggaggtgcac 78600
gcgtgcacgt ccgtgatcac cgcggtggtg tggttgatct gctccacgcg ccgccgcggc 78660
atctcgatga aggccgggcg cgggcacagg ttcttgacca tgtagccgat gaagctcagc 78720
tccatggagt aggggaactc cttggcgagc ttggcggcgt cgaaggtctc gtcgtagacc 78780
atgacgcagg cgacggggtt cagcgtgacc gtgaccgtga ccttgctgtc gctgagcttg 78840
agcgtgctga aggtcttgtc cgcgtcaaag ggcgtcttga tgtaggcgtg cacgcaggcg 78900
gcctccttga tgacgtcgtt gggcgagctc ccggtggaga ggtcgttgag ctcgcgcgag 78960
aagcccgaga gctccatcac gcgctcgttg tccaggcagg agtcgaacag ctcctcgccg 79020
gaggtctccc agatggtgtc cgcggcggag ttcacggcca cgtggcggat gagcttgtac 79080
gcgatgtagg gcacgtagca catcttgccc acgcccttta tctcgggcag gtccacgctc 79140
agcacgaagt tgttcatggc cgagatgtac ttgtcgcgga tctcgaaggt cacggtgacc 79200
gcgtcgctgg tggtgtccac cacgccctgc gtggtgatgt actgcggcat gtacaccgtg 79260
ggcgcgcggt ggtccgtggc gaacacgctg gcgcgccgca cggcgtcgtc gccgcccacc 79320
aggctcacca cggagttatt catttattcc ctgggaaaac cagttaaata aggctcttca 79380
gagccatgcg caccgtccgg ccgtcgggct ccaggtagca gcgcccgtag acgccctccg 79440
tggcgcgcgt ctcgttgatg agcgcgcgca cgcggtcggg gtccgcgtac atctccagcg 79500
gcagcagctc gatcttgggc tcctcgcgca gcgcgacgag gtgccggatg gagcccgcga 79560
aggagtcgcg gcacaaccgc gagcagaact cgcccacggc gccgccgtcg agcgtctcca 79620
cggccagcgc ggccgtgccc acgcgctgac ggcagaacca gcacgtgccg tccgcggcgc 79680
gcagcgccag ccgctccgcg gacaccgtgt tgaagtactt cggcagcacg tactcgatgc 79740
ggcacgccgc cggcggcgcg caggccgacg cccgcggcgc ggatatgtcc acccgcgaga 79800
gcgcgatgcg cttcatgggc ggcggtggct gctatttatg tcgcccgcgg cttttcaaag 79860
gtcgagcgag cacgccgcga agcgcgcggg cgagaacacg tactcgtggc cgaactccgg 79920
gatctgcgcg gcgcgcttgc gcgcgcgcat gtgcgcgagg aagttctccc aggtgagctg 79980
gttgctgttg ttcttcgcgt agttcttcac ggtctgcgga cgcaggttgc gcgtcacgcc 80040
cgtgacctcg aagatcttgt ccaggaagaa ggagtagttg atggttttgg tgggcgtgat 80100
ctcctggcag aagaagacca gctgcttgaa tatctcgatg acctcgttga tcttctcggt 80160
gctgaggtcc agcttctcgt ttttgacctg gttgatgatc tcgaagacca gcttgtagtc 80220
cttcttgttg atcatttcgc tgtccttgag gaagctggag acgtagttgg cgtccacgtc 80280

```
ctcgggccgg atctggtgcc ggtccatcat cgcgcgcagg tcgcggatga cctcctccga  80340
gcactgcttg gagagcagcc gccggagcac gttccgcagg tggatgagct tgttcgacac  80400
gtggaagttg gacctcttct gcacgcggat gcccatggga aacacggtct cgcagaacag  80460
gcagaactcg tagtccgcgt cggacacgag cccgttgcgg cggcagccgc cgcacatgcg  80520
caggttcatg cttgctccag ccccagcacg cgcaggatct cgcggtccag cactttagtg  80580
tccagcgtgc gggttctaca gaactggagg aagcccgcga gcgcgcgcgc gcgccctggc  80640
tgcgagagca gcagcatgcg cgcgttctcg ggtcctcgt  tgatgacgcg cgtgaggttc  80700
agcgagcacc gcgtgcagcg ccgcggcggg tcgagctcga ccgagtacgc cgcgaaccag  80760
acgttgtcgc ccatgtatta tttattaaca cagaacgtcg cacatgttgc gcgaggacat  80820
gtacgggtcg tactcctgcc cgtagatgag gatggtgcag taccgcgaga tcatgagcat  80880
ggcctcctcc atggtgagca ggtcgtcctc gaacatggcc ttgtgctgca tgtgctggct  80940
ctgcttggcg gccatcgcgg ccgcgccgtc gccgcgcagc cactcgttca gacactggcc  81000
gtcgccgccg gcgccgctct cctgcgcgaa ggtgttgcgc atggcgcgca tgagcctggc  81060
ctccctggcc gagcggctga gcacggtcat ggggtcgtag agccaggggc ctgcgtccgt  81120
gaacaggatg gtgcagtacc cgttggcgaa ggcgtccgcg ccgctgcagt tgtcgatgcc  81180
gtcgccgacc ctgtagcaca ccgcggagac cagccggtac atgatgccgt tgagcatcat  81240
gtcctgcgac acctcgatgg gaatgtcgct gatcacgggc cgcatgttcg tgaagcagtc  81300
gcccgtgctg gccatgcccc cgcgccggtt caccaggaac accagcacgc cgttcgtgat  81360
cacgggcgcg cggtcgcgct cgtagaggta gccgctcgcc gcgcacacgg cggacgccac  81420
gtccgtgcgc gagaccgcgc ccatgttctg ggcgggcatg tacagcactc gcccggcctc  81480
cgcggtgcac gagaagggct gctccccgcc cacgtggatg ggcgccgtcg atgtcgtgat  81540
catcttgctg gagtccacca ccaggtaggg caccgtgtgc atggccatgt cgcccatgcc  81600
cccgatcccc gttccgaacg acggccgcga cacgctcacg agcgtcggct tgaacgagac  81660
tatggagaag atggaggcca agatctgctc ctcgtcggtc atgatggagg cgcacgaggg  81720
gtggatgatc ttcattaggg cgttgtcgat ggactcgtcg ctctcgcagt agaagacgcc  81780
catgcggagg ttcaggatgc accgccgcag gttggtgtgc agcaccgcgc gctggatctc  81840
catggacacg gagtcgccga cgccgggcat cacgatgggc gtttcctcgg tgagcttgtt  81900
caccagcagc tggtagttgt tgggtcgcac gcggctgttg tggtggagct gcgccaggag  81960
cgagaggctg tccccgttga cgaacgcgga ctcgatggcc ggcagcttta cgccgaacag  82020
cgccatggcg atggggtgca cgaagcccac ggagtcggac gacttgaacg agaagagcag  82080
gtcgccccgag gagctcatgt ctttgaagtg cacggactgg aactgcgtgg cggagagcaa  82140
gttctggtag ctggacatgc tctgcaggtc gtcgatctcc ttcatctgct tgtttacgcg  82200
ggtcgagttc ctcccgtaga tgatcaccag cgggtgcgtc tggctcacgg atagccccga  82260
gtccgtcatg gcggcgcgca cgctgttcag cagatcgaac agctccgacc ggtctctgtt  82320
ctggatcccg acctttgcca tcacagacat gagctcctgg atggtcatgt tcttgtggtc  82380
tcggcgcgtg gaccgcaggt agtcggcgat catctcgccc tccttacgga tcttcatctg  82440
ccagtcgtgc atggaggtca tgcggtccac aggcatgagc acgctgtcgg aggacgactg  82500
cgcggcgctg ccggactggc gcgagccagg gcgcgcggac gacgggcgcg cggagctgcc  82560
gcggctggag gaggacctgg acctccgcga ggatcttcgc tgcgaagagc tgcgcacggg  82620
ccgctgggcg cgcgccccgg cggaaaccat gtcctcgcgg tttatgctga ggagcgagct  82680
gcagaccgcg cacgacagcg actgctttgg aatgtggatg tggtcgcact ccagggacat  82740
gcccgcattg tcgtagcccg ggaccaagtc gaactttgca ttaaaaaaat ctgatgcgca  82800
cgcgggcgat tccatttata ccggaagttt ttatgaggtg ccggtattat ccacgcgatc  82860
tcgcagtgtg ctgggagact ctagcgtagc cacggacccc gtgagcagac gacgcaagtc  82920
gttgatggcc gactgcgtga cggacttcgc cgtctcgatg tcgcgcgtaa ggctgagcga  82980
ctcggcgttg aggtcgcgca cgctgtccgc gatgtcggcc agctcctttt tgatgaaatc  83040
cttatcatta tcggcgttga tgactttgtc cggcactcta gactctagaa ccggtgacgc  83100
ggcgggcgcc ttgatcgtcg ggcagctgga cgccgggtac tggggcggcg gcaatgattg  83160
ctgtaggaag atgggcttag cggcaggcgc cggttttgtc ggcaagggcg gcgccggcgg  83220
gcactgtcgt gtagacggcg ggcacgccgg cgcggggcac gccgccggtg gcggacatgt  83280
cggcgcagtt gcgggtggac acgcgggcgc gggcgccggc gcgggacacg tcgcggcagg  83340
agccgggcac gcgggagccg gagctggagc cgggcacggc gcggcaggag ccgggcacgt  83400
cgcggcaggc gcagggcacg cgggagccgg agccgggcac gccggcgcag gaggacatgc  83460
cggcgcagcg gcaggacaga ccaccgctgt cgcggagcac gcggcaaccg gcgtggagca  83520
cgcggcgggc attacgttca gaggcgtcga ctggaccttg gcaccgcggg gcagtagcga  83580
tggcgctagg ggcgactgtc cggtggttgg acgctgcatg caggcagtcg cagatttcag  83640
acgggactgg taatacctgc ccgcttcctt caccgtgtac ttgtcgacgg agtctacctc  83700
ctcatctgga ggacatggct gctccggtgc gggaacgact gtttgaggac acttggtgaa  83760
cagactggag ctggtctccg atagcaccag tttagacttg gccaagtcgg aggcaaacct  83820
tcttctgaga tccatttaag ccttcaaaat tgaacgtgta cgccgaccgc taaatggaag  83880
aatcggtggc cgtcgagtac gcggacgagg acgaagatga gattgaggag tacgaggagg  83940
aggacgatga cgaggaggaa gagtctgccg agggcgccgc cgcctcctcg gtcagcgacg  84000
tagcgctctc tgccgccgag aagctggtgg cctcggaggt cccggacgac gcggctgccg  84060
```

```
cggacaccaa cgtgcgtcaa cgcgtcaccg cgcgcgtgga ggagcttaag gcgcgctaca 84120
cacggcggat gagtctattt gagctcaccg gaattgtagc agagagtttc aatcttctgt 84180
gtcgcgggcg gctgccgctc gtggcggacg ccgcagaccc ggcgctcgac aacgagctaa 84240
aagtggtggt tcgggagctc gaggagggcg tctgccccat cgtcatcgag aaaaacggcg 84300
agttcctctc gccgggcgac ttcgaccccg agtgcctgcg ctaccacctg acgtacatga 84360
ccgacctctg gaagtcccag gggcgcatgt agccgcggct acgccgactc ggcggcctcc 84420
gcgatttttt cttttatcat gtccagcagc tcgcgcacca cgatggggcg gccgcagtac 84480
gtgatgccgt tttcggatat cacgctctgt gcgatgtcca ccagcgagcc ctcgcgctcc 84540
cagtactcgc gcgcgagcac ctccttgtac aacgcgcggt ggttggccac gtaccgcacc 84600
agcgtctgga tgttcttcac gcccacgctc ttgaggtcct gcggcgagaa cttctcgcgc 84660
agcgacacga agacgtcccg gacgagcttg ccgatctcca cgttggtctt gaactcgttg 84720
tacagcacca cgtagagctt gcacacgacc gtagcgaact tcgcgggctt gagatccttg 84780
ttctggaaga ccagcatgct gctcatcacc ttcttcatga aggccaggta cttcgcgcgg 84840
tcgccctcga cgctcacgct cccgacctcg aggtcggaca cgcagcggat tccgtgctcc 84900
gcgctctccg cggagacgcg cagcagctcc tggtactcct tgagcttctg cttgtccgtc 84960
atcagcgagt tgtcgaatac cgccaccagc ttgagcacgt agttctcgtc cgagaagacc 85020
ttgttcagac acttcaccag gaagctgtag tggctctgca ggatcttcat gaccgcgttg 85080
gcgccgctgg ctccgcggac gtgcgatatc atctccatga tcttcttaga gtcgtcgatg 85140
atctcctcgg tgtcgttgcg catgttgcgg tacattgcgt tcagcgagac cagcgtctgc 85200
gcggccagga gcacgtcgcg gaacacgcgc gcgaactcgc gcttctcctc cgcgtcggtg 85260
atgctgttgt acacggactt cgccaccgcg ttcgacttca ggaaccagaa ggagagcgcc 85320
tggtagttga agtgcttcat cagcgccagc acgtccgcct cgctcatttc cggcgcaatg 85380
gggcacaccg agctctcgag cacgggcacc atgctgacga gcgtgtccac gtccgtgtcg 85440
aagtccaggc agtccacgca gagcccggtg ccgcggctca ggtgatcgcg gctgatgtcg 85500
tagaagcgct cgtagcaggt gcggaggcgg tccatgtcgg ctgcgtttta gagagacaca 85560
cactcttgaa ttatggctgc gggtagaact cctgcagcag cgccggcgca cgcgcggagt 85620
ccggctccac tcccagcttc agcgcgcagt tcacggacca ggtcttcatg aagcggtcgg 85680
gcgcgtccgt gaccacgtgc cggaagagct tcgcgaagtg gcggctcacg gcgttgggca 85740
cggtcgcgtt gcgcacgaag gccgtgaagc gcgaggtcag cttcggcgcg aagcgcttgc 85800
cgtccacgaa gaagcccgag gtggtgagcg agagcccgtt ctcctcgcgc accacgcgtc 85860
gcgcggcctt gtgcggaaac atgctcgcga ggcgcccgct cgcgtcctgg tctaggtgga 85920
tggcgtccgt ggccgcgtcc ttgcggatgc gcaccacgtc gtgcacgatc tcctggatga 85980
ggatgcgcgt ggccgcggtc tccgccagcc gcatggggaa gtagaccatg tccccggaga 86040
tgagcacgtt cccgctagcg tttacgtagc tcactatctc ggacacggtg cgcagacgca 86100
cgatcgcgcc ttcgcagcag tgcaccacgt agtacccagc ggtggcgcgc aggcgcttgt 86160
tgtccgcctc gaagtccgcc tccaacccct cgttgaagta cttgtcgaat atgatgggca 86220
ggaaggatag ttttgactcg gtgaccacct ttccgaagtt gaggatgtac gggttcagcg 86280
cgctgcggtc gacctcttcg tcgtacacgc aggacttgaa ggtgtcggtg tgcgcctggc 86340
tgcgcaggaa gcagcacgga atgcagatgc gctgcaggcg gtggaagatg gagaggaagc 86400
ccacgctgtt gtagcgcccg tcggggtcca tgcacgaaaa catgacgccg tttccgttta 86460
cgaagacctc gcgcgtctcg gacttgaaga agttgttgct gaccttggcc atgttcgcgt 86520
ccagcgactg cacgatcacg ggcttgcggt tcttggtctt ggtgttctgg cagatgcgcg 86580
accagtacac ggtctccacc ttggtgaagt ccgaggactg cttcacgttg ttgaacatca 86640
cgctgatggc cacgatcaag aacgtgaagt acttctcgat gttcgggatg tagttcttta 86700
ccttcacgga cacgtgcgac ttcgcgagga tgatcgagat gcgcttgtcc gtggagagca 86760
ggatgttgtt ggtcgccgtc tccacgaaga tgaagctcgt ttccatgtcc agcttcatct 86820
tagacgtgat ggtcgtgttc agcgacacct tgtaggtgat gtcgcccttg acgcggtcca 86880
tctttacgtc catgctctcg atgagcttcg tgaacagact cacgtcgttc accgtgagcg 86940
tcttcccgtc gctcgagatg accaggtcgc cttccgggcc ccacaccgag aggttcagcg 87000
gctcgtccac cagcaggaag cgagtccccg tcatcgacac gaagaagtcg tccgtcttcg 87060
agagcaggat gtcgaagtcg cccacctccg ctcgcttgtc cggcgactgc tgcgcgatcg 87120
cgcggagccc ggactcgcgc aggttcgtgc ggaagatgtt gttgaacttg gtctccacgt 87180
tcatgtttag gtcgaggttc gcgaactcgc ggatgagccg ctcctcgaac ttgaggatgg 87240
agtcgttggg ctcctcgaag gagccgaact ccggcgcgga ggtgtccgcc gcgcgcgcca 87300
cccagaccac caggaagttg cacgcgtccg cgtacgcgtt gtagaggatg ccgtccgtgc 87360
ggatgagcgt tttcttttgc gtgggcgaga acgggttgaa gatggtgttg tccacgtagc 87420
tgtactccag gttgttcttg tgcgagtaca cgatgatctc gtcctgcagg cccagcaggc 87480
tccccaagta ccccttgagc tgccgcacgc gcatggtcag caagatgtgt ctgcgcacgt 87540
gctcggggtc cttctggatg tactgcttcg cgaagaagta gatcggcgag gcctcgtcca 87600
cggagtcgta cagcgatagg tacagcacgc gctcgatctc ctggtggcgc cccaccagca 87660
ccaccagctg cggcgcgacg gtgtagagca tgttcgcgcg ggcgtattta tagccggcgt 87720
taaactgaaa taaaatacgc gggtcgcgag gcagcgccat gttccagccg gtgcccgaca 87780
tggccgccga ggccgacatc gacctcggcg acgtcagcgt ggacgcgacg cgcgcgggcg 87840
```

cgcgcgagaa gaccgtcttc ttcgcgcgca acaagcgcat gtacccgcac cgcagcaagg 87900
acgaggagcg caagctgtcg ctgggcttct tcttgcagcg gctggacttc ctcacgtcgc 87960
gcgaggtcaa cctgcagttc cggtcgctgg acgcgctgcg caccgagaac gtcatgaaga 88020
agaacaacgt gctcgtggcg ccgtacatcc tcatcgcgac gctcgcgggg cgcggtttcc 88080
gcatgacgga gaccatggtc gagctctact tccccgagct gtaccgcgag accagcaagc 88140
gcttccgctt ctgcgcgcag ataaaggtca tccaggactt cctggggttc gcccacgaca 88200
gctaccacac ttacgacttc gagacgtact tcgcgttcgt ggcgctggtg ctgcgcggcg 88260
cggactctgc ggccgaggcc ttcgacgtcc gcgccgagag cgggcttgtg cgcagcctca 88320
ccgagatcac gtaccggctc tacgtgatgc agctgcgctc cgacgccgcg cagtggagcg 88380
tgagcaccgg cgccgtagtc tcgcaggcgg tgaacaccgt gctgtcggtc gtcggcgacc 88440
tcgctgcgcg cgcggaggcc gagcggctca cgcccgtgtg cgacctcgcg cgcgagaacc 88500
cgctctcgct cgaggacctg cgcaagtacg gcccgcggct gcgctcgctg ctcacgacca 88560
tggcgcgcgc gcgatccttc aagacgaacc ggcgggacaa ggacgcgctg tcccggttct 88620
gccgactgac ggcgggccct agcccgtctg cgtgccgcgc gtcgccatag gcgtcggcgc 88680
gcgctcgccg ccggaacact cggggtcgct gaacatgtag atgagcgcga cgcctagcag 88740
caggtacatg atcatgctga tcacggtttt gaacacgacg gcggcgaacg tgttggaccg 88800
cagtcggtgc tcgcagaagt gcatgaacag gtgccgcatg aggtcgatgg ccccgttggc 88860
cacctggaaa agggcgaggc cgccgatgga cttgatcacc gtcacgtagc acggccgcat 88920
tccgacgacg ctatttactc actgtcaaaa gaaacggcgc catccgaccg gaggttgagg 88980
ttgcgcttca tgttgttcca gtacatctca ccgatgctcg agtagtacgc cgtcagccgc 89040
gatatttttt ctcgcaccag ctcgtaggcc ttctgcatct ccgcaacgcc gatctccgcg 89100
tcgcccacgt accggccgct gcggcgcacg atcagcagca gcgccttcag gttctccagc 89160
gcgatcatgt ccatgtacag cgacttcgag agctgcacga agaggttgta ccgctccagg 89220
atgctgttct tcacctcgtc cgcgatcggg actccgaaga tgcgctccgt ggtgtacacg 89280
gactgcgtga gctgcttgaa gagcgcggag atgcagcagg tcgcgcgctt gacggcgtcg 89340
agctgcttct cggagcgcgc gctcgcgatg ctcagcgcgc tgttcacgac gttgctcgtg 89400
tcgcgcacgt agcgcgtctt cagcgcggcg ttgatggcat ccgcgatctc gttgctgctc 89460
acgctcgagt cgtccgagct gcccgagacc tcgtccagca gccccgagat cgtgatgtcg 89520
ggcgagccgc cgacggtcac cagacggtcg agcaggttgc agggcatgga catgaggatg 89580
ccctcgctcg agaggcagcc ctcgtcgatc atgctctgca ggttgcgctt gaaggccgtg 89640
ttttcgggca tgaacccgtc cacgctcatg agctcgtcga ccgtgctcgc ggaaaagatg 89700
cccctcacgt tgatgcggtc cagcatgccc atgtcctgcg agcacagcac caccgactgg 89760
tcggccgtct cggcggcgtc cttggcgccg ccgtagatga tgcgcggaaa ccgccagctc 89820
gccggaaaag agaaggaggg aaaccggcac tgcgcgctcg ggcctcggta gccctgcgcg 89880
tcgcgcacgt tggtggccgt gaccatgaac tgcagcaggt cgtgcgcgga cgccatgatc 89940
ttctccacct cctccttgct gcagcagacc ttgcccaggc tgcgcgcgat gttcgttttg 90000
ctcactgagg gcgagaccgt gacggcggtg tgtcggcggc tgccgagcgt gtacgcgctc 90060
acgctaacgc ggtacccat ggcgccgaag agcagcttca cgaagtccag gtagctctcc 90120
ttattgatgt agtgcggcgc gcccttgtct tccatcctca gcccggcgta ggccatgagc 90180
acttccttca tcgccgtctc ggggtccgag ttgcacacca gccgcagcat ctggaagaac 90240
tgcatgaagg cgcgctgcga gagcccgatg tggtggttgg gctgcgtcga ccggcgcggg 90300
aactccctgg gcgtcatggc gttgatgccc gagagcgtct ccatcacgag cgcgcccacg 90360
gtcttctggc ccatgacgcg cgggtaaaag cacacgcgga ggggctcctt gccggccgcg 90420
agcgcgtccg agagcagcga gcagtacgtg acgttgtcgt ggtcgaagag cgcgaaggtg 90480
tagcagacgg agctcatgaa gagcgagtcg gcggtgctca tggacttgaa ctccgtgtac 90540
gcgattccgt cccagaacag gctctttccg ggcgcgatca gcggggacgc gcggtcggcg 90600
cgcatcagca tggagagcag cgtcacgtag taacggatgt tggcggaaat gtctacgaac 90660
tgcatgccgg gcgaggccac gcgcagggtc gcgcccgagg tagtgagcac ctccaggctg 90720
tccatgagcg tcacgctggg gtgcagctgc gcaaggcgcg ccagctggct ctggtagaag 90780
atggacacgg cgaggctggc cacgctgccg cgtgccatgc gcaggttttg cccgttgaag 90840
gtgagctggc gcagcgagaa cacggagtcg aagtactgga agaaggtgag caggtacttg 90900
agcggcatgg tcgtcagctc ggtatccacc tgcggcgtct gtgcgagcac gattccgttc 90960
ttggccgcgg cgtcggggat gtcgtacatg gcgtccattc tggcgcggga ggcgtcggtg 91020
agcagcgcgc gcacgttgag cagcatgagc aggtcccgcg ctagcatggt cccgtcgacc 91080
agccgggcgc gaaagccgat ctcggcgggg ccggcgatgt tggggtagat caggttcagc 91140
aggtacgtgt tgtcgaagct cagcgagggg aaggagatgg gagacttcgc cgggaggccg 91200
gtggggtagc gcacgtagcc gccgcagatg cgcgcgtgcg cctcaaagct ggtcacgcga 91260
gtcttcagca ggttgcgggt gaagggcggc acgtccttga aggactgcgt gcagatcacg 91320
gggttggcgg tgtcggtcag cttgaggttg gtgggcttaa gctccgcgaa gttggggccc 91380
agcagcacgg ggacgaggtg cgagttggcg gcgctgtcga gcaggaagtt gatgccgaac 91440
tgcttcacgg cgacctcggt ttcctcgtcg ctggcgagct tctccgcgtc ctcgaggaag 91500
agcgcgtcca gcgggtgcac gtacgtgcgg ttgacgtcgt agctgggctt gaagtccgaa 91560
cacagcgtgg ggagcacggt ggagacgagc tggaacatgt attccgcgcc ctccacatgg 91620

tgcaaggcca tgtgcacgtt tggggccgtc atttatttag tattaaatga cggccgtacc 91680
ggtaaccgat attcctggag actacgggcc gacgtccttt tcggaggaca actacccgct 91740
gaacaagcac tacgagctca ccaaaggcca gctctcgatc ctgcgcacgg tcaacgacaa 91800
gctgctcgcg cgcaccgtgc agcactcgga cggggagagc gatgagagcg aaagcgagga 91860
ggacgacatc tccagtccgc tgccgccgga cgaggaggag ccggactcgt gcgtggcccg 91920
ggtcatgccg cgggacgcgg acctggcggc gccaaaaaag gccgacggct acatcattgc 91980
cgccgagcag cagcgccagc agcgcataaa cattctggta tccgatcgag aggccgtcgt 92040
ggagcgggag ccggttcaga cgtcgttcgc gcgcgtctcg gctatcccga tccacgggga 92100
cggcgcgcgc cgcaccaccg cctccttctc cgcgaccacg ccgtcgctgg gcgccgtgtt 92160
cgacgacgcc aagcgcgtgc ggctgctgga ggaggaggtc aaggagctcc gcagaaagtg 92220
cgcgacctct caggataacg gaaacctgga gaacttcacc aaggtgctgt tcggcaaggc 92280
gccgcgcgcg agcgagctga acaagcgcgt ggtcatcgtg aactacgcca cgctgaacaa 92340
cgtgacgctg tccatggagg acctcgagaa gtgctccgac gaggaagtgg accgcatgta 92400
ctcggtcatc cggcgctaca acgagacgcg gaagaagaag atcctggtca cgaacgtggt 92460
catcatcggg atcaccgtgc tcgagcacgt gctggtgaag cttggcttct cggaggtgcg 92520
cgggctcagc gccgacctct cgtcggagct catcgacgtg gagatcggcg aggactgcga 92580
gcacatcgcg gagcgcctgg ggttcgggaa cagcccggtg ctaaacgtgg cgctcttcgt 92640
ggtaaagctg ttcgtgcgga agctgaacct gatctgatca acacatgccg ccgtcgaggt 92700
ccatggcgtt catgaggttg gaggcgcggc ggcgcgcgcc ggtggaagcg gtggaggcgc 92760
tcgaggtcgt ggagcaggga gtgttgctgg aggaggcgcg gcggcgggag ctagaagcgg 92820
aactcgaggt tccgctggtg gtgctgcggc gactcgtgcc gctcgtgccg ctcctgctag 92880
tgccagtgcc agtgccgctg cggcgtgaag taccggtgcc ggacctgccg ctggagcttt 92940
tcttgcggcc gccgttaacg ctgtcgatgc cgagcaggtc ctcgcacacc tcgccgacgg 93000
ttccctgcac gtccaacttg ccgttcttga caaccccgta cacgatcttg ccgcagttgg 93060
acacagcctg gatggtggtc tcgtcgctgt caaaggcgtt cattccgccg cacccgccgt 93120
cgttgtttct tcgagaaggc gcgccgctgc ggcgactcct ggtgctgccg ctggaccgag 93180
ttccggagga cctggagccc gtggaccggc tgccggtcga cctggtgccg gtagtgcgct 93240
ttctggacga agaggaggag gcgcttccgc ggcgggtgga cgaactagcc tccagcgcac 93300
cggcgccgcc cacacaatcc acgtcggcgg cggcgcctcc gcgaatgacc tgctcgttgt 93360
tgagctgcgt caggagagat cgcagctgcg gcgcgatctt ctgcaaggtg ctcacgtagt 93420
cgtcgtagct gctctgcggg cgctgcgcca tttttttcgga cgccatttat tacgcggaat 93480
atctacgacg acgcagcact gaatcggttt ctcgcgacgg gagattccgc ggtcggcgcc 93540
ggtgcggggt tgtcaccggg cgacgaggta accagcgcgt ggaaggcgcg cacctggtcg 93600
tccgtcatct tgtcctcgaa cgaggacgcg cccgggggga gcaggtcctt gttgcgcgga 93660
acggcgggcg ccgagacgca cgaccggcgg tacatcatga tgacgatgta gcacacgatc 93720
gagatgacga tcacggtcag cagcgcgtcg aggagcccca tttattacct gtatatgccc 93780
gcgtttaccg ggcggtgagc tcaatgtcgg tgttgtttag ccgggcgtac gggacgctgc 93840
cggagcactt cctgtacatg ctgaacacga acagcccgag cagcagcacg gcgcccacta 93900
tgaagcaggt tacgcacagc gcgcgccaca cgtagtcggt gacgttgttg gtgttcttgc 93960
tgaaatccac gaaggcgaag acgcaggcgg ccgtcagcag cagcacgccg catatcagca 94020
ctccggagta gtaagagctc aaggtctcga atatgtccat ttatctgagg agaaatttaa 94080
attactgaat ggacgaagtg gaatagaaac cacgagaaca cgacggactg cagcacgaag 94140
atggtgctca gcttcgtctt catgggcatg cagaagttcg cggccagcgc catacagaag 94200
atgaacacga gcaccgccgg gtcgtagtcg gacaccattt acactacgct aaaaggcata 94260
tctcggcgcg cgacgtccac gagcaccagc acgcggacgc ccgcgggcgc gccggcggcg 94320
accgcggcga gctgcccggc cgtggggttc accagcagca gtgcgcgcgc ggttcgcggg 94380
acggggtctt cgtaggccat ggtcggcgtg gacccgggac gcagcggccg cccctgtctg 94440
tcgaagaggc cctcgggaaa cgaggtgccc ggaacggcca cgacgacggt gtcgctatct 94500
agaaacattt atggtcttgg tttccacgga tcgcctcgag tagaccgcca cgaagtagaa 94560
gatgacgccc gccgcgagcg ccgccaccag gaagggcggc acggcgggca ggttcgcgga 94620
cgcgttgtcg cgcacgccgg ggtccgggtc tgcgtagccc gcgcccacgg ccttgccgca 94680
gtcggcgatc atgtgcgcgc gcgagttctg catgaccagg ctgtccacgt cgatgcggca 94740
gcccacgtag cggcaccgcg agcgctgctc gtcctggctg aagaagagcc acttgcggtc 94800
gcgcgactgg tccgtgcact cgtgcgcgcg acagacgcgc gggcccaggt acttcccgag 94860
cgtggtgccc gcgacacacg cgcactccgg cgcggcgcgg tgcgcgtcgc agtagcgccg 94920
cagcgcggag tcgccgaagg cgaaggaggc gggccgcgcc acgcgcacga actccgagca 94980
gaagcgcgcg tccatgtgct tggcgcagag cgccgcgtag gtgtccagcg ccgcgtagcg 95040
gcccgtgcgc agccaggcca tgcactcggg cgcgtcaggc tccaccgcgc agcggctggc 95100
cataacgccg tcgcagtgcg cggtcttgta cccgttcgcg aacacggacg ggcacccggg 95160
ctccggattt gtgcagcagc gcgccatggc ggcgtccgtg ggcggcgccg aggcgccgat 95220
ctcgaacgcg cacatggtgc cctggcgcag gtacggcttc gcgatctcgg gaacgtagtc 95280
tgcgcgcagc agcgagcccg ggcggaagaa gagcgagtcg cagggcgggc ctcgcacgag 95340
ccgcgcgcgg ctcgccagct ctggcgagag gaagcgcccg cactgcccgg ggtccatggt 95400

cggcagcaga cagaaccgcg gccgtacggt cttcagcttc gggtcggaga aggtttctga 95460
ttcttccgcg aaggcgaagg tgtccgtggc gctcgtgtgt gtgacgcgca gcgcgtactc 95520
gcctggcgtc ggcgtgtcga gcacctccac cttggatacg gtgtccccca tttgaagacg 95580
ctatttacgc cgctgcctac tcggcgaaga ataggtcctc cgacttggcg cccgcgtaca 95640
ccgggcaggc gggcgcggcg gagcgagtgc gcacgatacc gcggccagtg aggcggaagg 95700
cgtagatggc gaacagcagg ccgagcacga tgtacatgaa agtggtggcg cccacagacc 95760
cggtcacgtg cgtcacgatg atggtgacga tggacatgat cgtgcacacg atggccatgc 95820
cggtgttgtt ggccgcgtag gggtgcatga tctgcatggc cgcacagtat ccgatgacca 95880
ggcacggcag cgggaggata agtgaggcaa tacctatcat tactagagcg agcacggggg 95940
tggacgtcaa ggccaataca aaaatcacaa tacctgttag tatgcggata tcctcgtact 96000
ggaggacgct gtaaggcgcg atattccctc cgggcactgg cctgggttta gccgggacta 96060
gggggagtc ggcagtgccg gggtccttgg ggagaaaggc attctgctcc tccgggctga 96120
agagctcggc gtcctgaacg ccgccggcgg tgaactcgtc gttatagtaa ctaaagtagc 96180
tttccattta tatgttgaaa aatgtttgga ggcgtacagg tggacgacaa actctacgcg 96240
tacctaaaaa aactcgccgg acgcgggcgg ccgctgtgtc tgttccgcga caacggcgag 96300
ttcgtcgaag tcttcgcggg gtccgcgttc cgctttgtgc tgcccgtggg cctcttcgcg 96360
gacctgcgcg tgcgcacgcg cggcgtggcc ttcccgaaac tgcgcgactc cgcgcgcatg 96420
cgcggcgtgc gggtggacgc gcacacgctg ccctcgctgt accccaacca gcgcatcgtg 96480
gtggacgagg tgctcgcggc ccgcgaccag ttgctggccg cgggccgcgc cgtgtacgtg 96540
acgctgcatc tggcttgcgg cttcgggaag acgctgaccg cgtgccacct catcgccacg 96600
cacggccgcc gcgcggtggt gtgcgtgccc aaccgcatgc tggtgccgca gtggcgcgcg 96660
gccgtggcgg agctgcgggt gcccttcgcg gtctcctgtg acggcgcggc ctcgctgctg 96720
cgctcgggcg agctcgaccg cgccatggtg gccatcgtgg tcagccggca cttcgccaac 96780
gacgacttct gccgcgcggt gagccggcag tttgacgtgc tcgtgctcga cgagtcgcac 96840
acatacaacc tcatgaacaa caccgcggtc tcgcgcttct tgaccaagta cccgccgccc 96900
atgtgcttct tcctgaccgc gacgccgcgc acggccaacc gcatctactg caaccgcgtg 96960
gtgaacgtgt ccgtggtcag ccgcctcacc aaggtagtgc gcgtggtgga cgccttcttc 97020
gagccgtaca ccacgcccaa gatccgcacg ctcgagcgca gcctcgaggg accccagaac 97080
aagtaccacg tcttcaccga gaagatcctc ggcgaggacg tgcaccgcaa caagctcatc 97140
gtggacaccg tggtcgcggc catggccgcg ggcgaggcgc ggcgcgtgct cgtgctcacc 97200
aagctgcgcg aacacatggt cgggctgcac gccgcgctct gcgagcgcct cggtgcggag 97260
acggtctttc tcggcgacgc caagaacagg aagacgcccg aggtcacgcg cgcactgcgc 97320
gacaaggacc gcttcgtgct cgtgtccacg gtcttcttct cgggcacggg cctggacctg 97380
cccaacctgg acgcgctcgc ggtggccgcg gccgtgctca accgcatggt catggagcag 97440
atgatcggac gcgtgtgtcg cgagtcgcac gccaacacgc gcacgctgtt cgtgttcccg 97500
gactcctccg tgcgcgcgat ccgcgacacc gtgtctgcgt ttgcgcagcg gctcgtggcg 97560
ctggcggtgg acgggctggg cttcgtccgc gagcgcgccg ccgccggcgc gaagaacgag 97620
ccggcgctgt acagcgccat cagcgggcga gatctcgcag cggtgtaagc gcggacccgc 97680
acgccgcgca cgagagcgtg ctggagcagg cgagtcccag cgacagtgtg gacagcctgt 97740
ccacgtcctt gatgctcacc agccgcgagt tgcacgacga gcacacgggg tcgctactat 97800
catcgaccac cgtggtgacg cggcggcgtc tgcgcttttt gtttccagcg gcgacatcga 97860
ccacgcctcc cttagagccc cccttcgccc ccgccttagc tttcaccgcg ctcatctttt 97920
atttatcata aaaacacgtc tgcgtacgcg ttcgcgcaca cgtcccgcaa atccgcgcgc 97980
gcgccgcagc gcgtgaagcg cgcggcgtcc gcctccgcga tccgcgcgca cggcagcggc 98040
gcgcccttct cgtccgccat cacgcgcgca gagatcccgg tggcccccag cgcgtacgac 98100
accaccacgt cgccgacgca gcggtacacg ttgccggagc cggcgaggcg gtcgaacgcg 98160
gcgccctcct ggcgcagctt gtcgaatatg cgaggaacga ggatgttaaa aatgagaacg 98220
aaatagcaga tcagcaaaaa cagcgagatc atgacctccg agagcgattt atataccttg 98280
aaagagctaa tacgacttcg ggactcgctg cacctcgcca ccggcgccgc cgtcgagcgc 98340
tacaacgcgc tcgtggagtg ggccgcgcgc acgtactgga cggtcgcggt gctgccctcc 98400
gcgccgtgcg cctccatcga gaagtactac tgcgtgtgca aacccgactg cgcgctcgag 98460
cccggcgagt actccgtgag ccggctgcac ttcggactca cgcacgcctg ggtgcgcggc 98520
gccgccttca actcggccag cggcgccgag gtcgagccgc cagaggaggt gcgtagggcc 98580
tgcgaggcgc tcgacgccgc cttcgcggac ctcaccttcg tgcgcttctc ggtcttcggc 98640
cgcgagtgga cggtcgacga cgccgtcaca gaccactcct cgcgcgacga ggtgctcgcc 98700
gcgtgcgccg cctccggcgt gcgcgtcgcg cgcacgctgc gtgtgcgcgt gcgggcggga 98760
gagtccttcg cgcgcgcaga cttcgacgcg gtgcacgccg cgctgcgcgc ggagggcgac 98820
gtcgctcgcg gcaccgcggt ctgtctcgcg ctgcgcgggt catcgcgccg ctggatagcg 98880
gaccgcgcgc ctcgatgctt catgcgcgtg cgccgcgtgg agctcgagcc cgtggacgcg 98940
cggcaccact gcccggtgct gatctccgcg cgcggcgacc gggtgctctg ccgcggcgtg 99000
gggcacctcg cggacgcgcg cgcgcgcgag ggcgtcttcg tggccgtgcg caggtacccg 99060
gagtgtctgg tgctctgcga cgaggcggcc gccggcgcgg cggagtgctc gcgcgaggag 99120
gcgctgcggc tgctggtgcg ccgcttcggg cgcgacttcg ccgtcagcga ggagggctac 99180

```
gtcttccgcg tgcaggacat ggacttgcgc ggcgtgtccg cgcgactggg gctcgcgccc  99240
tgcgcgagcc tggaggagct gcgccgagcg gtggagcgcg accgcgcgct gatgaggcgg  99300
ctgcgcgcgg agggcgccgt gcgcctcgcg tgcgagtgcg tgggataccc gcgccagaac  99360
gcggtggagc tcataaataa tatgcgcttt caaataacgg aagaaggcgc ggtggcgaac  99420
tttgagctgg cgaacgcgag ctgtctcggc aacccgaccg cggagtccat cttcgcgagc  99480
ttcgcgcagt tcgtgccggt cttcaacgtg ctctcggcga tcgcgcgcgc gcagccatga  99540
tcgtggcggc cttcgacctt ggcacgcgca accccgcgcg caccgtgctg gaggtgctcg  99600
acggcacggt gcgcgtggtg gacgtggcca agctggactg gagccgcgac tgggagaagc  99660
gcgtgcaccg cgacgtgacc gccttccccg cgaacgtggt gctcgtggag cgccagtgca  99720
agatgtcgcc tttttctaag ttcatatact tcatacgcgg gctgctctac gacgggcggc  99780
gccgcacgcg cgtgctcgcg gtgccgccgg ccatgaccgg cagcacctac cggcagcgca  99840
agcgccgctc ggtgcgcacc ttcctcgcgc tcgcggagag cttcggcatc ctggacgccg  99900
tgcccgcgcg gaagaagctc gacgacgtcg cggacagctt caacatggcc atcaattacg  99960
tgctccgaac aaactgaaat acgactggaa cgaataagtc atgctggcgc tgttcgagtt 100020
cctgcggtcc gtggaggact gctaccggcg caccatcttc aacttccaca tcgcgcacag 100080
cgcccgaggcg ggcgatgtct acggcgtgct gcgcgaccgc attttggcgg ccacgcgctt 100140
cgaggaggta gcgccgccag ggctcgcgga cgcgctggcc aaggtggtct actgcgacat 100200
aagcaccacc aagcacctgg tcaaccacgc ggccttcgcg gcgcgcgcgc ggccggcgcg 100260
gcgcggaggc agcctcgcgc agttcttcga cgtgcacgtg ggcgaggacg cggagagccg 100320
ccgcaccgcg gagatcttcg accgcgagcg ctcctcgctg gtctcgtacg tgaagaccac 100380
ggccaagcgc tgcaagatcg actacggcga gatcaagcgc accatccacg gcgggcggca 100440
gacctacttc tcggggcggc gctcggacga cttcctgagc accaccgtgc gcgcggaccc 100500
gagcaagccc tggatcaagt ccatctccaa gcagctgcgc gtggacatcc tgcaccacgc 100560
gatctgcacg cgcggcaaga gctccatcct gcagaccatc gagatcgtgc tcacgaaccg 100620
cacctgcgtg aagatattca aggactcgac catgcacata atcctctcca aggacgaccg 100680
cgagcgcggg ctcgcggacc tcgcggacaa gctcttcggg acctacgcga ccaccttccg 100740
cgtcatcgcg gccatcaccg gcaacgcctg cttcgcggcg gtggcggacg cggccgcgcg 100800
cgtggtcgcg ctcccggacg cggacgcgaa gctggcggcg gtgcgcgggc tcgcggagtg 100860
ctacggcgtg cgcaacttca aaatcggcat gttcaacctc accttcacgg gcgccatcga 100920
gcacacggtc ttcccctcgc tgatccccgc ggagagcaag atcaagttct tcaagggcaa 100980
gaagcttaac atcgtcgcgg tgcgctccac cgaggagggc cgcgagtgcg tggagcaggc 101040
gcaggcgctg ctcgcggcca tgcgcgagcg ctccgcgcgg ctcgcggccg cggacgtggc 101100
caccgcgagc gtggacttcc tcaaggagct gctggggcca tagtgaaata atactgattt 101160
cttaaatatg gagcaggcgc tcggatacaa gtttttgttg cccgacccca aggacgacgt 101220
ctactaccgc ccgctccact tccagtatga gtcatacgcc aacttcatca agcaccggct 101280
taaggacatc ctcacggtgc ggcgcacgct gctcaccttc aagaacggca ccgagtccat 101340
cgtgctcgag atcgacgacg tgaagatctc ggcgccggag ttctcgccca tcgtggccag 101400
catcaagggc cacagctacg aggcgctggt caccttcacg gtgaacatct accggcacgt 101460
gatgaccaag gacggcctca ccgttaccaa gatcaacagc tacgagggca ccgactcgca 101520
cctcgtcaag ctcccgctgc tcatcggcta cgggaacaag aacgcgctgg accccctccaa 101580
gttcgtggtc ccgaacgcca tcggcggcgt cttcatcaac aagcagtcca tcgagaagct 101640
cggcatcaac atgatcgaga agatcaccac ctggcccaag ttccgcgccg tgaaggccaa 101700
ctccttcacg ctctccttct cctcgatctc gcccgtgcac gtgatgcccg cgcggtaccg 101760
acactacaag atcctgctcg acgtgaacca gcccgacaac ttcgtgatct cctccgcgaa 101820
gaccttcatc accgtgaacg tgatcgtgat ggtgcagttc ctcgcggacg tcacgctcga 101880
gttcgtggcg cgcaacctct gcttcgacat gccgcccgag gccgcgcacc tggccaccgc 101940
gctcgtggag agcgcgaaga ccgtgcccgc gggcgcggac gtggccgagt acgtgaacgc 102000
gctcatcgcg gccgagcacg cgaagcagaa gtcgacgctg tccaaggagg agttccgcta 102060
cgagatgctc agcaacttcc tcccgcacat gcaggacagc gccaaccagc tcaagggcct 102120
gtacctgctc tcgctggtgc gcaagatggt cttctgcgtg ttcttcccga accggtaccc 102180
ggaccgcgac tcgctggtct gccaccgcgt gtacacctac gggcgctact tcgaggcgct 102240
ggccatggac gagctcgaga cctacatcgg gaacatccgc aacgacatcc tcgcgaacca 102300
caagaaccgc ggcacctgca ccgtgaacat ccacgtgctg accacgcccg gcttcaacca 102360
cgccttcgcg gcgctgctca gcggcaagtt ccgcaagtcc gacggcagct tccgcacgca 102420
cccgcactac tcctggatgc agagcatctc catcccgcgc agcgtgggct tctaccccga 102480
gcaggtcaag atctcgaaga tgttcaaggt gcgcatgtac caccccagcc agtacggctt 102540
cttctgcgcc tcggacgtgc ccgagcgcgg gccgcaggtc gggctcatct cgcagctctc 102600
cgtgctcgcc tccatctcga acatccgcac cgcggacttc gtcgagctca ccaagcgcgt 102660
ctgcgactac gtgcgctcct accccgcgcg cgacatcagc tacttcgaga ccgggttcgc 102720
ggtcaccgtc gagaacgcgc tcgtggcctc gctgaacccc gcgatcgtgg acgcgttcgt 102780
gctcgacctg cgccggcgca agcggctcgg cttcttcggg aaccgcgaga tcggcgtcgc 102840
gctcgtgcgc gaccgcatga acgaggtgcg catcaacttc ggcgcgggcc ggctcatccg 102900
cccgctgctc gtggtcgaga acggcgtgct cgtcatggac gcggaggcgg agcggctcga 102960
```

gcgcgacctc gccgcgatga ccttctcgga cgtgctgcgc gagttcccgc acgtgatcga 103020
gatcgtggac gtggagcagt tcagcttcag caacgtctgc gactccgtgc agcgcttccg 103080
cacgctgccg cccgaggagc gcgcgctctt cgacttctgc gacttcccgg ccgagttccg 103140
cgacgggtac gtggcctcct cgctcgtggg catcaaccac aactccgcgc cgcgcgccat 103200
cctcggctgc gcgcaggcca agcaggccat ctcctgcctg agcgcggacc tgcgcaacaa 103260
ggtcgacaac ggcatccacc tcatgttcgc ggagcggccc atcgtggtca gcaaggcgct 103320
ggagacctcc aagatcgcgg acaactgctt cgggcaccac gtcaccatcg cgctcatgtc 103380
cttccgcggc atgaaccagg aggacggcat catcctgaag cggcagttcg cggagcgcgg 103440
cgggctcgac atcctcacct gcaagaagta ccaggtcgag atcccgctcg agaacttcaa 103500
caaccgcgag cgcgtgcgct ccgcggcgta ctccaagatc gacgtcaacg gcgtggtgcg 103560
cctgaacgcc ttcctcgagc agggcgacgc catcgcgcgg aacgtgtcct cgcgcacgct 103620
cgacgacgac ttcgtcgccg acaaccagat cagcttcgac atcgcggagc ggtactcgga 103680
catctacgcc gcgcgcgtgg agcgcgtgca ggccgacctc accgacaagg tcaaggtgcg 103740
cgcgctgacc gtgcgcgagc gccgcgccat cctcggggac aagttcacca cgcgcaccag 103800
ccagaagggc acggtcgcgt acgtggccga cgagactgag ctgccctacg acgagaacgg 103860
gatcgcgccg gacgtgatca tcaactcgac ctccatcttc tcgcggaaga cgctctccat 103920
gctcatggag gtcatcctca ccacggccta cgggcacaag cccttcgccg aggacggctc 103980
caaccgcccg atctgcttcc ccagcaccaa cgagaccgac ttcgagacct acatcgagtt 104040
cgcgcggcgc tgctacgcgc tctcgcaccc cgaggccgcc gcggacgacc ccgagttcga 104100
gcaccgcgtc ttctgcgagc gcgtgctctt cgaccccgag accgacgagc ccttcgcggc 104160
gcgcgtcttc ttcgggccgc tgtactacct gcgtctgcgg cacctcacgc tggacaaggc 104220
cacggtgcgc tgccgcgggc gcaagaccaa gctcatccgg caggccaacg agggccgccg 104280
ccgcggcggc ggcatcaaga tcggcgagat ggagcgcgac tgcatgatct cgcacggcgc 104340
ggccttcacc gtcgccgaga tcctgcgcga ctccgaggag gacgcgcagg aggtgctcgt 104400
ctgcgagaac tgcggcgaca tcgcggcgcg gctcaacggc acgcacgtct gcatccgctg 104460
ctccaagatg agcctctcgc cggtgctcac gcgcatggac tccacgcacg tgagcaaggt 104520
cttcaccacg cagatgaacg cgcgcggcat aaagatccgc gtggagttcg agaagcagga 104580
cccctgcttc tacgggactc cgaaacggtt cagcctcgcg cccgacgagt cgctgttctc 104640
gccggaggac tgaacccgcc gtcgcgaccg cgtcgcgacg actagcttat cgttcgactg 104700
atgcgaaacg cgcggcggcg ccgcgactta gcttatctcg actgatgcga acgcgcgacc 104760
tctcgcgact ttctagcttc tcagactgat gctaccatat cgcggcgtgc tggccccacc 104820
accagggctt ctcgccgtgg ctgacgcggg gctggctgcg acgcgcgctg cagtagctgc 104880
gcgcgcccca gtcgccgcgc acgtgcgccg gggcaggct cccgtccagc gcgtgccgcg 104940
tcacctcggc gccgggccgg cggcacgtgt gcacgtccgt cttgttggag acgagcaccg 105000
cgtactgccg catggtctct atgtgatgct ccaagtgctt gcccgccatc cggttggact 105060
cgcagcacgt ttttgcttcg gctaaggttt tttctagagg ggatagtagc ttatccacgc 105120
gctcgggcag gacgcacgcg gagccgtcga accctacttt gaacggggtc accttgatgt 105180
tcccgtcgta gcggtcccac agcatcctga ggtaggttgt accgtcgggg tctgggtctg 105240
tccacactct aagcttttcg ctacagcggc cgtcgtacgt aagacggtct ctacgctcgt 105300
agtagtttct gcttatgttg ttggggtctc catgctcgta gtagtataaa tcgtacgcgc 105360
ctggcttttt taagtcgttt tcgtcgttgc tgacgtgtat cacgtcggga taataggata 105420
tcctaactgc actacaatct atagtatttg gtctagtaag ctgttcgaga tcaccttgtt 105480
catcatgatc tactgatttg tacacggcac cgtcgtgttc cgacggacgt atgaatatgt 105540
ccatggtaaa cgatgtaccc actttggaaa acgtatccca tgcagtaaag catagtccgt 105600
ccattataaa ctcaggaaca ctcataacaa atcgaaatct gtgaagtttt tcgaacacca 105660
cttttacatg gtctttgtca cgaacatcat tgccgtttac ttcagacatg aattgaagga 105720
acgctaaaga gtttcttgtt tcttcatgaa tctttccatt atacgtccat ccagtttcta 105780
gaattctata tatgcttttt gcatcgaccc cgtaccacca gtacatggga actccgaaat 105840
atatagctgg gtttgagtac caatgggcaa gagtgcccat tgcgtttaaa aagtcttgac 105900
aaaaaaatgc agtttttctg tcgataactt gacttggact acgttcgtgg acatcgtaca 105960
tgtccataat tggttcattg gtaacggtta catgacccgt cattatcttt ttaacaatca 106020
taagatacag tttgcctaaa gtcgaaatat gtataacgtt aatttttaca tgttctccta 106080
acgtaattgc gtttttactt agccattcgt cgtctacaaa aatcttacga tacataggat 106140
ttctctctac gtatcttcta aagtatagat ttaccggtct accggcgaca ttagcgccat 106200
ctatagcagg agcaagctgt atgtatcgtc gtataatgtc tcgtataagc tttctgtctt 106260
ctctgggaat acacgacacg gaacttagag actggtgcca gtgtctttca accaaagact 106320
tgaacctagc aaccaacgcg ttgtcactct ccatttataa ttaaataatt atcccaactt 106380
cgtatgttaa tccttattac cagatagcac cgctccttcc tctccaccac gtactatcta 106440
aaggatacct gtaagggtaa tgtctggata acgggcgtgt gagccaagac gtgttatggt 106500
gtcttcccca ccaacggtcc acttctctaa ctaccggagt gctagacgtt gtcgatccca 106560
ctactgttgt ttctccatta cctgtaatct ttgaagcgca acaagtgtta gtctttgcca 106620
aatcttctaa cggcttaatt aggtcgttaa gtctgtcaat atccatgcac tgaggtgtat 106680
cgttaccagt ttccccaact ttgggaggaa cttcctgttt agtgtccaaa taacccataa 106740

acctgtctct aagcattctt ctgtacgtgc ttttgtcatt gtctatgtct cccaaaaacc 106800
tgtgatttac gcatccgttg tacgtaagtc tgtcccttcg ctctcgctcg ttatttccta 106860
cttcttctat tgtactgtaa tgttgatagt ccaagtaata gccactgttt tcatgatttc 106920
ttgtaaatat aatcggtgtt ttattattga catcgtgttg cctactgtac gtatcttcca 106980
tggatctagg aacttgtcta gaaaattgag gactagaaat acgccttcca aatcctggat 107040
gataaaccaa acgcaatgca ctacagtcga catcgtcact gtctctagtt atcccatcaa 107100
caactccttc tttgtgatgc tccactgttt taaagttaac tcctctgtat atgttgagtg 107160
ttaaaaaaat atccacattg aacgcagttc catattttgt tattttatcc cacgacgtat 107220
aacacaaacc atccataatg aattccggaa ccgatactac aaaattagta tcgaacttat 107280
caaacgaaat gtttactcgc ggttgtgctt cgtatttttt gtcatacaca tctgtcatgt 107340
attgtacaaa atctatagct cctctagcat cgggcatgct aacgtctgga aaatcatttt 107400
ttaactgttc tagtacgaac tgttgatttt ggtcatctct ccaccagtac attggcaatc 107460
ctatcacaag agacttattt ttataataat ttgctacaga agctatatgc cacatgaaat 107520
tatagcaaaa ataatccatc tgtatgttta aaaccggttt actagtctgc tgagagtagc 107580
tatccatgat agtgtttccc tcgccaatac ttcctgacat tattctgtaa acaaccatta 107640
acaaaaatct tcctaccgtt gttatgttgt caaaagttct tatgttttga gcactttcga 107700
gtaaccatat gttattttca aatatacttt tgtaaactgg atttctgtcc acataactct 107760
ttaaaaatag atttactgga aggccgcttg ggttatctcc ctgtataggc ggcgcttgct 107820
ttatgtattc gcgtaacaaa tctcttacaa cttttctagt tcttctaggt atacatgacc 107880
tattattcaa aggctttgtc cagttagcgt ttataaacgt tgtaaagtca ctgactagct 107940
tctccattta taattaaata attacagacg gcaacacagc ggttatctaa tatctgctgt 108000
atcctgtctg tacatctatt tttctgttga gatcaagaag agctttacgt agactctcca 108060
agtgtctttc tagtctgtct aaccggttac ctgtttctct gcagcaatca gttatagttt 108120
tgtaactgtc taacaagctt acgaggcgct cttccacact ttctttagtt ggagctccag 108180
ccgcgtacac tccgttggtt gaattgcctg tatcatcagg ctgagtcaat aggttttctc 108240
cgtcattttc atccatattg agtccaacga acacaaacga gtaagtgttc ctctatttaa 108300
agtattgatt ttagaaaaag gcaagcctcg ctgccctgat tcggcggcaa acacgggttg 108360
aacacgcgga agtcgctcgc ggccgtgaag atctcgtccg cgcacgcctc cacgctcgcg 108420
aagcgcccag gcgagacgcc gtcgtgcgag cggaacccga actccgaggc cgccaccgcc 108480
gcgcccttga agagcacgca gcgccacttc ttgcgcacgt cgaaggcctc gtcgttgggg 108540
tcgaacacgc gccggtccac gcgcgggccg cccgccgtgc gcgcgaactc cagcgccgcg 108600
ttcgccgcgt tgaactcgcg gatgttgtcg tagttctcgt agacggccca gagctgcagc 108660
gccacgaaca tggcggccgc cgccgcgagg gccacgcaga gcgcggacac cgcgtccatc 108720
ttttatgtgc agaattattc gttggcgcgg agctcgcgca gctccgcggc gcgcagccgc 108780
gcgaaggctg ctttgagcgc gcgcagcagc tcctcggtgt ccgcgcgcag catgtcgaag 108840
cggtggtagc tgtccaggcg cgcgcggcag ccgaagaagc gtgcgacgca cgcggtgacg 108900
atgtcgttca cgtagagcac gcccgaggcc gtgcagtaca cggagcgcgg ctcgcgcggg 108960
tccggcggca cgtccacggc gaccgcgtgc gcggccacgt cctcgagcac cttgcgctcg 109020
agcacggcga ggaagtcgcg cagctggcgg cggttgtcca gccaggcgta ggtggtcgcg 109080
aagagcgtga gccgcccgcg cggcgcgatc gcggtgtagg gcgcgtaccc gcggaactcc 109140
cgggggtgca cgaccttgac gttctcgtgc tcgcgacgga aggcctcggt gtcgagcagc 109200
gccgcgagcg cgtccacgag cttgtcggag acctccacgc ccgcgccgaa ggcgatgagc 109260
tcgatcttct gctcgctctt ggggcggaag tcgtggaagg tgtgcagcag catctcgcgg 109320
agctgcggcg gcttctcgac ggcctcgagc gcgtcgccgc ggacgaggaa gtagtcgagg 109380
tcgtgcagcg agacgtgctg cccggcggcg ctctgcacaa acttgaggaa gacgcagagg 109440
cccgcgcggc gctcgagcac gtcctcgacg tgcgcgtgga atacgtgccg cgagggcatg 109500
gcctcgatcg cggagagcca ctcctcgttg acgcaggtgg tggtgttctc cagcaccacg 109560
ccctgcgtga gcgcgggcca ctgcaggtgg aaggcgaact cgtgcttgat gagcgaggcc 109620
acggccgggt ccaggtccac ggccagcgcg gcctcgccga cgaggggagc gtccgccatc 109680
acgcggagga cgcctggccc atctcctttt tcgccttttt attcaggatc attattcttt 109740
cgttgacgag gtccatgagc atcttgatgg cggcggccgc ggccgccgcg tcgccgccgc 109800
acatctgcgc gatgcgcgtg agcatgtgca gcagcgcggc ctcgtttagg tcctcctcca 109860
tttagaggcc gtgaggcgcc gcgtcgtcgc gacgagggga cgcctcccgc ggcagcgtgg 109920
tgcgcacggc gaaggcgagc agcgcgccgg cgcactgcgt gagcacacac tccgcgagcg 109980
cgacgaggag ctcggagggc gcgagcacca tttagaggcg cgcgcgggtt taattgccgc 110040
cgtcagagtc ggcatcatcg cccttgtcgc cgccgtcctt gcagtcgccc ttggtctcgg 110100
cgtcgacgat gtcggcgagc cgcgtcttca tgtgcgagaa ctgcgcgagc aggatgccgg 110160
ggtcgagaca gcgcttgacg acgctctcgt cggcgaagtc gtagcagatg cgctcctggt 110220
tctggcagaa caccgagtct tcgatgatca acaccctcct ggtcccggcc gaccgcatga 110280
tggccatggc ccggatgagc ctcttcttcg atccgcgtat ggacatggac cggagcacgt 110340
tctccacgtc ggagtcggag acgttgcagc agcagaggtg cgtgatgctg gcgcgcccgt 110400
tgacggggat gtgcttgtag gtctggcaga gcagcaccag cgacacgttg atgtgccgcc 110460
cgtagttcat gaggcccaag agtgtgggcg accgcgtctg cgtgtcgccc atatcgtcga 110520

gaatgatgag gaacttctgc ttcttcgtct gcgcgtgccg ctcgatcttg cgcttggcga 110580
ccgagaggtt gtactcgagc tcctcgtgcg tggtgacctt gtggatgtgg tccggccaca 110640
cgaagccgtc gtaggcggcg ttgtagacgg gcgtgaagag caggatgtgc ttgaagcggc 110700
gcacgagcgt gcggaagagc gagagcaggt aggcggtctt gccggagccg gagccgccga 110760
cgagcgccat cctgaagggc gcctcgatga gactctcccg cttgaagcgc acctcctgca 110820
cgacatccat cgtatattta ctgtcactaa attaccggct ccgagaaata tagaaattag 110880
agcctcctag agcacaccga ggcgcatcgg caagatggca cataacacgt tcgaaaacga 110940
tagcgagtcg gcagctaaca accagtacgt ggcgtcagtc aagcgccaga aaatgattcg 111000
gcgatacatt aagatgttct tccggttcgt tacggcgata gctatcattg tcctggctat 111060
tctagttgtg attctgtcgc tatctctaga cgaatgtctg cacagagaac accctcatga 111120
ctattcacat gtacaaaatt caacatgcga cggcattact ttaggtggtg aaaagtgtct 111180
cagacttaat ttgccagcaa cgtgggaaga tgctaataga caatgtggta atcttgggtt 111240
ttacctacca tctactggcc ttgaaaagaa atttccttgg cttgtgacct atctcgacgg 111300
aacttgggga aacactcaga actccgtatt tggaccaacc ggtgacttgc agaatgtcat 111360
aggaccgaaa gaatacaaat atttttgtgt gtccgattag atgattataa tctaataaat 111420
gggttgctgt aaggtcccta accgccagtc tataaggact ttgaaaaagg cgtcctgtcc 111480
ggtcgccagt ctcgtcacca ttctctccct agtcaccagc ctcggtgcga tagtcagata 111540
caccaatttt tttctaaaag aagcatgtga cgaaggatgg atgcccataa aaaacatatg 111600
cattttaaac acgcactttg aagccaccaa tgacgatgcc cacaggatat gtgaaaacct 111660
agacggaaag ccgccggcca tccctaaccc tactctgtta aagggtgtca cagttctcac 111720
cggcgaaaag aaattttgga tgacccatca cgaagactat actactgtgt ttgagcatat 111780
agacgatagg acgactccta aaaacacaga ctatgacagt aaaaaacaca cttgtttgat 111840
gagcgaggac ggattgatac accataactg catgatgaac gtgactgtgg tatgcatgaa 111900
ggagatgcac ggataactga aaatatactg tttgaacgca aagacgccat gtcgcgactt 111960
caaatactga cctcatttgg acaaatcttc gcacccgacg aagctcggct gcgcgagatc 112020
gcgcgtgatt tgggaatatg caccataaaa cgcgcattcg gcgacatgct gtacggcttt 112080
atagacttcg acccggtgcc cctgacccaa gtaaacatgc tcatgtccaa ctgctacttc 112140
gcggtcaacg gcaacctgct tccgtgcacg gaggacttcc ggctcagact cccggcaacg 112200
gagatctctg cggcctacct gacgagaacg ggacggacga tcctgtgcgg caaagacttc 112260
aacatagtag cgccgtcggg gttcaagccg tccatgcggc tgcgcgacct cagtcacgtg 112320
tctgcgcttg tagagatcct ggaagtctac gacgagtccg gggagtacca attcgtgctc 112380
ggccccagcg cgcagttcat gctgcggctg atggagaagg agaacgtctg tctgttcggc 112440
agcgggtggt gcatagtgga cctgcgcaag ctggacgtac ccatataatc agcatccttg 112500
tttttatcct gtctttttat cagtttttta gctagttaaa acataaatag taaagctaaa 112560
aagaggagtt ctggagtctt gcaacaacca ggatgaaggc ggtgttgttg ctggcgttac 112620
tgggagcgtt caccaacgca gcgcctttgt tagaaagcca gcgttctaac agtgaggaaa 112680
aagcaaattt ctgctcgacg cataatgatg aagtgtacgc caggttcagg cttcagatgc 112740
gcgtgggtgt acgacacagt ccgctctaca ctcccagcaa catgtgcatg ctggacatag 112800
aagactccgt tgaggacata gaagagtcca cagaaaaaga atacgcgtct acggccacgg 112860
gtgaggcggc cggagtgaac gtgtcagtgg cactagtggg agaaggcgtg agcataccgt 112920
ttagttacat aggccttgga ttcaaccccgt cgcttgaaga tagctacctg tacgtcaacg 112980
tctcgtcacg agctccttgg gttaaacaga cttcggacct atccgcgaac ggcggctggg 113040
gtatcaaaca ggttctagaa aaagagttac tggccatcca aatagggtgc gacaaccaaa 113100
aatttcccga agaacccaca actacacccc cctcacctgt cactacaacg ctttcctcaa 113160
caactccaga tctgaatgaa gaaaacacag aaaatacgcc gacgaccacc ggcgccagtg 113220
tagacagaaa gcgcaatcca gctgacattg acttctcgct gctcgtggac ccccgatgcg 113280
tgacctctgt agacctgcac gtcgagctca gggacgcgtg catagactac aaacaagagt 113340
cgccgttgtc gctgaagggg aaatatggag acggcgaact agtaaaaaag gagattaaag 113400
acgtgggaaa gaatcacaat atgtgcagtc ttaacctcaa ccctggcaat tgagctgttt 113460
ttattcggca atataatagg tgattattga acattaaaca aaacttatcc cacaacgccg 113520
caacaatgga agtgctggtg atcatctcta ttatcgtcgc cgtaatatgc ttgaccggag 113580
cggtaatgta cctccttatt gaactcggct tagccgccga gcgcgctaac aaacgcgcgc 113640
gcgtgaagaa aaatatgcgc aaattagcca ctcaattggg aaatggatct gtcgactccg 113700
gcataggcat aggcccgtgc ataatgtcgc gcaccatgga ctctggaccc agtcgctggg 113760
acagcgacag tgaggatgac ggggacagcc tgtccacgac gtccaccagc ggagggggga 113820
ctctcacccg agtgtgggtt gggagcgccg ggcctatgta cgaaaacttc tgcgggaacg 113880
gcacccgcca ctcccccacc aacgaccctg gctaccactc gcgggagact ctctgcagcg 113940
gacctccccg tcaggcgccg gcgctaccgc ccaccccgaa gcccgacgag gtaacggtgg 114000
acgtggggcc cggtcccgac gaccaacacg gtccgtacga ggaacctgat cccattcccc 114060
cgcaggaacc cgagccgccg gtgcagattg aggtaaccat caacgggccc ggtggagaag 114120
gcgaggcgga aggagaattt ttctacgacg agtagccgcc aaaactgaat aactatcggg 114180
cttcgtaaac gcgcagacat gccgctgttc cggaagctca tggtttcgcg ctccctggtc 114240
aaggaatgtc tgactctgga cttccggcag ggcgagcgtc tccctacgcg atgcttcctc 114300

ccggtgcccg cggggacgac attccacaga gtctgcgaca cctcgccgct gacggacgaa 114360
gtctcccggc acgtgcagga gcccgtcatg ggcaccggac gggtccagta ctactacttc 114420
gagagcggcc agggcatgat cggcgacaac gcgggcatgg cgcgcatgct cgtgtgcacg 114480
cggtcggcgt acaacggcgg cgacgtcgtc gtgcggtcca cgcggagcag agcagacaag 114540
accgtggtcg cgccctgcca gggcatggcg ctgctgctga gccccttctg cgccttcgac 114600
atcacgccgg tcgagagcgg ctccgcgata ttcgcggagg tcatcgtcac cgcgcccagc 114660
atggactacg tcgaggcggt caccggcacg ggcgaggcgg ccgtgcggat attcaactcg 114720
caccacccgc tctggccgcg acacggctcg aacgtctgct tcgcgctgcg gttgctgcga 114780
gacgtgcgca cgggcgagcg cgtggtcgag cagatgttca tggacgggcg ctggcacacc 114840
gtgctgagga cgtcctgcgg caacaaggtc tgcgtgcccg ccgacctcgt gggccagacg 114900
aacctcgagg aggtgccctt ctgcgacgtg acgcccgaga tcatgcgccg cgcgctggcg 114960
atcgacccgc cgtacgaggc cgtggcgcac ccgcaccgct gcgtgtacgg cgccatggac 115020
gtccggtgcg cgaacgagta cctcgtgtac tgcaccttca agacggagcc gacacggcgc 115080
agcacgtcct cgccgggccc ggacagcccg ctgtcgcccg cgactccgtc gacctcgcgg 115140
gccgcggccg cgcgcgtccc cacgacgccg caggaagtgg cctcgccgac cacgaggctc 115200
ctggagacct gtctgcgcga cgccctcgac ggactctgac ccgaaggacc caccgtccac 115260
tcacattcca ctgccagaca actcaagctt tttctgcatc tacctcgcta ataattgaat 115320
tgttatagga caaacaggcg cactcgagca caatggcgtg ttttatcgaa ttgttagact 115380
ccatcttcaa ccgacgccac cgtaatttcg ggccggagga catgtacagg ccctctgacg 115440
ccccgccccc caaatcgcac acgcctcgca ctccccgcac cccgcggacc cagtgtcccg 115500
gacacccgcg gcgacaaagc tcctctccca tctacggcgc ttatgtggac tccctgccga 115560
ggaacagaaa gcggttccag aatcaacaca gttgtcccgg agattacgag cggtgtcaac 115620
tctcggacac catcagcctg gatgcgacgc tactcacggt tacagtgacg tccatctcca 115680
gcatatccag ctctagtagc tcagactcta tctctctggg gcagtgcaga ctgtccatgg 115740
tgtccgcaac atcaacctcc acctccacaa ccttctcctc ctcggaatga gcgccacact 115800
tatttttgta taatagtttg tattgaacct tagagacatc cacaaatagt taggaagcat 115860
gagtagttca agtagcgaga ccacccctaa gcccaagccc atccctgctc ctcccatgac 115920
tcaggaggag tttaacaaag aagtgaagaa acgaaaagaa cagaaaaagg aaaaatctag 115980
aaccgttgaa cgtgagtcag aaaccgtaac tgtatcttcc gacggatcag agataaaaaa 116040
gacttacgag cgcgagtctg agagaacaac cgaaacagaa aagaacaaca cgtcaaccga 116100
tgataataag cagaacaccc ctgtagagaa accagaggaa accaagcctg cttctactcc 116160
tgaaggtgtg aagccagccg agactcctgc cccgactact gacccccaac ctactacaca 116220
accacccgca gaatcaaacc ctggaagtca acccgcacct gcttcagaac caacccccgc 116280
acctgagcct gcaccggaac ccactcagcc tgcatcagta actcaacccg ctccaacacc 116340
agagccaagt ccagccccta agcctactcc ggcttctgaa ccaaccccag catctgagcc 116400
tacttctgct ccagaaccta caccatccgc agaaccaact cctcaaccaa ctgtagaaac 116460
accaccatct gctccagcac caactcccga ggcccaacca cccgccaaca gcaatcccac 116520
tactgaaact accactggta ccagcacctc ctaagtgagt acgtaagcat ttcggagtaa 116580
cgtcgtagca agcgctagtc cgccgcgagc ggttctcgca agttttttcg ggtaaaaagc 116640
gtacaccgtc gccttgtcgc ggcggtgtac gctttttca cgccctttt gcaaaattta 116700
aattgtaccc gcgccggctc taggaaagat ggcgtgcctc agagtgttcc tggcggtgct 116760
cgcgctgtgc gggagcgtgc actcggcgca atggatcggc gagcgcgact tctgcacggc 116820
ccacgcgcag gacgtcttcg cgcggctgca ggtgtggatg cgcattgacc ggaacgtgac 116880
cgccgcggac aacagctcgg cctgcgcgct ggcgatagag acgccgccga gcaacttcga 116940
cgcggacgtc tacgtcgccg cggccggcat aaacgtcagc gtgtccgcga tcaactgcgg 117000
cttcttcaac atgcgccagg tagagacgac gtacaacacg gcacgccggc agatgtacgt 117060
gtacatggac tcctgggacc cttgggtgat cgacgacccc cagccgctct tcagccagga 117120
gtacgaaaac gaaacgcttc cgtacctgct ggaggttctg gagctagcga ggctgtacat 117180
tcgcgtgggc tgcacggtgc ccggagagca gcccttcgag gtgatcccgg ggatcgacta 117240
cccgcacacc ggcatggagt ttctccagca cgttctacgg ccgaaccgcc ggttcgctcc 117300
ggcgaagctg cacatggacc tcgaggtgga ccaccggtgc gtgagcgccg tccacgtgaa 117360
ggcgttcctg caggacgcct gtagcgcccg caaggcgcgg acgccactct acttcgcggg 117420
gcatggctgc aaccatccag atcgccggcc aaaaaaccca gtaccgcgcc ctcagcacgt 117480
atcgtcgccg atctccagga agtgcagcat gcagacggcg cgctaagggc gctcaccgcg 117540
ctgacggcgg ccgtggtgtg cgcgatcgcc atcgcgctcg agcgcggggc ggaggccgac 117600
gccgtggacc ttatccttat aaaattttca atgatatgct agtttttatg cgaccttcct 117660
tagaaaattc ggaattcaaa aatgaaataa aacggcgttt agcacgcata ttattaatac 117720
cgaccaccat ggcaggcgtc cgcagctgcc agaagaaagt cccttctact gcgggctcca 117780
tgtcatttca acggggcaac cggagcatcc agccggcgat gtccgaggcg ttgcagaatg 117840
atttcagcta caacccgcga ccgcctccgc cgagcgcaga agagattgac ttcttctgcg 117900
tggacatgcg caaagtactg atggaaattg aggccaagcc cagcagctcc aagtaccccg 117960
atttcatcca cccggttgac agcagcccgc cgtgcacgcc ggcgcgcaag cgcaacggct 118020
tcggccgcaa ggcactgaac aaaacccagg tgccgcagca ggccaagcgt gacggctact 118080

cccgctaatg cagtccacac acttcacaca ctacatcagc actcaagctt ataatcacta 118140
cacaatgaat cagcccacca cgtgcgaagc acacacataa aatcacccac ctgtcctgat 118200
cgttcccaat actcccaatc accgtgcttt acacgcacgt taatcaccct ttccttcctt 118260
catgcgttcc tgatcgttcc tcctccttaa tcacacacac accccgtaat tttgtacttt 118320
tgtactttaa tttgtacact ttacacactg actttgtact gcctttgtac tttatttttg 118380
tactgaaatt ggacgatact tatctttgta ttcacatcca agttttgcaa attccacagc 118440
cggtagcgaa aagtgaaatc gtaccgtttt aggcttcgat ccccctcccg cgcgaagact 118500
cgccagcatg gactctcgta ggctcgctct tgccgtcgcc ttcggaggcg tcctcgccag 118560
catgacacag cgccgccgcc tggcttctct catcgccagc atcggccaac ggctgatggg 118620
cggcgacggc atgcgtcgcg tcgccgttcg gttgatcgac cagctcatgg ccggaccccc 118680
ggacatcaac gacgaggcct tccagcgcga gatccgcgtg ggcgagctct tccaggcgct 118740
ccaccgcgtg gtcgagcagg cacgccgaga gaagtacttc gaggtctgcg gcgccggcaa 118800
cgacgccgac gcgcccgtcg tcgagatgga caccgcggcc gcacccccgc agccccagcc 118860
cgcgcccctc gtggtcacgc cgcagaacgc gttcatgttc gtgccgcaag gcagccacgt 118920
gcacgtggac gagagcgtgg acccgttctt cggcatgagc ccctccatct tcgggcgcga 118980
cctccccccct cagccgcccg aggagctgct gagcgactac gacccgctca tgagccaggc 119040
cggcgagccg ccgagcccgc ggtcgccctg cgaggccgac ctctggtgct tcgagacgct 119100
cggcgacagc gacagcgatt gagaccgcac cacaccctac ctcacccacc ccacactcca 119160
cctcacctca ccctaacacc cgacacccaa cacttcaacc ggacaatgaa ggagtcccac 119220
atttcactga aggacgcgga tgaagccgca ctcccccaca tgaaggattg gcaacggtca 119280
aacatttcac ctgcaatgaa ggacgatgcg cggtcgcatt ggcctgcgac cgacatcgca 119340
cacatgaagg acacaattgg tttgttaatc cggacaatga aggacaaatt gtttttgtta 119400
atcaggacaa ttagaacaca atcaaatttt tgtacgatca taaaatcgat atttgatgca 119460
catatattag taagtatatt agactaaatt ctccggggag gcaagcagtt ggatacggcg 119520
gggcggggca cgacgtgcac ggagagttcg ggcgggtccc ccttcccccc acccccacgg 119580
caccacgatg cgcctaatct tagcgctcgt ggcctgcttg ttggcggcgc cgatgccgtt 119640
atcgggtcgt tcgacaagca ctccaaacac gtccccctcc gcactcggct cgacgagttc 119700
ggaaccaagc tcggaagacg ctgtggcttc gagcacaacg acaagcacac tcacaagcac 119760
tacaagcaca ctcactatgt ccacaagtgt ggacaccact actacctcgg gcgctacaac 119820
gtccgcaaac agcactcctg cagcgagtgt gagctcctcc acacccgcaa ctaccgaggc 119880
atcgacggca ccaacgacgc cgtcgacgcc gacgacagtg aaggtaacga agggcaagga 119940
agacacgaag gcgtctgcct acctcgtttt actaatcacg ttcatggtca tgaccacgct 120000
cgtgatggtc gtggtcgtgg tcgtggtcgt gtacaaacag ggactctgta actgctgctg 120060
taagtttccc ctgctgcaaa gagctcaagg actacctcga cgaggaggag agcgccgggc 120120
tgtacgacgc cttgacgtgg agccactcag actccggcct ccggctcgtc gtgcgcgcgg 120180
accccagatg atgaggatcg gataagatcg gcgtgttttt cccgcccgtc gcgaacatta 120240
tgcctctaaa tgccgagaat taactgaaat tcaaacacgc tttgggactc aactccgtga 120300
cccacactca accatggctg gcttcctagg cgcattcaga ggcgtgtgct ccgacttatg 120360
gcagtcgctc cgtggacacg gacaccactc ttccagctgc ccgcgacgac gcgccaacag 120420
catggacgac cgcgaccggc gccgacaccg ccaccgcgag atccccaaca gctcggcgtc 120480
gctgaacagc gacccgatgc cgcaacgcag tgcgggtgcg cgccggcact acgactgccg 120540
cccctcggaa aagagcagac actcctccga caagcaccac tcggcggacc gacaccactc 120600
ggcggaccga caccaatcgg cggacaggga cagacaccgt cgcagtcgca agaactacga 120660
ctcgcacccg tcgcgcagga accgcaacta cgagcgggcg gactaccaga gacacccctc 120720
acaaacccac ccagacgccc ccgcgcagac ctcgacgctc aaggtgacct ccctaagcac 120780
cagctgcagc accctgtccc aacatcacta cgagaccccc gaccacatct acgacatccc 120840
ggaagacagt cgcggggcgt cggctccccc tcgcgcggac ctcgcgctcc ccccgctcgc 120900
catgcccaaa tccaagccgc gtcgcacgcg cccggcgtcc atgaacgact gcctgatgaa 120960
gcactgcggc gccggcagac ccaacctcca agacgacata tgcacactat gtactgatat 121020
agagacacag ctgagcgcac tagagaagtc tctggagtca gagctcaact tctatcgtcg 121080
ctacatacaa gacactaaga cattgctcgc cacgcgagca gcaaacatcg gcagcaaagc 121140
tctgatctac accgacgact acaacggcag tggcgacgtc ggcgaaaagg agcactgctc 121200
ggaggagtgc tgcaaagtgg aggaagttct gtgagaaagt gcgtttttct gtaatgtgaa 121260
ataagatagc cttatgtgtg cacagacatg gcgaacaggc tcgtgttttt cgacccccgag 121320
accctagccg aggccgacgg catccccggc tatggggtgt tcgagcccgg caagaagaaa 121380
tgcatcttca caaagatccg caccagcgtc gcactcgcgt gccggtacgc cgtctcggac 121440
ggcggcctca tcgacgagtt cgtcatggct acatacggga ccagacgcgc gtgccggctc 121500
gtccggcacc tgacgatagg cgcggagggc gtgatgaccc ggcccgccag caactgcgcg 121560
ccgcacatgg tgctcatctg cctcagaggc gtggccgccg tgtccagcga ggacatgggc 121620
ttcggtcgct gcatcatgga gcgcggcacc atgttcatgg tcaagtccgc gcacagcgcc 121680
gtcgtctgcg gcaaccccgc ctgcgagctg ctcgtcctct tctacgacta cttcaccccc 121740
atccccccggc cgctctccgg agacgaggtg ctgttcaccc gcgacctcgc gcacgtggac 121800
tacgcccccg agtcggcggt cgtcttcaag atggattaca acctcgagac cgacgtggcc 121860

acgctgtttg tcgggggggta catattccgc gccaagggcc tgatgatgga gacgcgcgaa 121920
caagtgggcg acgagtgcga ctgctgccgc cacagctcgc cggtgctcgt catggatcgc 121980
gagaagatga tgtcgtcgct gcgcatgatc cccagcatcg tgcccggcca gcgggagatt 122040
tggcttcgcg agcgcggctg ggccgtcctc gagacggacg cccgcggaca ctgcgagccc 122100
ggcgtcctga ggctggcgct cgccggcctg cggctgttcg caggatgcct gcgctccgtc 122160
gtggggcggc gcgagctgtc gctgttctgc tacggcgtcg ctcccaagtt cggcggggag 122220
ttcgaggacg cgccgcgccc catggagatc gacggttagt tgtttttatc cctgtacata 122280
cgccgcaaac tgaaacttta gggcaccgcg taatagtgca cgaacgccca gtggaccgct 122340
tccgcagcca tggaaaacaa cgacggcaac gaacgcaaca acgaacaccc gcacgttcga 122400
gaattcaagg aggcgtccct gtacgggttt ctggtggcgg ccgcggacgt gaccgtcgaa 122460
gacgtgcacc ggtaccttca gttcggcgcg gacgtgaact acaggggcgc gtacctgtgc 122520
acgccgctgc acgcgtacct gcagtccggc tgcgaaaagc gcctagacgt cgtggacgcg 122580
ctgctggacg ccggcgcaga catcaacgcc aaggagatct gcggtctcac gcccgtgcac 122640
ctgtacgcga gctacgcgga cgtggacgta gagttcatgc gcgggctcat cgagcgcggc 122700
gcgagcgtgt gcggcgagag ctcggtcacg ggctgcctgt actcgtacct gtacacacac 122760
agcgtggacg gcggcgcgcg cctggacgtg gtcgagctgc tcgtgcaggc gggcgcggac 122820
gtgaacgtcc gcggcgaggc gcgcaagacg ccgctgcacg tgcactgcgc gggcttcgag 122880
gtggattcgg acatcgtgga tctgctgctg cgcgcgggcg cggaccccga ggcgctcgac 122940
gaacacgggc tcacgcccgc ggacgtgctc gtgaagtccg tgggcgccaa cgtggagacg 123000
ctgcggctct tcctcgacgc gggcgtgagc gtggccacgt cgcgcgacgc gcgcggacgc 123060
acgccgctgc accaccacgc agactccttc cgggcgagtg cgggcatcgt gcgcgaactg 123120
ctcgccgccg gctgcgacgc ggcggccgcc gacgacctcg gaaacacgcc cctgcacagc 123180
ctcgccacct tctgctcgtg ccggcgctcg gtgctcgacc agctcatcgc cggcggcgcg 123240
gacatcaacg cccgcaacca ctacggccac acctgtctgt actacgcgtc catctacaac 123300
ccctccgtct gctcgcggct catcgccgcg ggtgcggacg tgaccgcgcg cacgccggac 123360
ggacgcacgc cgctctcggg catgatcatg cgcaagcaca cgcgcgccgt gcgcgccgcc 123420
ctggcgacgc ggcctcccgc ggacgccgtc gccgcgtcgc tggacgtcgc agtacagccc 123480
gagcccacgg acgccactcg cgcgtgcgtg cggtacgtgg tgctctgcgg cggcacgctc 123540
tcggcgcgcg tgcggtcgcg acacgcggac ttcgtgcgag agtgcgaaag cgaggtggtc 123600
gtgctcagaa ccaccgtggt ggggctgccc ggcacctcgc tgctggacat cgtgcgtgcg 123660
gcgcagccgc cgccggtact gctctccccg cgcgtgcacc acgtgctgca gaagctgtgc 123720
gtgtacgcgg agttggtgga cgcgcggctg cgcgagatgc ggcacaagac caacctcgtg 123780
gacgcggtgt cgcggctcgt gtgtccgtgc gcgctgccgc cggaggtggt gcgcggcatc 123840
ctcgtgcacg tgccgataga cagcctgcgg cacacgttga ccctcggcgt ggcgcaggcc 123900
tcgcgtttcc ttccctcgca taaatgaaat attatttttt gtggtagacc ggatctcccc 123960
gatggacccc gccggacaac gactgcgcgc gccagggccg tggcgcctga acccgccgac 124020
cgcggccgcg ctggaaagcg cgctgctgcg gcccgcggcg tcggcgggcg ccgaccgctg 124080
cgcgaacgcg cacgtggacc gccgcaacat gggcgtcggc gagggccgcg aggtgcccgc 124140
ggacgtcgag gggctcatga ccgagatcca cctgcggtac ggaatgacgc gcgtccaccg 124200
gaacgttcac ttcgtgcagt tctggcacgg cgagcacgtg cgccggcgcc ccgcgcgaca 124260
cgtgttcacg gtctggatct gcctcagcgg cgaggtgcgc atctacgcag agtgctgcca 124320
ggcggggcac ggcttcgtgc tctgccgaca gatggcggca gggtacatgt tcgtgaccga 124380
acccacggac tcggtcacgg tctcggtgcc gcaccgactg cgcaactcgc ggtcgccggt 124440
gtggctggcg gcggtcttcg ccacgcggca cttcgagccg ctgccgccgc ccatgtacgc 124500
cgtgcccggg cacgtggtgc tcgcgcgcag cgcctccatg ctctgcgact gctggccgtc 124560
ggacccgcgg cgccgcaacg tgatcttcta catgcggctg tcgggcgcga tggtgcgcgt 124620
ggtcgtgccg ggcgcggagc tcgagatcga gtgcacctcg ggttccggc cggaccactt 124680
ctccatcaac gacgagtgcg tgtgctgcga gcgtccgcac gtcgcgcgaa ccgcggtgtg 124740
gacgctggcg gagatttgcc gcggcgccac ggtggtgctc gcgccgccac tgccccgcga 124800
ccgcgccgcg gggctgctcg cggagatccg cctggcctcg ctgcgatggg tgcgcgtgcg 124860
tgcggtccgc agcggcagag aaagcgtggg cccgttcccc tcggtggtgt gggcggcggt 124920
cttctccgcc gttcggctct tcctggacgg aaccgtgcct gccttcccgg cgtgtgtgga 124980
gaatggacgc gcggcgtacg gcatggtgta cgtgcccccg gaggagccgc ggatggacgg 125040
gctctgtgtg ttcccgacgc ccgccgagcc ggcggcgctc ttcgtccgcg gagaccaggt 125100
gcttgaggcc ggcgcggccg ccgccataat cgcggccgct gagaagcgcg tccaggccgc 125160
caatgggtct cctgctgccg cggaggagga cataggtgcg gcggccgatg ccgccgcaga 125220
gagcgtggag caggaccagc gcgtcgagtt cgaccttggg cctgggcctg accccagcca 125280
agaagcgccc gcggacgcgc agcgtgccga ttcggacgac gacaccggct ccgagaccga 125340
gaccggcgac gagagtgtgg gcggcgagga tgacagcgac tcctcctcct cttactcggt 125400
gatgtcggac gacgaaaacg acagcggcga cgagggctgg ggcgactcta gcgactccgg 125460
catcgaggac gacgacggcg gtgtcgccag gccgccgagg aagaagagga ggaagagcgc 125520
gacgtcctcg gcgcagcggc ccagatgctc ggagactgac cggtggtgaa aacataaaaa 125580
taaactgttc aacacttgta ctccgggcac caacactact atccataccc accctccctc 125640

cacacactac aatggcaaac agagaagaga ttgacgcctc cgccgtcatg gctgcctacc 125700
tcgcgagaga gtacgcggcg gctgtagaag aacagctgac gccgcgcgag cgcgatgcgc 125760
tcgaagccct tcgcgtttcc ggcgaggagg tccggtcgcc gctgctgcaa gaactctcga 125820
acgcgggcga gcaccgcgcc aaccccgaaa actcgcacat ccccgccgcc ctcgtctccg 125880
cgcttctcga agcccccacc tcccccggcc gcatggtcac tgcgattgag ctctgcgcgc 125940
agatgggccg ggtatggacg cgcggccgcc agctcgtcga attcatgcgg gtcgtgtacg 126000
tgctcctaga ccgtctgccg cccacggccg acgaggacct cagcacctgg ctgcaggccg 126060
tcgcgcgcgt gcacggcacg cggcgccgcc tgcaccgcgt tctcggcgtc ggggccgtca 126120
tggcaggcgt cggtatgctg ctgctcggcg tgcgcgtgtt gcggcgcaca taacttttta 126180
tctcggctca aactgaaata cgacattgga ctacgaaacc tatgattttg ctcacggccg 126240
cgcgagatag gataataaat aacctttgag caactaacat ggccgatgag agagaggccg 126300
acggcgcgct gttccggtac ctggagagcg aggaccgtcc ggacgtggag cacatgcgcc 126360
ggctgctgga cgagggtgcg gacgtgaact acgcgggccc gcgcgggtac gcgccgctgc 126420
acatgctcat gcgcggcaac ccgctagacc ccgacgcggt gcgactgctg ctcgccgcgg 126480
gcgcggacgt gaacgcgaca tcgctctgcg ggttcacgcc gctgcactcc tacatgtgct 126540
tcgggaccgt gacgccagac acgctgcgtg cgctcatgcg ccacggcgcg agcgtcagcg 126600
acctcgagcg caacatcaac gcgctgatcg agtacttcaa ccgcgacggc tgcatgggcg 126660
gcgcggaggc gaccgtgatc gcactgctgg tggagcacgg cgcgcacgtg aacgccaaag 126720
acgaccttgg acgaacgccg ctgcacatct acctgtccgg cttcttcgtg tcggcaccgg 126780
tggcgctcgc gctgatcgcg ctcggcgcga acccgaacgc cacggacgcg tacgggcgca 126840
cgccactgca cgccttcctg cgctcccgcg acgtggaccc cgctgtgctg aagacgctca 126900
tcgccgcggg cgcagacccg ctcgcgcgcg acatcatccg gcgcacggcg ctgcactacc 126960
actgcgagtc cttcaagacg cgcgctagtg ttatagagac gctggtggcc gccggctgcg 127020
accccgcgag cacagacctg ctcgacaaca cggcgctgca cagcatggcc atgggcagct 127080
cctgccgcgc ctcgctgatc cgcccgctgc tggccgcggg cgtgtccgtg aacgcgcgca 127140
acgcgcggct gcagacgccg ctgcacctcg cggccgtgtt caacccgccg gcctgcgcgc 127200
ggctgctggc cgcgggcgcg gaccccgcgc tcgcggacct ggacgagaca acgccgctgc 127260
tgagcatggt gcggcacaac tgcgcacgcg cgctgcgcac ggcgctgccc ttggcgccgg 127320
acgcgctggt ggccggcgcg gtgaaccgcg tgaacgcgcg cacgccgagc gcggccacgc 127380
gagagtgcgt gatggcgctg gcgctgcgcg gcgcgctgga cctgctgagc gcggagagcg 127440
ttgccaccca cgcggccgcg atccgcgcct gcgaggcgga ggtcgcgctg ctgcggagca 127500
cgcgcctggg cgcgccgccg acgacgctct tcgcgctgct gacaggacga ccgaacacgc 127560
tggtttccgc gaaggcggcg cgacgcgcga tggcggacgt gtgtgtctac cgcgcggcgc 127620
tggccgcgcg cgtggagcgc gtgcgccgca agtcctcgct ggtcgagcgc ctcaccgcca 127680
tggtgtgtcc gtgcgctctg ccgccagagc tagtgacgcg catcctcgcg ctcctgaccg 127740
tggaggaact cgcttgcgca atgcgcaaat aataatgaac tataactagg cttattagag 127800
gcactatttg tgcagagtcg ttagttatag ttagtgtact taccattgga atgtcgaaga 127860
acaaaattct ggtgtgtttg gtaattattc ttacttatac attatacaca gatgcgtatt 127920
gtgttgagta tgaggaaagt gaggaagata aacaacagtg cggtagtagt agtaattttc 127980
ctgcgagttt accgcacatg cttagagaac tcagggcagc gttcggaaag gtaaaaactt 128040
tcttccagat gaaagaccaa ctgaacagta tgctactcac acagtcgctc ctcgacgact 128100
tcaaaggcta cctcgggtgt caggcacttt ctgagatgat acagttttac ttggaagagg 128160
tgatgccgca ggcggaaaat cacgggccgg acatcaaaga gcacgttaac tcgctgggag 128220
aaaaactcaa aacgctgcgt cttcgactgc gtcgctgcca ccgcttcctg ccgtgtgaga 128280
acaagagtaa ggccgtggag caagtcaaac gtgtgttcaa catgctgcag gaacgaggtg 128340
tttacaaggc catgagcgag ttcgacatat tcatcaacta catagaatca tacatgacta 128400
ctaaaatgta aaaatgtata caacttttag ttatcgttcg gattctcgta tcgttctgca 128460
tactatgtat ataaaatgta tattaacata gttacagtta cagttacagc tatattttta 128520
tgctcacaag atgctatata attgaaagga aattgttcac tctctgtcag ggcgccatgg 128580
actttctagg cgccgcgctt cacgactacg ttgccgatgc gcccaaggtc tgcgccgagg 128640
aggtgcggcg gctgctggcc gcaggcgcct ctgtggagta cgcgggcgag ttcgggaaga 128700
ccgcgctgca ccagtacatg ggccgttccg gcgcggaccc cgccgtcgtg cgcgcgctgc 128760
tggacgccgg cgcgcgcgtg gacctcccgg agacctgctg cggctgcacg cccgtgcacc 128820
tctgcctcat ggccgccaat atcgacgtgg aggttctccg catgctcgtc cacgagggcc 128880
gcgtcgagga ctgcggccgc gccgagctcg cctccgtggt gctcaaggag ttcgtggtga 128940
accgcgcctt cgacgagaac gtcagcgagc gagtgatgcg cgttcttgtg gccgcgggcg 129000
cggacgtgaa cgccgccagc gtggtcgacc gcacgccgct gcacgtctgc ctcacgggca 129060
tgtccacgca cccgggcacc atcgccgcgc tgctgcgctt cggcgcggac gtgaacgccg 129120
tggacctctg cggcatgtcg ccgctggcgg tgctcgtgcg ctcgcgcgcg gcgaccgcag 129180
agctggtgcg catgctgctc gacgcgggcg cagacgcaca cgctgtcgac agtcgcctgg 129240
actcgctgct gcaccagcac tttcagtccg cgcgcccgcg gccggaggtg gtgcgcgagc 129300
tcatccgcca cggctgctcg ccgcgggcgc ggaaccgaat cggcaacacg ccgctgcacg 129360
aggccgcaaa acactcctcc tgcaaacact cgctggtggg gccgctgctg gctgccggcg 129420

cgagcgtgga cgcgcgaaat aacacgggca ggacgccgct ccacttggcg gtggcgtcca 129480
acccgcgcgc gtgccgccgg ctgatcgcgc ttggggcgga cgtggtcgcg cgcagttacg 129540
cgggcgtcac gccgctggcg cagctgatcg cggacaataa ctccgcgctg gtgaccgcgg 129600
cgctgaacac gcagcccgag ccgcgggccg tggcagagtc gctgcgagcg accacgcccg 129660
tcggcgagac agcgtgctcg cggctctgtg tggcgtacgt ggtggcgcgc gcgccgagcg 129720
aggtcctcgg cgagcccgag cgcgccctgc acgcggcctt cgtggcggag tgcttagcgg 129780
aggtagcggc catacacgcc gtgcgctgcg gcacacctcc ggtctcgctg ctggagatcc 129840
tggtggccgc gcgcccgccg cggagcctgc tctcgcgccg cgcgtggcgg ctggccagcc 129900
ggacgacagt ttaccgcgcg ccgctccgtg cacgcatcgc ggccatgcgc catcgctcgc 129960
gactggtgga gcgcgcgctg cgcacgctgc gcggctgcgt gctcccgcgc gaggtgctgg 130020
agcgcgtgct gcggtgtctg tccacacagg acctgcgggc ctccggactg gccgagtagc 130080
tttttctgag ataagtgaat aaacatggtg ggattcgatc ggcgccgcca acgccacgcc 130140
atggacgccg ccgagatgga ggatctcgac atcaacgcgg agtcggcgct gtacgactac 130200
ttcatcctga acgcggacag agcccgcgtg ggcgaggtgg ttatgcttct cgcacagggc 130260
gcggaaataa actacgcgga cagcttcgac aagacgccgc tgcacctgta cttgcacacg 130320
cgacacccgc gctcggacgt gattctggcg ctgatggagg cgggcgcggt cgtggacacg 130380
ccggagcgct gctgcggcgc gaccgcggcg cacctgtaca tcctcaacgc ggccaaggtc 130440
gacctgtcgg tgctggaggc catgctgacc tggggcgtgc gccagaacga ccagcactcg 130500
gagcgggtgc tctcgagctt gttgcgcgag tacgtggtga cccgcgccta ctcggatcag 130560
accgagccga tcatggactt gctcatcggc atgggcgccg acgtggacat gccggtcggc 130620
gtgagtcgca cggggctgca cgcctgcctt acgggcctga acgcgaaccc gtgcatgatt 130680
cgcgcgctgc ttcggcgcgg cgccagcgtg accgcaaaag acacctacga gatgacgccg 130740
ctggcggtgc tgctgaagtc cgcgagcgcg acgccggagc tcgtgcgcat cctcgtggag 130800
gcaggctccg acgtgagcgc caccgacttc cgcctcaacg gcatgctgca ccagcacgcg 130860
cagtccacgc gcccgcgcgc gagcgtcatg cgcgagctca tccggctggg gtgcagccca 130920
gcggccaaaa acatgtttgg gaacacgccg atgcacatgc tggccatgga aagctcctgc 130980
cgccgctcac tgatcctccc gctgctggag gcagggcttt ccgtgaacga ggagaacccg 131040
cactacggca ccgtgcctct gcacgtggcc tcggggtacg acaacacgca gggctgcctc 131100
aagctcctcc gggatggagg agaccccacc gtcgtgtcgg ccgccggacg cacgccgctc 131160
tcgaacatgc tcgtcaaacg caaccacgtg gcggtcgccg gcgcgctgtc gacgcacccg 131220
agcgcggcag tagtcgtgca ggctctcgag caggctctcg agaacgtgct gaacgccggg 131280
cccagcgagg cctcgcggct cgccgtggcc tttgtggtgg cgcgcgccgg cgcatccgcg 131340
ctaccggagg ccgtgcgccg tctgcacgag ggctttgtcg ccgactgcga gcgcgaagtc 131400
gagctgcttt cccgcaccat gctcggcaca ccggccgtga gcgcgctggt cgtgctggtc 131460
agcaaggagg tctttggcac tgttatctcc tcgcgtgcgc tgcgcgtcgt gcgggaggtc 131520
cgcgtgtacg caaggccgct ccgcgaggcg ctcataaatc tgcgccacaa atgccgctta 131580
gtttccagcc ttaaaaggca ggtgggacct tgctcgctgc ccggcgaact ggtggagcgc 131640
gtgctcgcga ccgtgccact gaccgacttg cgccgctcgt gcggccgccg cgcgcccgag 131700
taactgcccg tcccgttgct acgcgactcg agactgcccg ctgtttttct ttccccgttt 131760
cttcttatta ggagttgttg cccgcctcca tgatcctcgc acgcgccggc gggcaacctc 131820
gcacgcccgc ggcggccgcg ggcgccgccg aggacggcga gcacagtgat cgccggaagc 131880
gcaagcgcaa gacgcccaac tgcgaagacg ccgacaactc cgacgacgag ctagcgcaga 131940
cgccgtgcga ccgcgagtgg ccggactgtc gcgcgagctc gatcacgagc tccgactcgg 132000
tctctctcgg cgacgagatc tacctgcgat acgtggcctc gcaggtggac ttcgcgcaga 132060
cctgggcccc gccagtgcgg ctgctgcgct ccttcgggaa cttctcgaag gaaacgctca 132120
accgcatgtc gcggcgcggg tacgtgaacc gctcctactt ccagatggcg cacgcgcgct 132180
tctcgcccac caacgacgac atgtaccaca tggccacggg cgggtacggc atcgtgttcc 132240
gcttcgaccg ctacgtggtt aagtacgtct tcgagcaccg caacggcatg tccgagatgg 132300
acgcctctac ggagtacacg gtgccgcggt tcctgcgcaa taacctcaag ggcgacgagc 132360
gcgagttcgt ggtctgcgcg ctgcccatgg ggctgaacta ccggctgggc ttcctgcact 132420
cgctgtaccg gcgcgtgctg cacacgctgc tgctgctcat gcgcgtggag gaaggccagc 132480
ggccctcggt ggagatgtcc aagaagccgc tgctgcgctg gttcgaggcg cgcaaggaca 132540
gcgagtcctt cgtgcgcctg atctcgtact tctacccctc ggccgtgcag agcaacgtga 132600
acctgatcaa caacttccac cacctggtgc acttcttcga gcacgagaag cgcgcgcggt 132660
acgtgttcga ccgcggggcc gtgatcgtgt tccctctggc gcgcgggtcc gcggactcga 132720
tctcgccgga ggcggcggcg gcgctgggct tcgcgccgca ctcggagttc ctcaagttcg 132780
tgttcctgca gatcgcgctg ctgtacctga agatctacga gctcccgggc tgcacgaact 132840
tcctgcacgt ggacctgaag cccgacaacg tgctcatctt cgacagtgcg cgcgcgctca 132900
gcgtgaccgc ggccggcgcg actttccgct tcgaggagcc cgtgcgcgcg gcgctgaacg 132960
acttcgactt cgcgcgcgtg gccaccatcg agaaccgcaa gatcgcgggc agcgtccgcg 133020
tgccgcagaa ctggtactac gacttccact tcttcgcgca cacgctgctg cgcgcgtacc 133080
cgcacatcgc cgcggaggac ccgggcttcc acgcgctgct ctcggagctc acggtctcgt 133140
gctcgcgcgg gacctgcgac cgcttccggc tgcgcgtgtc ctcgccgcac cccatcgagc 133200

acctcgcgcg gctggtgcgc cgcgacgtct tctcccgctg gataaatgcc gccgcggacg 133260
cccccgacgc cgccgcactc tcctgagccc acgcccgcgg cgccgggctc gctgtacgac 133320
gtcttcctcg cgcgcttcct gcgccggctg gccgcgcgcg cggcgccggc ctcggccgcc 133380
tgcgccgtgc gcgtgggtgc ggtgcgcggc cgcctgcgga actgcgagct agtggtgctg 133440
aaccgctgcc acgcggacgc ggccggcgcg ctcgcgctag cctccgcggc gctcgccgag 133500
acgctggcgg agctcccgcg cgcggacaag ctcgccgtcg cgctcgagct gggcgtggac 133560
cccgagcacc cggagctgac gccggacccc gcctgcgcag gcgagagcgc actcgcacag 133620
aacatcgaca tccagacgct ggacctgggc gactgcggag accccaaagg ccgccgactg 133680
cgcgtggcgc tggtgaacag cggccacgcg gccgcgaact gcgcgctcgc gcgcgtggcg 133740
accgcgctga cgcgccgcgt gcccgcgagc cggcacggcc tcgcggaggg cggcacgccg 133800
ccgtggacgc tgctgctggc ggtggccgcg gtgacggtgc tcggcgtggt ggcggtttca 133860
ctgctgcggc gcgcgctgcg ggtacgctac cgcttcgcgc ggccggccgc gctgcgcgcg 133920
tagccgcgca aaatgtaaat tataacgccc aacttttaag ggtgaggcgc catgaagttg 133980
ctcgtcggca tactagtagc cgtgtgcttg caccagtatc tgctgaacgc ggacagcaac 134040
acgaaaggat ggtccgaagt gctgaaaggc agcgagtgca agcctaggcc gattgttgtt 134100
cctgtaagcg agacgcaccc agagctgact tctcagcggt tcaacccgcc gtgtgtcacg 134160
ttgatgcgat gcggcgggtg ctgcaacgac gagagcttgg aatgcgtccc cacggaagaa 134220
gtaaacgtga cgatggaact cctgggggcg tcgggctccg gtagtaacgg gatgcaacgt 134280
ctgagcttcg tagagcataa gaaatgcgat tgtagaccac gattcacaac cacgccaccg 134340
acgaccacaa ggccgcccag aagacgccgc tagaactttt tatggaccgc agatccaaac 134400
gatgatgcga tcaggtcatg cggaagaagg cgccacggag caaagtgaaa aaggaccgcc 134460
tagcagtcga gaccctcccg ccgcagccgc ggacacccca cacccgcctt ccacccgcca 134520
gacgccaaca ccgcagccaa caagcatgca cccctcgccg cgcaggctgc tcggcgcgct 134580
cgcgctggtg gcgctgggct tcctcctcgg cgggctcttc cgccccgcgg cgccgccgct 134640
gccggccgcc ctcgtggagg cgggccccgt ccgcgcgaac ggctccgcct cggtgacctg 134700
cctgaccgtc ggcggcgacg ggcggcacat ggcggtggtc gcgcacggcg gcgggacgct 134760
ctcgccggtg tacccgctcg ccgccggcat gcacgcgacc ttcgcctcgc tgcgcaaggg 134820
cgcgctgctg ctgaacgtcg cgaccgtgca catctacgac gtgcgcgcgc tcgggccgga 134880
gttcgagctg acctgcgtcg cggtggcggg cggctacaac gcggcctggg cggccgcgcg 134940
gcccgcggcc gagtggcgcc gccagctggc gcggatgcac cgctcggagc tgtgaccctc 135000
tccctcccgg tctcccatcc gtttttgtaa tcggccttag tagattagac cagcatcccg 135060
cgcccttgtc cgagaacaag taacagtaac cgttacctca ctcgccactc ctcggaataa 135120
tagaacgaga gaacgagaga acgagttaac cgttgctcac tcgctcactc ggtgtgagag 135180
aacaagtaac gttgctcact cgctcactcg gtgtgagaga acaagagaac gagtagctgt 135240
tgctaactca atcacccctc ggagtaagag aacaagagca gtcaactacc cactcagtct 135300
tggatgagag gcagaggacg agttgacgag ttgaacagtt aatcctcact cactcagagc 135360
gagagagcga gagagtggag gacgagttaa caagtcaatc ctcactcaga gtgagagagt 135420
gagagagtgg aggacgagtt aatggttaac agttatcacc actcagagtg agagcggagg 135480
acgagtcaac cactcgctcg cccactccga gttagagagg gaaccagtgc gagttaacgc 135540
gcacacgagc gagagaacgt aaactcgctc gcgcgctcgc tcggctaacc gtcggcctct 135600
cccaaaactc ttcgtaaaac tttcccgatg acagttcacc ctccaaaact ttgtaaaact 135660
aaactgttcg gaggtcggtc tgctgcctct ctaactctcc gtaaaacgtt tgtaaactgt 135720
cggaggtcgg tgacccgctc aaccgtccgc gaaaactttt cgcaggcagt gtctgcctct 135780
ctcggactct ccgcaaacac tttcgcggaa cctcggaggg tggtcgacct ctctccaaac 135840
tcttgcaaaa ctttttcgcg gaaccgttgg aggccagtcc tccctccaaa ctctttgtaa 135900
aatcttttcg aggccagtcc tcctctccaa aacgttccgc aaaatctttg ggaggtcggc 135960
ctctcctctc cagaacgttc cgtaaaactc ttggaggccg cccgcggcac gcgaggcgga 136020
ggatccgagg tgtcgacctc cctcaaaaac tttgtaaaaa ctttttataa aactttccgc 136080
ggaacctcgg agagtaggtc gacctctctc aaaactctta taaaactttt ccgcggaacc 136140
gttggaaggt aggtcgacct ctctcaaaac tcttataaaa cttttccgcg gaaccgttgg 136200
aaggtaggtc gacctctctc aaaactctta taaaactttt ccgcggaacc gttggaggca 136260
ggtcggcctc tcaaactctt tgcgagaact cttcgcgaga actcttcgat aactttagga 136320
ggtcaggtcg acctcccaaa acttttgcga gaactctctg taaaacttta ggaggtcggt 136380
acctccctca aaacttttta taaaactttt cgcggaacct ccggagacgg gccgccgccg 136440
cgaccgcggg agcggagagg ccgacctccc gagacgttcc gcgttaccgt cggggtaggc 136500
gtcctctcga gaacgccaaa agacttcgtg caaaaacttt tcggaggggc gcggagggcg 136560
ggtcagctcc cgcgaactcc cgcagaacct tttcgcgcga ccgcgaaggc cggccgcctc 136620
tcccacactc tcaagagctt ttcggaggag aggaagggca ggtcgccccc acctccgacg 136680
ctttgtaaaa acgtttacgc ggaacctcgg aggcaggtcg cctccctcga aaactcctcg 136740
cgaaaccttt aaaaactttt tgcgaaaact tttcggagga tgtcggaggg cgggcggctc 136800
ttccaaacct ccgcagaacc ttttcgcgca accgttggaa gacaggtcgg cctctctcga 136860
aaacttttaa aactttgtaa acgcgttggc gggaccgtcg cgggagagcg gccgcccgcg 136920
gcacgcgaga ggaggaaccg ttggaaggca gtcggcctct cccgaaaact ttttataaaa 136980

```
acttttccgc ggaaccgttg gaggcaggtc ggcctctctc agagtctaaa aactttttgc 137040
gggactcgga cggcgcggtc acccgaccac ctgactcctg tctcacccgt actacttgga 137100
cttctgtttc cctgactccc gactccctga cctcccgact ccctgactcc cgactccctg 137160
actcccgact ccctgactcc cgactccctg actcccgact ccctgactcc cgactccctg 137220
actcccgact ccctgactcc cgactccctg actcccgact ccctgactcc cgactccctg 137280
actcccgact ccctgactcc cgactccctg actcccgact ccctgactcc cgactccctg 137340
actcccgact ccctgactcc ctgactccag agcgaggtct cgcggctgcg gggtgccgcc 137400
tccgcggagt cgcgttcccg cggacgcccg tcctcgaaag cattcagcag ttccagcctc 137460
tgccgtagct cctcccgcag gaactcctgg tccgcgttcg tcgcggcacc gcggctcagc 137520
cgccgcggga gccggccgcc gcccgcgaag ccgcggatcc                  137560
```

## Claims

1. Use of a purified and isolated polynucleotide with a sequence selected from a group of sequences consisting of nucleotides number 122616 to 136025 of SEQ ID 01 (PPVO insert of VVOV 82), 31003 to 46845 of SEQ ID 01 (PPVO insert of VVOV 96), 24056 to 33789 of SEQ ID 01 (PPVO insert of VVOV 97), 10264 to 20003 of SEQ ID 01 (PPVO insert of VVOV 215), 82324 to 92502 of SEQ ID 01 (PPVO insert of VVOV 243), 47952 to 66263 of SEQ ID 01 (PPVO insert of VVOV 245), 89400 to 103483 of SEQ ID 01 (PPVO insert of VVOV 283), 74804 to 88576 of SEQ ID 01 (PPVO insert of VVOV 285), and 102490 to 108393 of SEQ ID 01 (PPVO insert of VVOV 330) or their complementary sequences and functional variants thereof, to induce an immunomodulating activity.

2. Use of a purified and isolated polynucleotide with a sequence selected from a group of sequences consisting of nucleotides 9989 to 8070 (ORF 3r), 11195 to 10062 (ORF 4r), 11493 to 11227 (ORF 5r), 11802 to 12038 (ORF 6), 12358 to 12080 (ORF 7r), 13980 to 12364 (ORF 8r), 14826 to 14053 (ORF 9ar), 15080 to 15394 (ORF 10), 16838 to 15423 (ORF 11r), 19021 to 16847 (ORF 12r), 19704 to 19156 (ORF 13r), 20314 to 19736 (ORF 14r), 26908 to 23873 (ORF 18r), 26926 to 27213 (ORF 19), 27626 to 27216 (ORF 20r), 29754 to 27616 (ORF 21r), 32217 to 29800 (ORF 22r), 33380 to 32418 (ORF 23r), 33602 to 33393 (ORF 24r), 34466 to 33612 (ORF 25r), 34735 to 34502 (ORF 26r), 35905 to 34739 (ORF 27r), 37194 to 35905 (ORF 28r), 37200 to 39248 (ORF 29), 41037 to 39229 (ORF 30r), 41374 to 42066 (ORF 31), 42336 to 41731 (ORF 32r), 42407 to 41997 (ORF 33r), 42410 to 43765 (ORF 34), 43770 to 43958 (ORF 35), 43980 to 44534 (ORF 36), 45727 to 44537 (ORF 37r), 45760 to 46557 (ORF 38), 46567 to 47568 (ORF 39), 47572 to 48303 (ORF 40), 48352 to 48621 (ORF 41), 49887 to 48634 (ORF 42r), 49917 to 50693 (ORF 43), 50719 to 51102 (ORF 44), 51059 to 51511 (ORF 44a), 51584 to 52591 (ORF 45), 52509 to 53066 (ORF 46), 53523 to 53023 (ORF 47r), 53607 to 57473 (ORF 48), 58070 to 57528 (ORF 49r), 57700 to 58662 (ORF 50), 59674 to 58673 (ORF 51r), 62089 to 59678 (ORF 52r), 62198 to 62881 (ORF 53), 62909 to 63862 (ORF 55), 63858 to 64271 (ORF 56), 64309 to 66831 (ORF 57), 76120 to 74207 (ORF 64r), 76749 to 76186 (ORF 65r), 77698 to 76799 (ORF 66r), 79343 to 77709 (ORF 67r), 79816 to 79367 (ORF 68r), 80529 to 79858 (ORF 69r), 80774 to 80529 (ORF 70r), 82815 to 80788 (ORF 71r), 83835 to 82834 (ORF 72r), 83874 to 85583 (ORF 73), 85535 to 84402 (ORF 74r), 88096 to 85574 (ORF 75r), 87759 to 88667 (ORF 76), 88920 to 88642 (ORF 77r), 91652 to 88938 (ORF 78r), 91667 to 92674 (ORF 79), 93466 to 92681 (ORF 80r), 93761 to 93486 (ORF 81r), 94060 to 93788 (ORF 82r), 94238 to 94080 (ORF 83r), 94508 to 94242 (ORF 84r), 95571 to 94498 (ORF 85r), 96187 to 95600 (ORF 86r), 96202 to 97665 (ORF 87), 97915 to 97643 (ORF 88r), 98251 to 99537 (ORF 89), 99537 to 99974 (ORF 90),100001 to 101140 (ORF 91), 101168 to 104650 (ORF 92), 106354 to 104795 (ORF 93r), 107947 to 106400 (ORF 94r), 168256 to 107990 (ORF 95r), 108719 to 108300 (ORF 96r), 109679 to 108738 (ORF 97r), 122350 to 123924 (ORF 120), 123962 to 125566 (ORF 121), 125193 to 124591 (ORF 122r), 125689 to 123935 (ORF 123r), 123839 to 123297 (ORF 123ar), 125652 to 126170 (ORF 124), 126121 to 125699 (ORF 125r), 126279 to 127769 (ORF 126), 127851 to 128408 (ORF 127), 128520 to 130076 (ORF 128), 130105 to 131700 (ORF 129), 131790 to 133283 (ORF 130), 133246 to 133920 (ORF 131), 133972 to 134370 (ORF 132), 134418 to 134693 (ORF 133a), 134402 to 134992 (ORF R1), 134853 to 134419 (ORF R2r), 135628 to 135897 (ORF R3), 136780 to 137112 (ORF R4), and137558 to 137022 (ORF R5r) of SEQ ID 01 or their complementary sequence and functional variants thereof to induce an immunomodulating activity.

3. Use of a recombinant protein encoded by a polynucleotide of the use of claim 1 or 2 or functional variants or fragments of more than five amino acids thereof to induce an immunomodulating activity.

4. Use of a vector comprising a polynucleotide of the use of claim 1 or 2 to induce an immunomodulating activity.

**5.** Use of cells containing the vector of the use of claim 5 or a polynucleotide of the use of claim 1 or 2 to induce an immunomodulating activity.

**6.** Use of any of claims 1 to 4, wherein the immunomodulating activity is an immunostimulating activity.

Figure 1

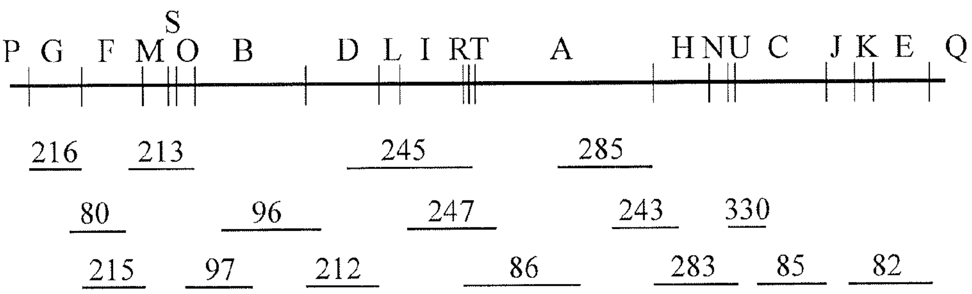
P G F M S O B D L I R T A H N U C J K E Q
216
213
245
285
80
96
247
243
330
215
97
212
86
283
85
82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 3504940


### Non-patent literature cited in the description


- **CASAL.** *Biotechnol. Genet. Eng. Rev.,* 2001, vol. 18, 73-87
- **ELLIS.** *Curr. Opin. Biotechnol.,* 1996, vol. 7 (6), 646-52
- **ROY.** *Intervirol-ogy,* 1996, vol. 39 (1-2), 62-71
- **GAUDIN et al.** *Gen. Virol.,* 1995, vol. 76, 1541-56
- **HUGHSON.** *Curr. Biol.,* 1995, vol. 5 (3), 365-74
- **UHLEN et al.** *Curr. Opin. Biotechnol.,* 1992, vol. 3 (4), 363-369
- **ARSHADY.** *Biomaterials,* 1993, vol. 14 (1), 5-15
- **CLELAND.** *Pharm Biotechnol.,* 1997, vol. 10, 1-43
- **HANES et al.** *Pharm. Biotechnol.,* 1995, vol. 6, 389-412
- **JANES et al.** *Adv. Drug Deliv. Rev.,* 2001, vol. 47 (1), 83-97
- **MERCER et al.** *Virology,* 1997, vol. 229, 193-200
- **NEEDLEMAN ; WUNSCH.** *J. Mol. Biol.,* 1970, vol. 48, 443-453
- **HENIKOFF ; HENIKOFF.** *Proc. Natl. Acad. Sci. USA,* 1992, vol. 89, 10915-10919
- **VILCEK et al.** *J. Clin. Microbiol.,* vol. 32, 2225-2231